# EUROPEAN PATENT SPECIFICATION

(11) **EP 2 922 972 B1**
(45) Date of publication and mention of the grant of the patent: **26.04.2017**
(21) Application number: 13824361.3
(22) Date of filing: 26.11.2013
(51) Int. Cl.: C12Q 1/68

(54) **COLORECTAL CANCER CLASSIFICATION WITH DIFFERENTIAL PROGNOSIS AND PERSONALIZED THERAPEUTIC RESPONSES**
KOLOREKTALKARZINOMKLASSIFIZIERUNG MIT DIFFERENTIELLER PROGNOSE UND PERSONALISIERTEN THERAPEUTISCHEN REAKTIONEN
CLASSIFICATION DE CANCER COLORECTAL À L'AIDE DE PRONOSTIC DIFFÉRENTIEL ET DE RÉPONSES THÉRAPEUTIQUES PERSONNALISÉES

(30) Priority: 26.11.2012 WO PCT/IB2012/056728
(43) Date of publication of application: 30.09.2015
(73) Proprietor: Ecole Polytechnique Fédérale de Lausanne (EPFL), 1015 Lausanne (CH); Oregon Health & Science University, Portland, OR 97239 (US); Beth Israel Deaconess Medical Center, Inc., Boston, MA 02215 (US)
(72) Inventor: SADANANDAM, Anguraj, Tamilnadu Pollachi 642002 (IN); LYSSIOTIS, Costas, Ann Harbor Michigan 48103 (US); HANAHAN, Douglas, CH-1010 Lausanne (CH); GRAY, Joe, Lake Oswego Oregon 02130 (US)
(74) Representative: KATZAROV S.A.
(86) International application number: PCT/IB2013/060416
(87) International publication number: WO 2014/080381

(56) References cited:
- EP-A1- 2 236 626
- WO-A2-2006/135886
- WO-A2-2010/145796
- PIERO DALERBA ET AL: "Single-cell dissection of transcriptional heterogeneity in human colon tumors", NATURE BIOTECHNOLOGY, vol. 29, no. 12, 13 November 2011 (2011-11-13), pages 1120-1127, XP055115348, ISSN: 1087-0156, DOI: 10.1038/nbt.2038
- ANGURAJ SADANANDAM ET AL: "A colorectal cancer classification system that associates cellular phenotype and responses to therapy", NATURE MEDICINE, vol. 19, no. 5, 14 April 2013 (2013-04-14) , pages 619-625, XP055115260, ISSN: 1078-8956, DOI: 10.1038/nm.3175
- ANDREAS SCHLICKER ET AL: "Subtypes of primary colorectal tumors correlate with response to targeted treatment in colorectal cell lines", BMC MEDICAL GENOMICS, BIOMED CENTRAL LTD, LONDON UK, vol. 5, no. 1, 31 December 2012 (2012-12-31), page 66, XP021137785, ISSN: 1755-8794, DOI: 10.1186/1755-8794-5-66
- SADANANDAM ANGURAJ ET AL: "Reconciliation of classification systems defining molecular subtypes of colorectal cancer: interrelationships and clinical implications.", CELL CYCLE (GEORGETOWN, TEX.) 1 FEB 2014, vol. 13, no. 3, 1 February 2014 (2014-02-01), pages 353-357, XP8168939, ISSN: 1551-4005

## Description

### FIELD OF THE INVENTION

The present invention relates to gene sets, the expression levels of which are useful for classifying colorectal tumors and thereby predicting disease-free survival prognosis and response of patients to specific therapies that are either novel or currently available in the clinics for treating colorectal cancer patients.

### BACKGROUND OF THE INVENTION

Colorectal cancer (CRC) is a cancer arising from uncontrolled cell growth in the colon, rectum or in the appendix. Genetic analysis shows that colon and rectal tumors are essentially genetically the same type cancer. Symptoms of colorectal cancer typically include rectal bleeding, anemia which are sometimes associated with weight loss and changes in bowel habits. It typically starts in the lining of the bowel and if left untreated, can grow into the muscle layers underneath, and then through the bowel wall. Cancers that are confined within the wall of the colon are often curable with surgery while cancer that has spread widely around the body is usually not curable and management then focuses on extending the person's life via chemotherapy and improving quality of life.

Colorectal cancer is the third most commonly diagnosed cancer in the world, but it is more common in developed countries. Most colorectal cancer occurs due to lifestyle and increasing age with only a minority of cases associated with underlying genetic disorders. Greater than 75-95% of colon cancer occurs in people with no known inherited familial predisposition.. Risk factors for the non-familial forms of CRC include advancing age, male gender, high fat diet, alcohol, obesity, smoking, and a lack of physical exercise.

Colorectal cancer is often found after symptoms appear, but most people with early colon or rectal cancer don't have symptoms of the disease. Symptoms usually only appear with more advanced disease. This is why screening is effective at decreasing the chance of dying from colorectal cancer and is recommended starting at the age of 50 and continuing until a person is 75 years old. Localized bowel cancer is usually diagnosed through sigmoidoscopy or colonoscopy.

Diagnosis of colorectal cancer is via tumor biopsy typically done during sigmoidoscopy or colonoscopy. The extent of the disease is then usually determined by a CT scan of the chest, abdomen and pelvis. There are other potential imaging test such as PET and MRI which may be used in certain cases. Colon cancer staging is done next and based on the TNM system which is determined by how much the initial tumor has spread, if and where lymph nodes are involved, and if and how many metastases there are.

Different types of treatment are available for patients with colorectal cancer. Four types of standard treatments are used: surgery, chemotherapy, radiation therapy and targeted therapy with the EGFR inhibitor cetuximab. While all can produce responses in patients with advanced disease, none are curative beyond surgery in early stage of disease. Notably, some patients demonstrate pre-existing resistance to certain of these therapies in particular to cetuximab or FOLFIRI therapy. Thus only a fraction of CRC patients respond well to therapy. As such, colorectal cancer continues to be a major cause of cancer mortality, and personalized treatment decisions based on patient and tumour characteristics are still needed. EP 2 236 626 A1 (UNIV MADRID AUTONOMA [ES]), WO 2006/135886 A2 (UNIV MICHIGAN [US]; CLARKE MICHAEL F [US]; WANG XINHAO [US]; LEWICKI J), and WO 2010/145796 A2 (MERCK PATENT GMBH [DE]; STROH CHRISTOPHER [DE]; VON HEYDEBRECK ANJA [D]) describe methods for the classification of colorectal cancer and the prediction of treatment response with the help of expression markers.

### SUMMARY OF THE INVENTION

To solve the above-identified problem, Applicants classified colorectal cancer in to six subtypes based on the integrated analysis of genes expression profiles and cetuximab-based drug response. These subtypes are predictive of disease-free survival prognosis and response to selected therapies.

Thus in an embodiment, the present invention provides an *in-vitro* method for the prognosis of disease-free survival of a subject suffering from colorectal cancer or suspected of suffering therefrom and who has undergone a prior surgical resection of colorectal cancer, the method comprising
(i) providing a biological sample from said subject comprising colorectal cancer cells or suspected to comprise colorectal cancer cells;
(ii) measuring the expression level of one or a combination of genes selected from the group of genes listed in Table 2, and
(iii) classifying said biological sample as "Stem-like", "Inflammatory", "Transit-amplifying (TA)", "Goblet-like" and "Enterocyte" on the basis of the gene expression profile according to Table 2,
wherein
- "Stem-like" type of colorectal cancer indicates poor disease-free survival,
- "Inflammatory" type of colorectal cancer indicates intermediate disease-free survival,
- "Transit-amplifying (TA)" type of colorectal cancer indicates good disease-free survival,
- "Goblet-like" type of colorectal cancer indicates good disease-free survival, and
- "Enterocyte" type of colorectal cancer indicates intermediate disease-free survival.

The present invention further provides an *in-vitro* method for predicting the likelihood that a subject suffering from colorectal cancer or suspected of suffering therefrom and who has undergone a prior surgical resection of colorectal cancer will respond to therapies inhibiting or targeting EGFR, such as cetuximab, and/or cMET, the method comprising
(i) providing a biological sample from said subject comprising colorectal cancer cells or suspected to comprise colorectal cancer cells;
(ii) measuring the expression level of one or a combination of genes selected from the group of genes listed in Table 2, and
(iii) classifying said biological sample as "Stem-like", "Inflammatory", "Transit-amplifying (TA)", "Goblet-like" and "Enterocyte" on the basis of the gene expression profile according to Table 2,
wherein
- high expressions of AREG and EREG genes and low expressions of BHLHE41, FLNA and PLEKHB1 genes in "Transit-amplifying (TA)" type indicates that at metastatic setting said subject will be responsive to cetuximab treatment and resistant to cMET inhibitor therapy and this signature defines a subtype of TA type designed as "Cetuximab-sensitive transit-amplifying subtype (CS-TA)".
- low expressions of AREG and EREG genes and high expressions of BHLHE41, FLNA and PLEKHB1 genes in "Transit-amplifying (TA)" type indicates that at metastatic setting said subject will be resistant to cetuximab treatment and will be responsive to cMET inhibitor therapy, and this signature defines a second subtype of TA type named as "Cetuximab-resistant transit-amplifying subtype (CR-TA)".

The present invention also provides an *in-vitro* method for predicting the likelihood that a subject suffering from colorectal cancer or suspected of suffering therefrom and who has undergone a prior surgical resection of colorectal cancer will respond to cytotoxic chemotherapies such as FOLFIRI, the method comprising
(i) providing a biological sample from said subject comprising colorectal cancer cells or suspected to comprise colorectal cancer cells;
(ii) measuring the expression level of one or a combination of genes selected from the group of genes listed in Table 2, and
(iii) classifying said biological sample as "Stem-like", "Inflammatory", "Transit-amplifying (TA)", "Goblet-like" and "Enterocyte" on the basis of the gene expression profile according to Table 2,
wherein
- "Stem-like" type of colorectal cancer predicts good response in both adjuvant and metastatic settings,
- "Inflammatory" type of colorectal cancer predicts good response in adjuvant setting,
- "TA (transit-amplifying)" type of colorectal cancer predicts poor response in both adjuvant and metastatic settings,
- "Goblet-like" type of colorectal cancer predicts poor response in adjuvant setting, and
- "Enterocyte" type of colorectal cancer predicts good response in adjuvant setting.

### BRIEF DESCRIPTION OF THE FIGURES

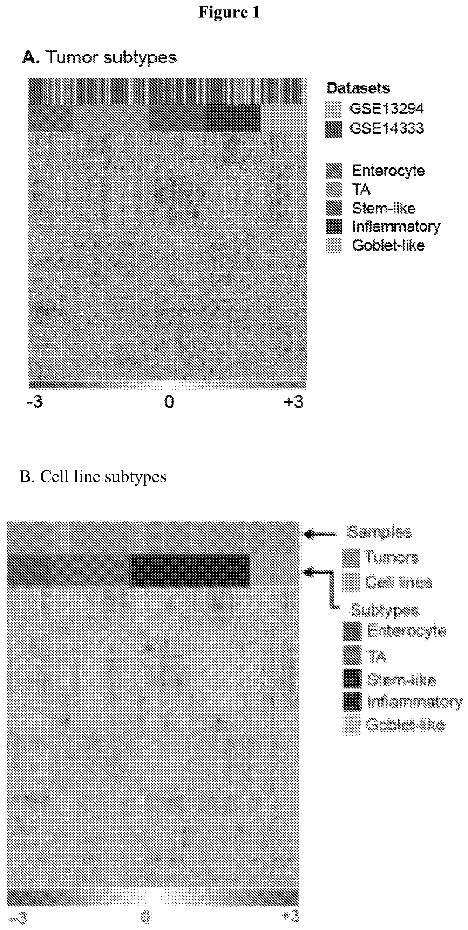

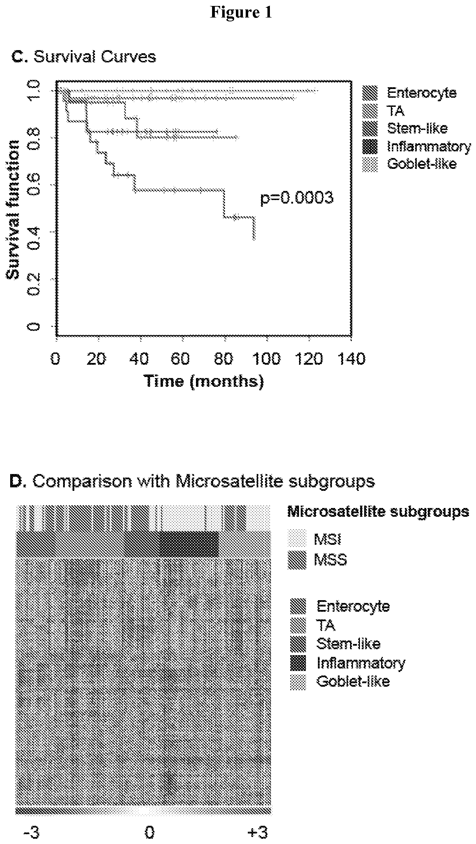

**Figure 1** shows *Classification of colorectal tumors and cell lines and their prognostic significance.* CRC subtypes were identified in A) tumors (from two combined datasets: core dataset, GSE13294 and GSE14333) and B) cell lines. C) Differential disease-free survival among the CRC subtypes for patient tumors from the GSE14333 dataset are plotted as Kaplan-Meier Survival curves. D) Heatmap depicting known MSI or MSS status for each of the patient colorectal tumor subtype samples from the dataset GSE13294.

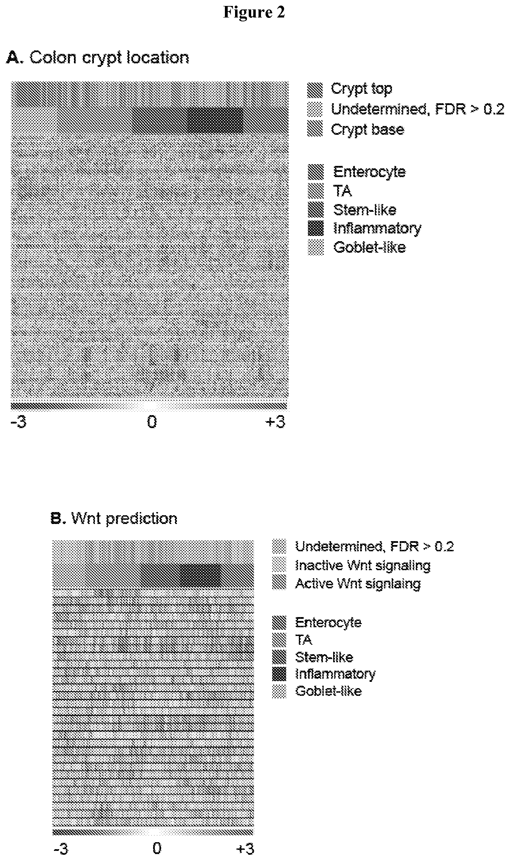

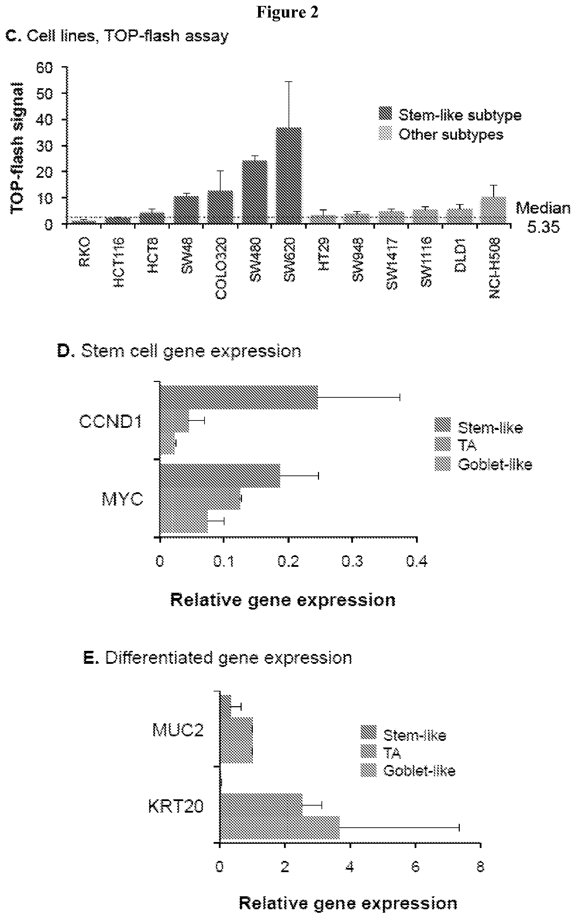

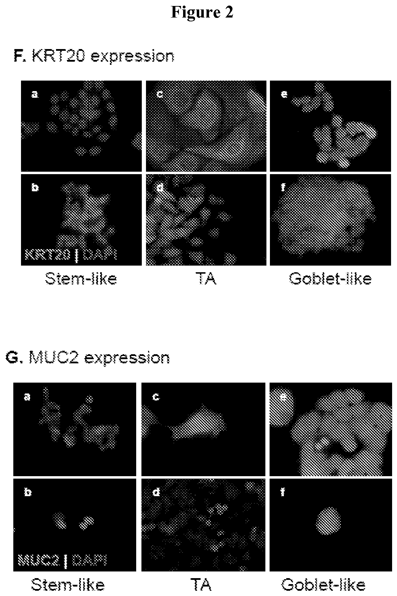

**Figure 2** shows *Cellular phenotype and Wnt signaling in the CRC subtypes.* Prediction of A) colon-crypt location (top or base) and B) Wnt activity in patient colorectal tumors by applying specific signatures and using the NTP algorithm. C) TOP-flash assay depicting Wnt activity in colorectal cancer cell lines. D) Quantitative (q)RT-PCR analysis showing the average expression of stem cell and E) differentiation-specific markers in CRC subtype cell lines (HT29 and LS174T from goblet-like; LS1034, NCI-H508 and SW948 from TA; and SW48, HCT8 and SW620 from stem-like subtypes). The qRT-PCR data is plotted relative to the house keeping gene RPL13A. Error bars represent standard error of mean (SEM, for biological triplicates). Immunofluorescent analysis of the differentiation markers F) KRT20 and G) MUC2 are presented in red, and nuclei are counter-stained with DAPI (blue). Cell lines a) HCT116 and b) colo320 belong to the stem-like; c) SW1417 and d) SW948 belong to TA; and e) HT29 and f) LS174T belong to goblet-like subtype.

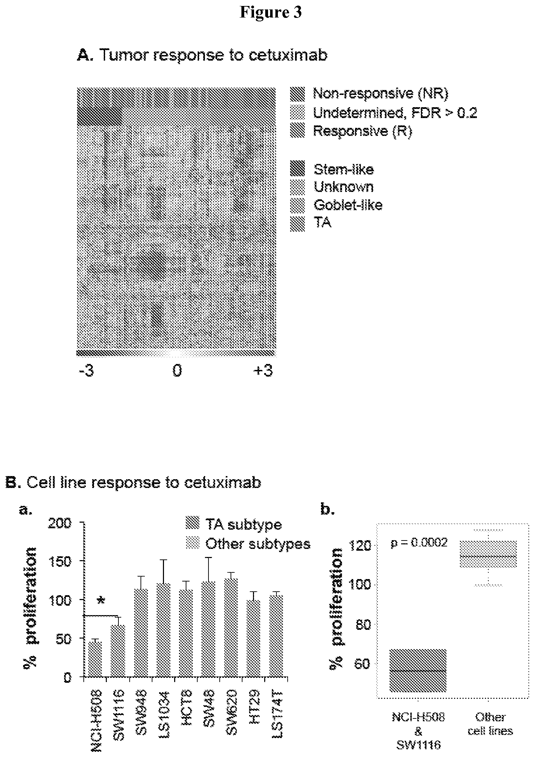

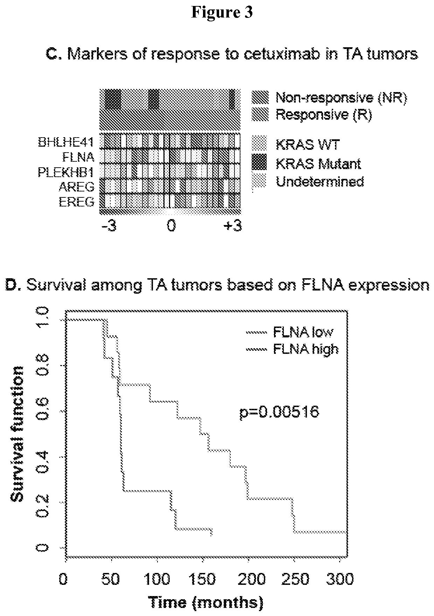

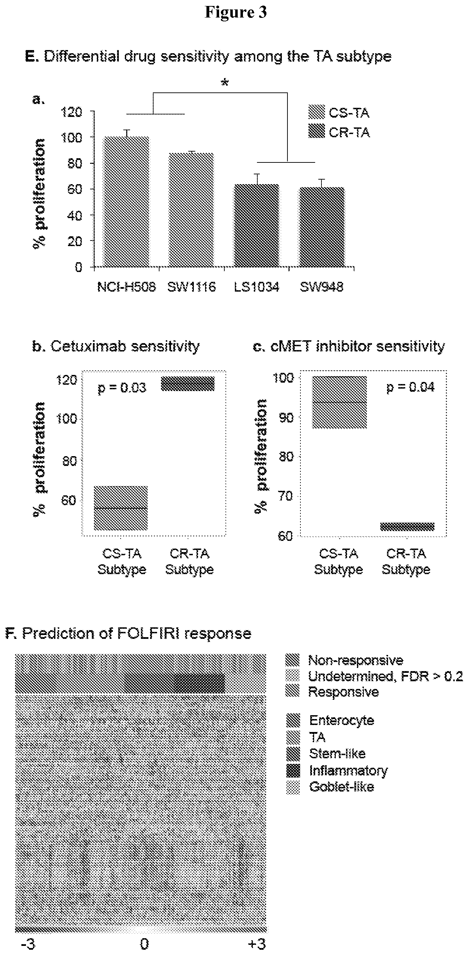

**Figure 3** shows *Differential drug sensitivity among CRC subtypes.* A) Individual CRC metastatic patient response to cetuximab treatment and its association with subtypes. B) Cetuximab response in CRC subtype-specific cell lines are plotted as percent proliferation of cells treated with 3.4 µg cetuximab, and normalized to vehicle-treated cells in a) bar plot and b) boxplot (sensitive versus resistant cell lines). Asterisk (*) represents p-value, as calculated using student t test (p=0.0002). Error bars represent SEM for technical triplicates. C) Heatmap depicting differential gene expression patterns and the *KRAS* mutation status among TA subtype CRC patient samples that responded (R; complete, partial response and stable disease were considered as response) to cetuximab versus those that did not respond (NR). D) Kaplan-Meier curve of differential survival based on FLNA expression in TA subtype samples. E) Differential response to the cMet inhibitor PHA-665752 (125 nM) in CR-TA and CS-TA subtype-specific cell lines, plotted relative to vehicle-treated cells as a) bar plot and b) boxplot. c) Differential response to cetuximab in CR-TA and CS-TA subtype-specific cell lines relative to vehicle-treated cells. Asterisk (*) represents p-value as calculated using student t test (p=0.04). Error bars represent SEM for technical triplicates. G) Prediction of individual patient colorectal tumor response to FOLFIRI by applying published FOLFIRI response signatures to the core dataset.

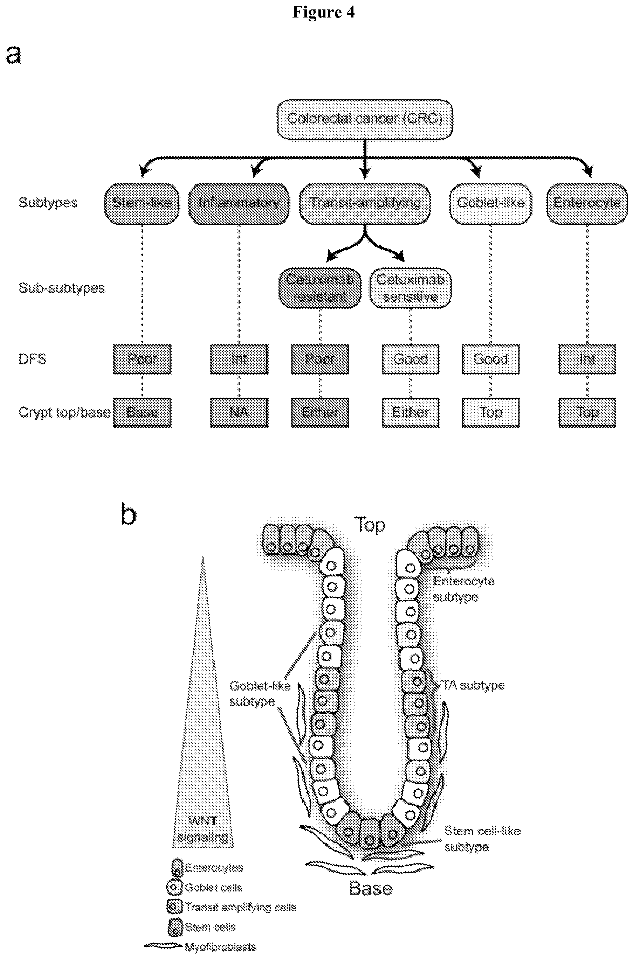

**Figure 4** shows Summary of the A) characteristics of each of the CRC subtypes and B) CRC subtype phenotype based on colon-crypt location. UP - unpredicted and ND - not done.

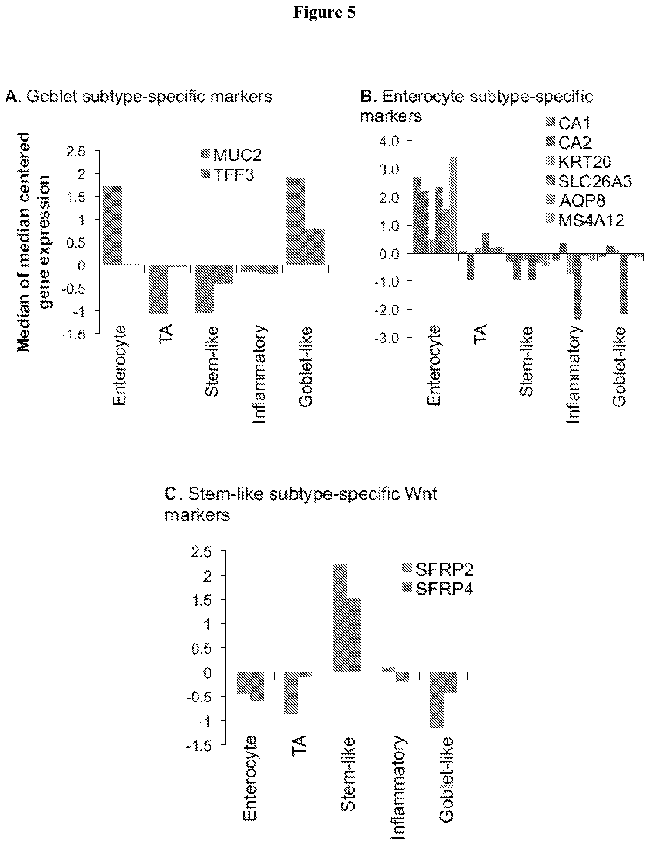

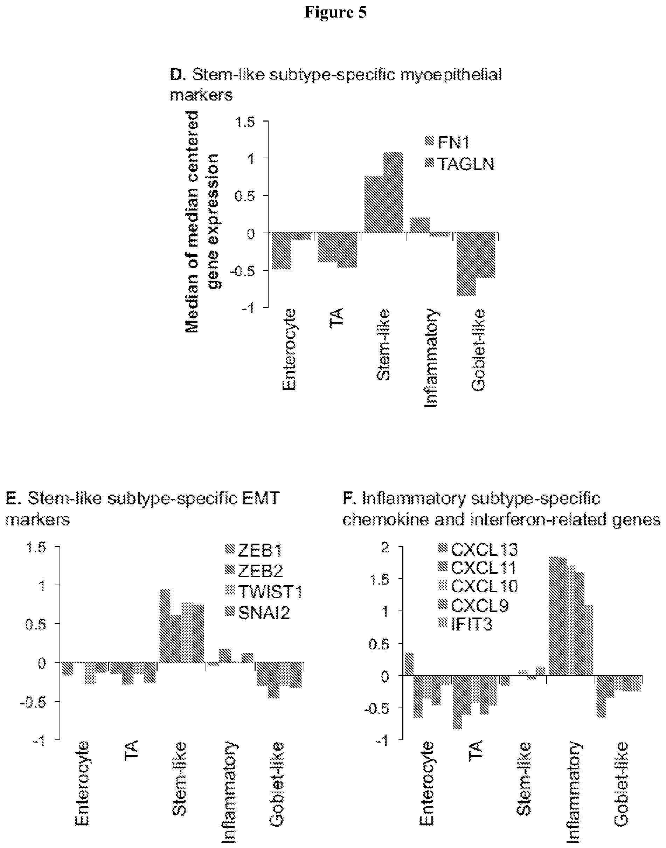

**Figure 5** shows *Mapping the cellular phenotypes of each subtype.* A) Goblet specific markers (MUC2 and TFF3) show high median expression only in CRC goblet-like subtype; B) enterocyte markers¹ (CA1, CA2, KRT20, SLC26A3, AQP8 and MS4A12) show high median expression only in CRC enterocyte subtype; C) Wnt target genes (SFRP2 and SFRP4), D) myoepithelial genes (FN1 and TAGLN) and E) epithelial-mesenchymal (EMT) markers (ZEB1, ZEB2, TWIST1 and SNAI2) show high median expression only in CRC stem-like subtype; and F) chemokine and interferon-related genes (CXCL9, CXCL10, CXCL11, CXCL13, IFIT3) show high median expression only in CRC inflammatory subtype. The gene expression data are presented as the median of median-centered data from DWD merged CRC core microarray datasets.

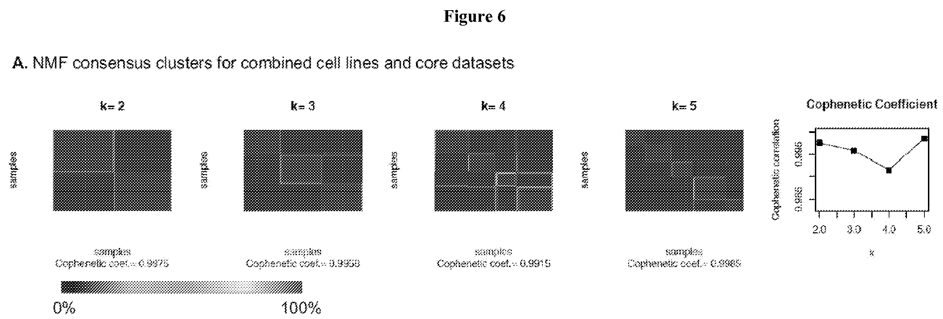

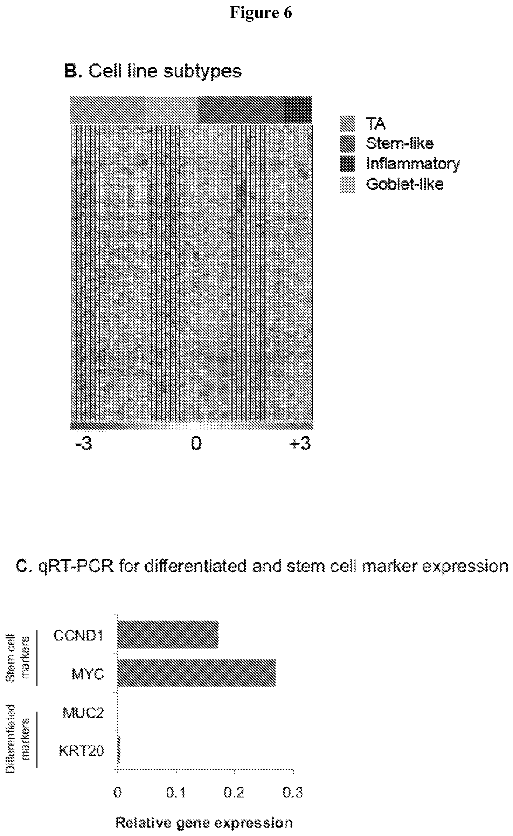

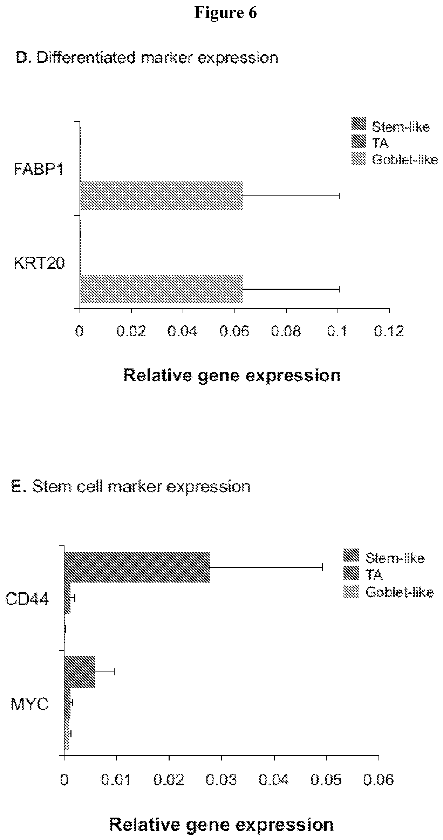

**Figure 6** shows *Subtypes in CRC cell lines and subtype-specific gene expression in CRC xenograft tumors.* A) NMF consensus clustering analysis and cophenetic coefficient for cluster k=2 to k=5 from combining CRC cell line datasets with the core primary tumor datasets; the maximum cophenetic coefficient occurred for k=5. However, CRC cell lines representing only 4 of the 5 subtypes were identified; no cell line for the enterocyte subtype was found. The cell lines dataset is presented after CRCassigner genes had been mapped. B) Heatmap showing CRC subtypes represented amongst a set of CRC cell lines as identified by merging core tumor dataset and cell lines as in Figure 1B. C) Quantitative (q)RT-PCR analysis of SW1116 cell line using stem cell and differentiated markers. D-E) qRT-PCR analysis of xenograft tumors derived from the cell lines HCT116 (stem-like subtype), COLO205 (TA subtype) and HT29 (goblet-like subtype) for D) differentiated and E) stem cell markers. The expression is relative to the house-keeping gene, *RPL13A.* Error bars represent standard deviation (SD; technical triplicates).

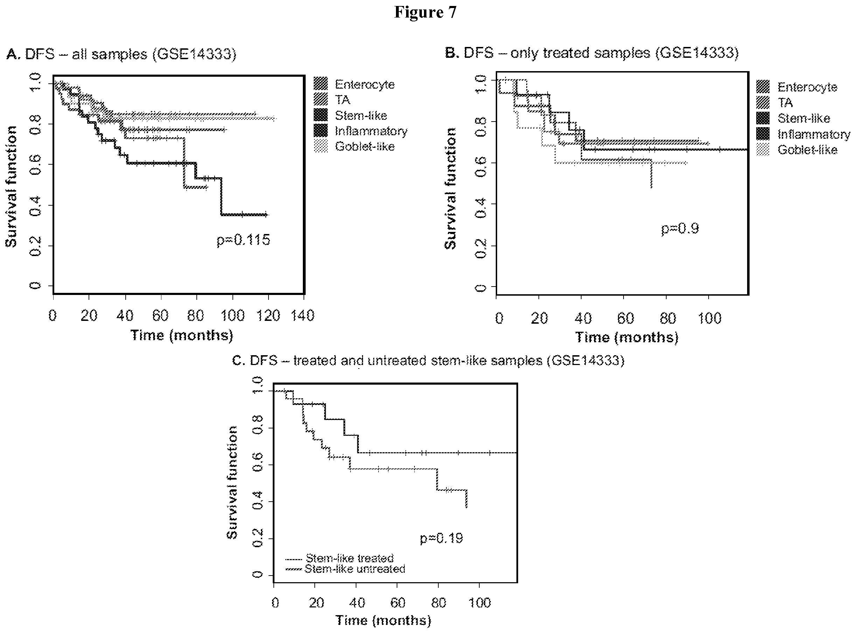

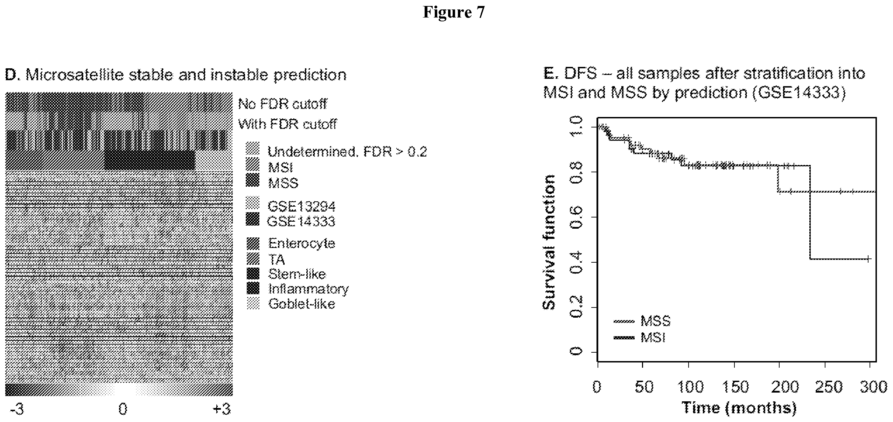

**Figure 7** shows *DFS comparison of CRC subtypes versus MSI*/*MSS.* A-C) Kaplan-Meier Survival curve depicting differential survival for dataset GSE14333, which A) includes both treated (adjuvant chemotherapy and/or radiation therapy) and untreated samples, B) only treated samples and C) treated and untreated samples only from stem-like subtype. D) Predicted MSI status for core dataset (GSE13294 and GSE14333) samples using publicly available gene signatures with the NTP algorithm. Predicted MSI status with FDR<0.2 or no FDR cutoff are shown. E) Kaplan-Meier Survival curve depicting differential DFS for samples from dataset GSE14333 that were predicted to be MSI or MSS.

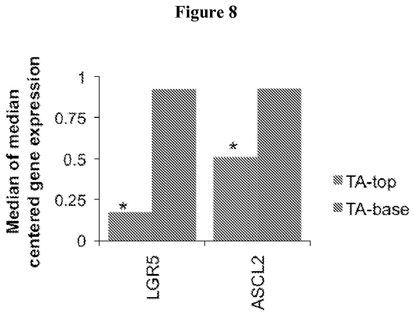

**Figure 8** shows *Differential Wnt targets gene expression in two different sub-populations of TA subtype tumors samples.* Bar graph showing median of median centered gene expression of the Wnt signaling targets *LGRS* and *ASCL2* in the core CRC microarray data for TA subtype tumors that are either predicted to be crypt top- or base-like.

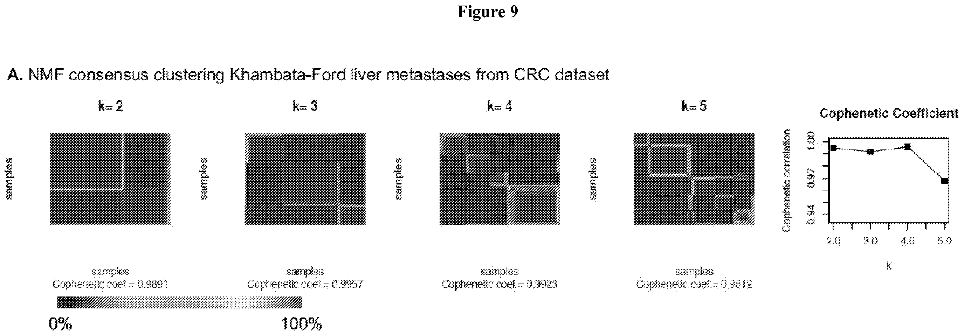

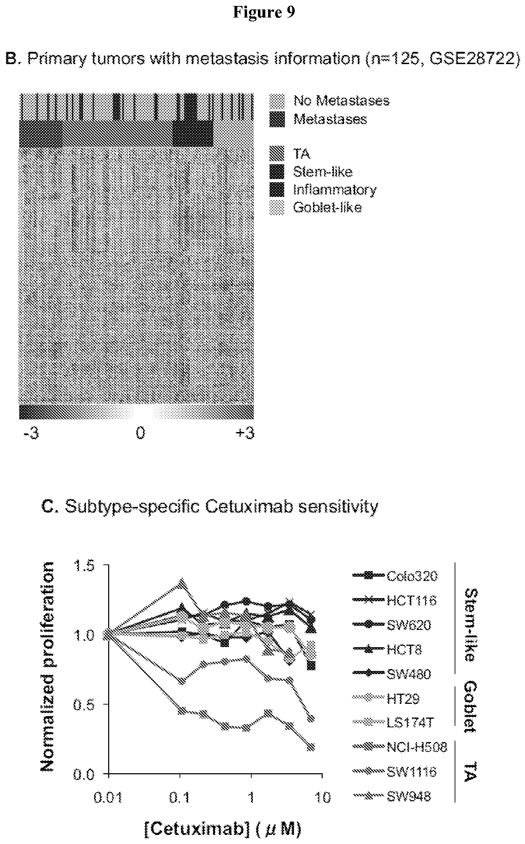

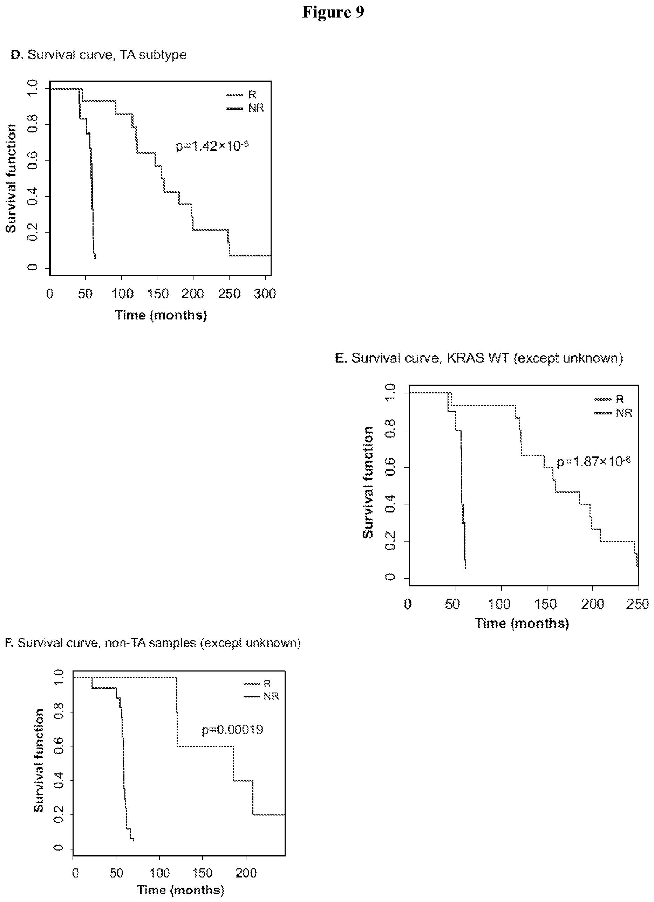

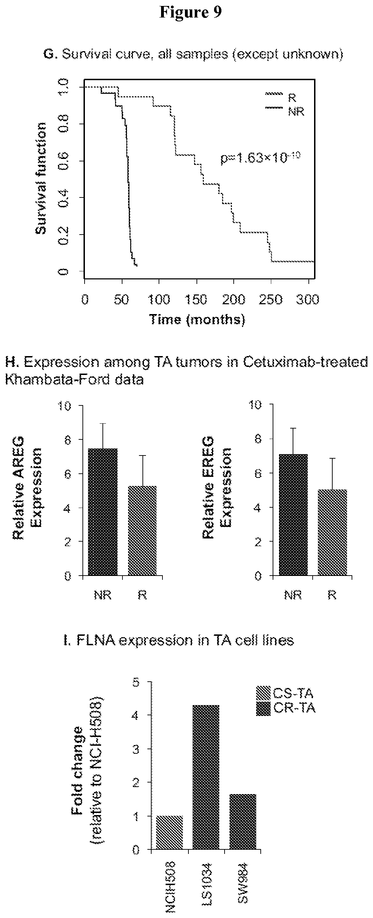

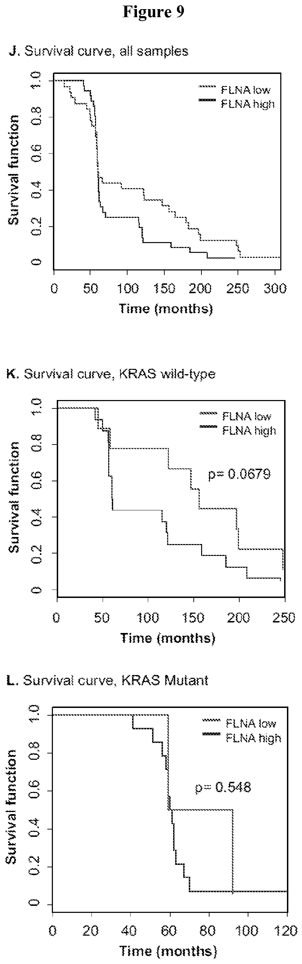

**Figure 9** shows *Cetuximab responses and progression-free survival (PFS) in subtype-specific CRC tumors and cell lines.* A) NMF consensus clustering analysis and cophenetic coefficient for cluster k=2 to k=5 of Khambata-Ford dataset. The dataset is presented after PAM colorectal subtype-specific genes had been mapped. B) Heatmap showing subtypes in GSE28722 (n=125) samples and their associated metastasis information. C) Cetuximab response in cell lines from different CRC subtypes. Data are normalized to vehicle-treatment. Kaplan-Meier Survival curve for patients (Khambata-Ford dataset) that are responsive (R) or non-responsive (NR) to cetuximab based on: D) only TA subtype samples; E) only KRAS wild type samples; F) all samples except those from the TA subtype and unknown (liver contamination); and G) all samples except those that are unknown. H) Differential expression of *AREG* and *EREG* gene predictors between R and NR, as measured by qRT-PCR analysis (data from Khambata, et al). I) qRT-PCR data showing fold change in FLNA expression. Gene expression was normalized to the house-keeping gene, RPL13A. The NCl-H508 is presented as a control. Kaplan-Meier Survival curve (Khambata-Ford dataset) comparing FLNA expression in J) all samples, K) KRAS wild-type samples or L) KRAS mutant samples.

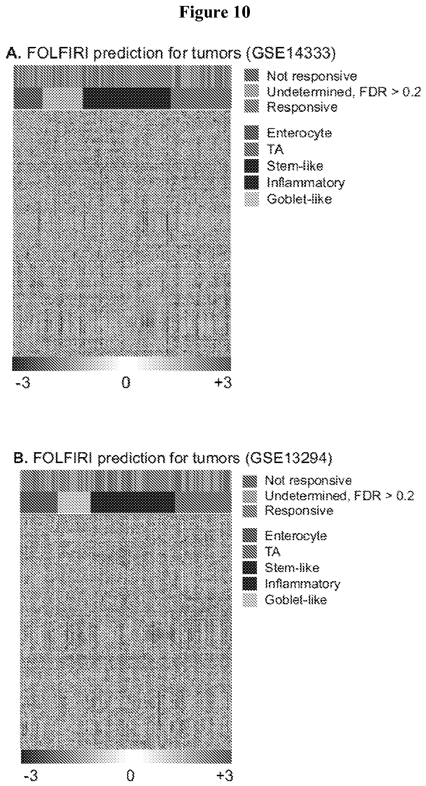

**Figure 10** shows *Subtype-specific FOLFIRI response.* Association of response to FOLFIRI in individual patient samples from the datasets - A) GSE14333 and B) GSE13294 by applying specific signatures using the NTP algorithm.

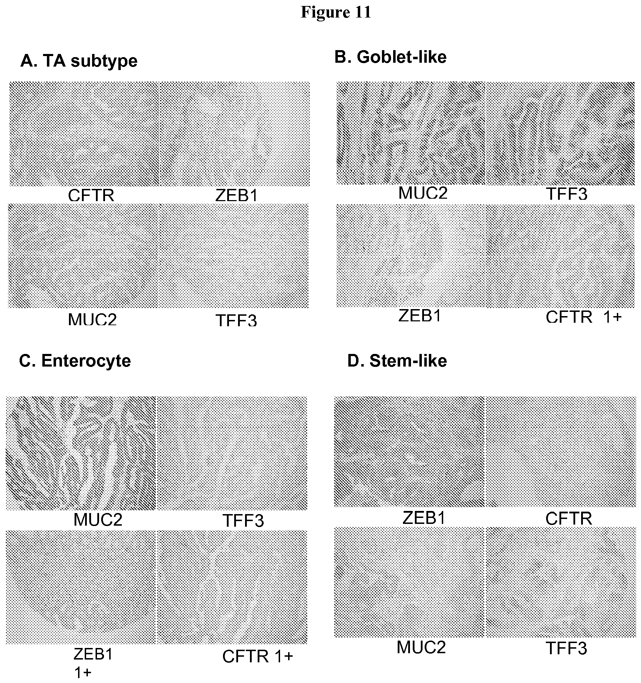

**Figure 11** shows immunohistochemistry markers for TA subtype, Enterocyte subtype, Goblet-like subtype and Stem-like subtype.

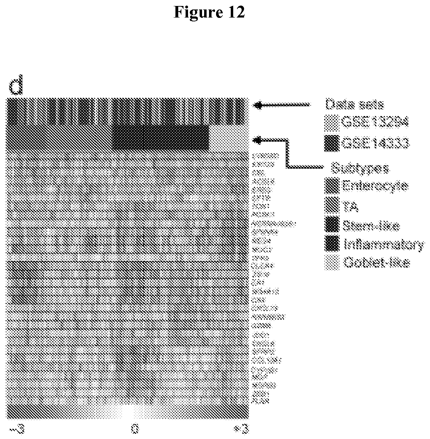

**Figure 12** shows heatmap showing CRCassigner-30 gene signatures.

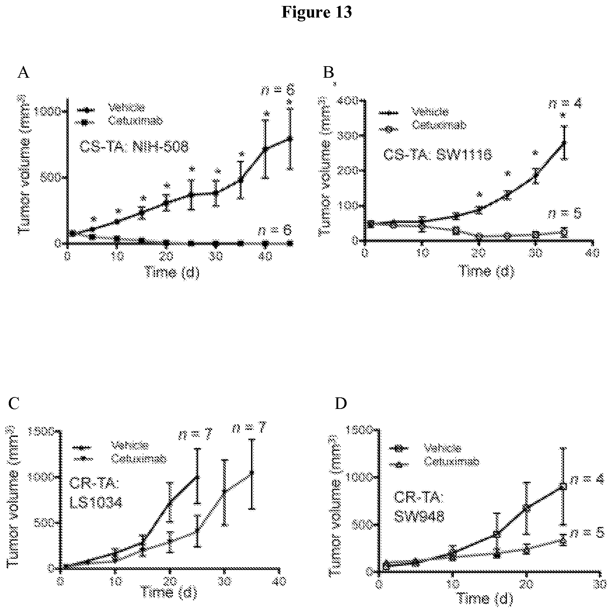

**Figure 13** shows cetuximab response in transit-amplifying sub-type-specific xenograft tumors using the CS-TA cell lines NCI-H508 (A), SW1116 (B) and CR-TA cell lines LS1034 (C), SW948 (D).

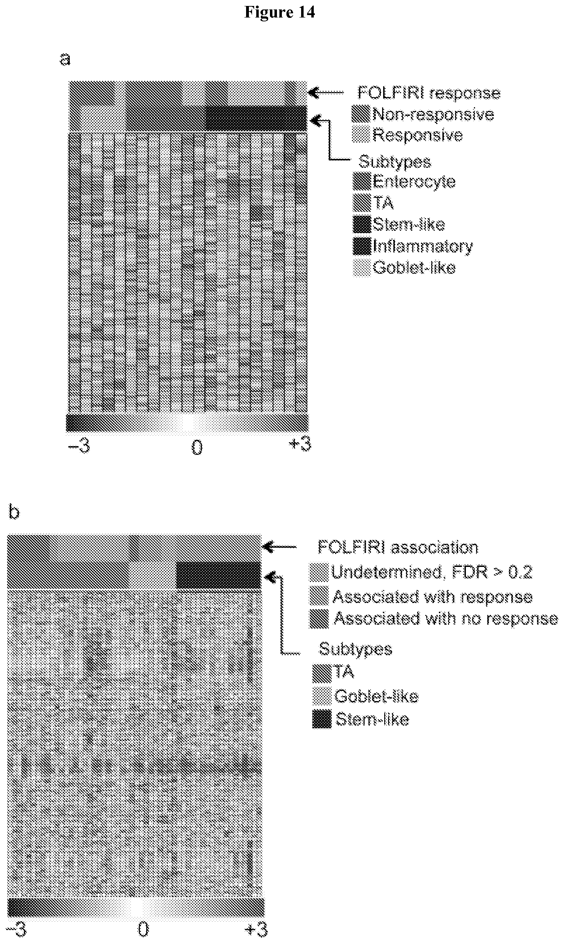

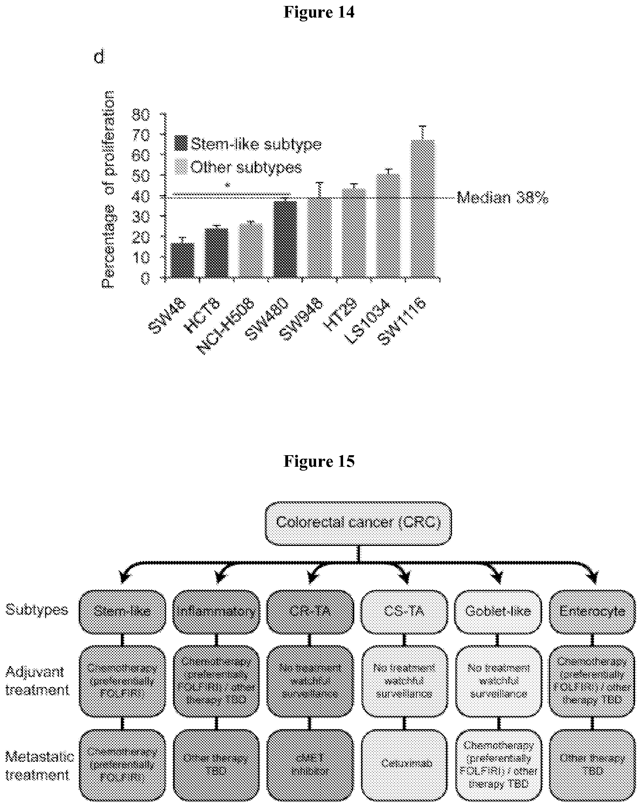

**Figure 14** shows specific response to chemotherapy in CRC subtypes. (A) heatmap showing individual responses of patients with primary CRC (Del Rio data set, n=21) to FOLFIRI treatment and their association with subtypes. Complete and partial responses and stable disease were considered as beneficial response, whereas progressive disease was deemed as no response. (B) heatmap showing association of individual patient CRC responses in the Khambata-Ford data set (metastasis) to FOLFIRI by applying published FOLFIRI response signatures using the NTP algorithm. In these analysis, statistics include only those samples that were predicted with FDR < 0.2. (D) CRC subtype-specific cell line response to FOLFIRI components. Namely, the combination of 5-FU (239 µM) and irinotecan (22,5 µM), plotted as percentage cellular proliferation and normalized to vehicle-treated cells. Error bars represent the s.d. of technical replicates from a representative experiment.
Figure 15 shows subtype guided therapeutic strategies suggested by the association studies.

### DETAILED DESCRIPTION OF THE INVENTION

Although methods and materials similar or equivalent to those described herein can be used in the practice or testing of the present invention, suitable methods and materials are described below. The publications and applications discussed herein are provided solely for their disclosure prior to the filing date of the present application. Nothing herein is to be construed as an admission that the present invention is not entitled to antedate such publication by virtue of prior invention. In addition, the materials, methods, and examples are illustrative only and are not intended to be limiting.

In the case of conflict, the present specification, including definitions, will control. Unless defined otherwise, all technical and scientific terms used herein have the same meaning as is commonly understood by one of skill in art to which the subject matter herein belongs. As used herein, the following definitions are supplied in order to facilitate the understanding of the present invention.

As herein used, "a" or "an" means "at least one" or "one or more."

The term "comprise" is generally used in the sense of include, that is to say permitting the presence of one or more features or components.

The term "disease-free survival (DFS)" in generally means the length of time after primary treatment for a cancer ends that the patient survives without any signs or symptoms of that cancer. In the context of the present invention, the primary treatment is preferably surgical resection of colorectal cancer. In a clinical trial, measuring the disease-free survival is one way to see how well a new treatment works.

"Adjuvant setting" as used herein refers to adjuvant treatment to surgical resection of colorectal cancer, whereas "metastatic setting" refers to treatment used in colorectal cancer recurrence (when colorectal cancer comes back) after surgical resection of colorectal cancer and after a period of time during which the colorectal cancer cannot be detected.

The terms "level of expression" or "expression level" in general are used interchangeably and generally refer to the amount of a polynucleotide or an amino acid product or protein in a biological sample. "Expression" generally refers to the process by which gene-encoded information is converted into the structures present and operating in the cell. Therefore, as used herein, "expression" of a gene may refer to transcription into a polynucleotide, translation into a protein, or even posttranslational modification of the protein. Fragments of the transcribed polynucleotide, the translated protein, or the post-translationally modified protein shall also be regarded as expressed whether they originate from a transcript generated by alternative splicing or a degraded transcript, or from a posttranslational processing of the protein, e.g., by proteolysis. "Expressed genes" include those that are transcribed into a polynucleotide as mRNA and then translated into a protein, and also those that are transcribed into RNA but not translated into a protein (for example, transfer and ribosomal RNAs).

As used herein the terms "subject" or "patient" are well-recognized in the art, and, are used interchangeably herein to refer to a mammal, including dog, cat, rat, mouse, monkey, cow, horse, goat, sheep, pig, camel, and, most preferably, a human. In some embodiments, the subject is a subject in need of treatment or a subject with a disease or disorder, such as colorectal cancer. However, in other embodiments, the subject can be a normal "healthy" subject or a subject who has already undergone a treatment, such as for example a prior surgical resection of colorectal cancer. The term does not denote a particular age or sex. Thus, adult and newborn subjects, whether male or female, are intended to be covered.

Applicants used non-matrix factorization (NMF) based consensus-based unsupervised clustering of CRC gene expression profiles from 1049 patient samples overlaid with corresponding response data to an epidermal growth factor receptor (EGFR)-targeted drug (cetuximab; clinically available) to identify six clinically relevant subtypes of CRC. These subtypes exhibit differential patterns of gene expression (CRC assigner signature) and associate with chemotherapy response and disease-free survival. Surprisingly, these subtypes appear to transcend the microsatellite stable (MSS/MSI) status traditionally used to subtype CRC in terms of predicting response to therapy. Interestingly, these subtypes have phenotypes similar to various normal cell types within the colon-crypt and exhibit differential degrees of stemness. In addition, CRC assigner signatures classified human CRC cell lines and xenograft tumors into four of the five CRC subtypes, which can now better serve as surrogates to analyze drug responsiveness and other parameters of CRC tumor subtypes. Recognizing these subtypes, their apparent cellular phenotypes, and their differential responses to therapy may guide the development of pathway- and mechanism-based therapeutic strategies targeted at specific subtypes of CRC tumors.

Seeking to extend and generalize these findings for CRC, and in particular as a step towards a more specific predictive clinical classification system for CRC, Applicants used consensus-based non-negative matrix factorization (NMF) to cluster two published gene expression datasests (after merging them using the distance weighted discrimination - DWD - method) derived from resected, primary CRC (core dataset, n=445). This approach revealed five distinct molecular genetic subtypes of CRC, with each of the five subtypes exhibiting a high degree of consensus. Because expression profiles obtained from the pooled data were envisioned to be used for identification of gene signatures (and marker gene components thereof) of putative subtypes, silhouette width (a measure of goodness of cluster validation that identifies samples that are the most representative of the subtypes and belong to their own subtype than to any other subtypes) was used to exclude samples situated on the periphery of the five CRC subtype clusters, yielding a 'core' set of 387 CRC samples. To identify markers associated with the 5 subtypes, Applicants used two algorithms - Significance Analysis of Microarrays (SAM, false discovery rate, FDR=0), followed by Prediction Analysis for Microarrays (PAM) - to identify 786 subtype-specific signature genes.

More specifically in order to detect *multiple* subtypes (some of which may represent relatively small fractions of the patient population), the clustering methods require moderately large numbers of samples - more than contained in any one of the individual CRC datasets published to date. With that in mind, Applicants began our analysis by identifying suitable and comparable microarray datasets (see Table 1) and selecting only those datasets that were described in Dalerba, et al, Nature biotechnology 29, 1120-1127 (2011), as not having redundant samples.

**Table 1. Datasets**

| **Dataset** | **Number of samples** | **Nature of samples** |
|---|---|---|
| GSE13294 | 155 | Whole tumor |
| GSE14333 | 290 | Whole tumor |
| GSE12945 | 62 | Microdissected |
| GSE16125 | 48 | Whole tumor |
| GSE20916 | 101 | only tumor samples - removed normal samples |
| GSE20842 | 65 | Whole tumor |
| | | Laser capture microdissected and whole tumor. |
| GSE21510 | 123 | Normal samples removed |
| GSE5851 | | |
| (Khambata-Ford dataset) | 80 | Liver metastases from CRC |
| TCGA dataset | 220 | Whole tumor |
| Rio dataset | 21 | whole tumor |
| GSE28722 | 125 | whole tumor |

Once the datasets were selected, the raw gene expression readouts were either normalized using robust multiarray averaging (RMA) or obtained as processed data from the Applicants, and then pooled using distance weighted discrimination (DWD) after normalizing each dataset to N(0,1). Consensus-based non-negative matrix factorization (NMF) was applied to the pooled data to cluster the samples into the initial set of three and then five CRC subtypes. Although NMF based consensus-based clustering algorithms can be used to detect robust clusters (i.e. clusters that tolerate a moderate degree of outlier contamination in the training set), the identification of genes (or markers) specific to each cluster is somewhat more sensitive to samples representing rare subtypes or samples of indeterminate origin. Therefore, once the clusters (subtypes) were identified using NMF, Applicants used silhouette width to screen out those samples residing on the periphery of the NMF-identified clusters. From there, Applicants applied well-established methods (Significance Analysis of Microarrays; SAM and Prediction Analysis for Microarrays; PAM) to extract biomarkers associated with the screened subtypes. Pooling datasets using **DWD.** When pooling microarray data, one of the main challenges is to pool the microarray datasets in such a way as to compensate for systematic biases (e.g. batch effects) without distorting or collapsing biologically informative and subtype-discriminating structures in the gene expression space. In this respect, a method known as distance weighted discrimination (DWD) was used to pool microarray data and showed that DWD demonstrates superior pooling characteristics when compared to alternative methods such as singular value decomposition (SVD) and Fisher linear discrimination, especially for high-throughput gene expression data in which Applicants must contend with small numbers of samples relative to the number of gene expression readouts (i.e. a high dimensional features space). As a variation on the support vector machine (SVM) approach, DWD is suitable for high dimensional features spaces, but it has the added benefit of minimizing the effects that data artifacts and outliers can have on the batch effect adjustments.

**Unsupervised clustering using consensus-based NMF -** By itself, non-negative matrix factorization is a dimensionality reduction method in which Applicants can attempt to capture the salient functional properties of a high-dimensional gene expression profile using a relatively small number of "metagenes" (defined to be non-negative linear combinations of the expression of individual genes - i.e. a weighted average of gene expression, with each metagene having its own set of weighting coefficients). As with principal component analysis, the familiar gene expression table (samples x genes) is factored into two lower-dimensional matrices except that for NMF the matrix factors are constrained to be purely non-negative values. This 'non-negativity' constraint is believed to more realistically represent the nature of gene expression, in that gene expression is either zero- or positive-valued. In contrast, PCA matrix factors can be either positive- or negative-valued.

Given an arbitrary gene expression table (profile), it is not generally possible to analytically factor the table into two matrix factors. As a consequence, numerical algorithms have been developed to accomplish this by first initializing the two matrices to random values and then iteratively updating the matrices using a search algorithm. There is no guarantee that this search algorithm will converge to a globally optimal factorization, hence one re-runs the algorithm using multiple random initial conditions to see whether the algorithm provides a consistent consistent factorization. At the end of the factorization algorithm, one obtains two lower-dimensional matrices, which when multiplied together will yield an approximation to the original gene expression table. The metagenes correspond to functional properties represented in the original gene expression table and can be viewed as 'anchors' for clustering the samples into subtypes. Specifically, each sample is assigned to a subtype by finding which metagene is most closely aligned with the sample's gene expression profile. Hence each sample is assigned to one and only one cluster.

As explained above, the robustness of clustering can be gauged by repeating the factorization process several times using different random initial conditions for the factorization algorithm. If the factorization is insensitive to the initial conditions of the search algorithm, then any pair of samples will tend to co-cluster irrespective of the initial condition.

In the NMF consensus analysis of the core dataset, Applicants found good consensus for both k=3 and k=5 clusters, suggesting that there was evidence for 5 consensus clusters and hence 5 functional properties in the core dataset

**Removing outliers using silhouette width -** For the purposes of identifying subtype-specific markers, the analysis includes only those samples that are statistically belonging to the core of each of the clusters. Excluding samples with negative silhouette width has been shown minimize the impact of sample outliers on the identification of subtype markers. Accordingly, 58 samples from the original 445 samples dataset were identified as having negative silhouette width and were therefore excluded from the marker identification phase of the analysis.

**Identification of subtype-specific biomarkers using SAM and PAM -** Applicants used a two-step process to identify subtype-specific biomarkers. The first step, identifies the differentially expressed genes and the second step finds subsets of these genes that are associated with specific subtypes. For the first step, Applicants used significance analysis of microarrays (SAM) to identify genes significantly differentially expressed across the 5 subtypes. This is a well established method that looks for large differential gene expression relative to the spread of expression across all genes. Sample permutation is used to estimate false discovery rates (FDR) associated with sets of genes identified as differentially expressed. By adjusting a sensitivity threshold, Δ_{SAM}, users can control the estimated FDR associated with the gene sets. the gene sets. For the analysis, Applicants selected Δ_{SAM} = 12.2, which yielded 786 differentially expressed genes and an FDR of zero. The second step in the process was to match the differentially expressed genes to specific subtypes. For this step, Applicants used the prediction analysis of microarrays (PAM), which is similar in nature to the centroid method recently applied by the TCGA consortium to glioblastoma data, except that PAM eliminates the contribution of genes that differentially express below a specific threshold, Δ_{PAM}, relative to the subtype-specific centroids. Threshold scales, Δ_{PAM} = 2 was chosen after evaluating various Δ_{PAM} values and misclassification errors. Leave out cross validation (LOCV) analysis was then performed to identify a set of genes that had the lowest prediction error. Applicants identified all of the 786 SAM selected genes that had the lowest prediction error of about 7% after PAM and LOCV analysis. The resulting subtype-specific markers (CRCassigner) are listed in Table 2.

Based on genes preferentially expressed in the each subtype, Applicants named the five CRC subtypes:
(1) goblet-like (high mRNA expression of goblet-specific *MUC2* and *TFF3*)*,*
(2) enterocyte (high expression of enterocyte-specific genes),
(3) stem-like (high expression of Wnt signaling targets and myoepithelial/mesenchymal genes and low expression of differentiated markers),
(4) inflammatory (high expression of chemokines and interferon-related genes, see Figure 5), and
(5) transit-amplifying (TA; heterogeneous samples either expressing high or low Wnt-target genes, as described below).

**Table 2. Subtype specific genes and their scores as analyzed by Prediction Analysis of Microarray(PAM); The scores are illustrative only and represent expression profiles (tendencies) of listed genes. Positive score means high expression, negative score means low expression and zero means no change in expression; Threshold used for PAM analysis was 2**

| **Genes** | **SEQ ID NO:** | **Inflammatory** | **Goblet-like** | **Enterocyte** | **TA** | **Stem-like** |
|---|---|---|---|---|---|---|
| SFRP2 | 1 | 0 | -0.2776 | 0 | -0.2306 | 0.879 |
| MGP | 2 | 0 | -0.1888 | 0 | -0.1475 | 0.7035 |
| COL10A1 | 3 | 0 | -0.1584 | -0.1232 | -0.1319 | 0.6845 |
| MSRB3 | 4 | 0 | -0.1956 | 0 | -0.1123 | 0.6763 |
| CYP1B1 | 5 | 0 | -0.0152 | -0.1274 | -0.1626 | 0.6511 |
| FNDC1 | 6 | 0 | -0.1582 | -0.0326 | -0.0494 | 0.6486 |
| SFRP4 | 7 | 0 | -0.0988 | -0.133 | 0 | 0.647 |
| GAS1 | 8 | 0.0412 | -0.15 | -0.0838 | -0.2186 | 0.6455 |
| CCDC80 | 9 | 0 | -0.1613 | 0 | -0.1424 | 0.6364 |
| SPOCK1 | 10 | 0 | -0.152 | -0.0326 | -0.1235 | 0.6318 |
| THBS2 | 11 | 0 | -0.1923 | -0.148 | -0.0586 | 0.6214 |
| MFAP5 | 12 | 0 | -0.1392 | 0 | -0.0635 | 0.6137 |
| ASPN | 13 | 0 | -0.151 | -0.0018 | -0.0499 | 0.6115 |
| TNS1 | 14 | 0 | -0.2049 | 0 | -0.1083 | 0.6071 |
| TAGLN | 15 | 0 | -0.1607 | 0 | -0.1298 | 0.6043 |
| COMP | 16 | 0 | 0 | -0.1835 | 0 | 0.5813 |
| NTM | 17 | 0 | -0.1099 | -0.119 | -0.0708 | 0.5714 |
| HOPX | 18 | 0 | -0.1438 | -0.0138 | -0.135 | 0.5637 |
| AEBP1 | 19 | 0 | -0.0861 | -0.0086 | -0.1081 | 0.5552 |
| FRMD6 | 20 | 0 | -0.1576 | 0 | -0.168 | 0.5545 |
| PLN | 21 | 0 | -0.1089 | 0 | -0.1183 | 0.5532 |
| FBN1 | 22 | 0 | -0.149 | 0 | -0.1139 | 0.5529 |
| COL11A1 | 23 | 0 | -0.1542 | -0.2209 | -0.026 | 0.5523 |
| ANTXR1 | 24 | 0 | -0.1075 | 0 | -0.0794 | 0.5469 |
| MIR100HG | 25 | 0 | -0.0574 | 0 | -0.0351 | 0.543 |
| PCDH7 | 26 | 0 | -0.0985 | 0 | -0.0669 | 0.5417 |
| DDR2 | 27 | 0 | -0.1251 | 0 | -0.1375 | 0.5383 |
| MYL9 | 28 | 0 | -0.2042 | 0 | 0 | 0.5359 |
| FERMT2 | 29 | 0 | -0.1167 | 0 | -0.0515 | 0.5291 |
| VCAN | 30 | 0 | -0.0782 | 0 | -0.0715 | 0.5162 |
| CDH11 | 31 | 0 | 0 | 0 | -0.0454 | 0.5127 |
| SYNPO2 | 32 | -0.0719 | -0.1083 | 0 | -0.0712 | 0.5068 |
| SULF1 | 33 | 0 | -0.2186 | 0 | -0.0949 | 0.5062 |
| FAP | 34 | 0 | -0.0265 | -0.0647 | -0.1393 | 0.5032 |
| COL3A1 | 35 | 0 | -0.0794 | -0.0304 | -0.1117 | 0.5029 |
| CTHRC1 | 36 | 0 | -0.1881 | -0.0265 | -0.0779 | 0.5023 |
| ADAM12 | 37 | 0 | -0.0799 | -0.1009 | -0.1043 | 0.5004 |
| COL1A2 | 38 | 0 | -0.079 | 0 | -0.0861 | 0.5003 |
| TIMP2 | 39 | 0 | -0.1207 | 0 | -0.1334 | 0.4964 |
| PRRX1 | 40 | 0.0088 | -0.117 | -0.0297 | -0.1347 | 0.4919 |
| BGN | 41 | 0 | -0.1115 | -0.0389 | -0.0659 | 0.4905 |
| GLT8D2 | 42 | 0 | -0.0607 | 0 | -0.0853 | 0.4893 |
| DCN | 43 | 0 | -0.1514 | 0 | -0.1093 | 0.4874 |
| FABP4 | 44 | 0 | -0.0096 | 0 | -0.0303 | 0.4815 |
| FBLN1 | 45 | 0 | -0.1223 | 0 | -0.0202 | 0.4789 |
| EFEMP1 | 46 | 0 | -0.105 | 0 | -0.0602 | 0.4771 |
| VGLL3 | 47 | 0 | -0.0853 | -0.0418 | -0.0742 | 0.4769 |
| SPARC | 48 | 0 | -0.1186 | 0 | -0.0553 | 0.4726 |
| ITGBL1 | 49 | 0 | -0.0379 | -0.1163 | 0 | 0.4715 |
| AKAP12 | 50 | 0 | -0.1005 | 0 | -0.0313 | 0.4705 |
| INHBA | 51 | 0 | -0.0115 | -0.0995 | -0.0605 | 0.4705 |
| COL5A2 | 52 | 0 | -0.1055 | -0.031 | -0.0409 | 0.4672 |
| RAB31 | 53 | 0.0435 | -0.1527 | 0 | -0.2026 | 0.4666 |
| ISLR | 54 | 0 | -0.1724 | 0 | 0 | 0.4604 |
| STON1 | 55 | 0 | -0.0541 | 0 | 0 | 0.4559 |
| NOX4 | 56 | 0 | -0.0082 | -0.1679 | -0.0011 | 0.4553 |
| LOX | 57 | 0.0199 | -0.1362 | 0 | -0.1302 | 0.451 |
| POSTN | 58 | 0.0134 | -0.1739 | 0 | -0.1652 | 0.4507 |
| ECM2 | 59 | 0 | 0 | -0.1134 | 0 | 0.4489 |
| LHFP | 60 | 0 | -0.0428 | 0 | -0.0242 | 0.4474 |
| SERPINF1 | 61 | 0 | -0.0925 | 0 | -0.0896 | 0.4419 |
| NNMT | 62 | 0 | 0 | 0 | -0.2092 | 0.4393 |
| PTGIS | 63 | 0 | -0.045 | 0 | 0 | 0.4345 |
| MYLK | 64 | 0 | -0.1502 | 0 | -0.0126 | 0.4325 |
| MAP1B | 65 | 0 | 0 | 0 | 0 | 0.4315 |
| CALD1 | 66 | 0 | -0.0892 | 0 | -0.045 | 0.4304 |
| GREM1 | 67 | 0 | -0.1838 | 0 | -0.2011 | 0.4289 |
| COL5A1 | 68 | 0 | -0.0193 | 0 | -0.0705 | 0.4235 |
| CNN1 | 69 | 0 | -0.0372 | 0 | -0.0098 | 0.4179 |
| TIMP3 | 70 | 0 | -0.3013 | 0 | 0 | 0.4153 |
| COL6A2 | 71 | 0 | -0.0842 | 0 | -0.1669 | 0.4137 |
| ZEB1 | 72 | 0 | -0.0686 | 0 | 0 | 0.4121 |
| PPAPDC1A | 73 | 0 | 0 | -0.1524 | 0 | 0.408 |
| OLFML2B | 74 | 0 | -0.0094 | -0.0578 | -0.0358 | 0.406 |
| HTRA1 | 75 | 0 | 0 | 0 | -0.0049 | 0.4052 |
| CXCL12 | 76 | 0 | -0.066 | 0 | -0.0859 | 0.4029 |
| DPYSL3 | 77 | 0 | 0 | -0.1132 | 0 | 0.4021 |
| PDGFC | 78 | 0 | 0 | 0 | -0.0277 | 0.401 |
| COL6A3 | 79 | 0 | -0.1016 | 0 | -0.0802 | 0.4004 |
| COL1A1 | 80 | 0 | -0.1083 | 0 | -0.0322 | 0.3978 |
| MYH11 | 81 | -0.0744 | -0.0394 | 0 | 0 | 0.3941 |
| AOC3 | 82 | 0 | -0.041 | 0 | -0.0664 | 0.3934 |
| SPARCL1 | 83 | 0 | -0.0965 | 0 | -0.1647 | 0.3929 |
| COL12A1 | 84 | 0 | 0 | 0 | -0.0187 | 0.3927 |
| GPNMB | 85 | 0.2398 | -0.1173 | 0 | -0.2938 | 0.3894 |
| BCAT1 | 86 | 0.1813 | -0.1075 | -0.1043 | -0.1465 | 0.3875 |
| PHLDB2 | 87 | 0 | 0 | 0 | -0.1801 | 0.3844 |
| SERPING1 | 88 | 0.1257 | -0.1389 | 0 | -0.2161 | 0.3804 |
| TPM2 | 89 | 0 | -0.1117 | 0 | 0 | 0.3803 |
| TGFB1I1 | 90 | 0 | 0 | 0 | -0.0126 | 0.3768 |
| MITF | 91 | 0 | 0 | 0 | -0.1126 | 0.3768 |
| GPC6 | 92 | 0 | -0.1114 | 0 | -0.055 | 0.3739 |
| NEXN | 93 | 0.0814 | -0.164 | 0 | -0.1467 | 0.3736 |
| MMP2 | 94 | 0 | -0.0197 | 0 | -0.0948 | 0.3709 |
| FAM129A | 95 | 0.1134 | -0.1219 | 0 | -0.2347 | 0.3671 |
| ADAMTS2 | 96 | 0.0641 | -0.1371 | 0 | -0.1016 | 0.3646 |
| FIBIN | 97 | 0 | 0 | -0.0298 | 0 | 0.3634 |
| TMEM47 | 98 | 0 | -0.1286 | 0 | 0 | 0.3621 |
| IGFBP5 | 99 | 0 | -0.2048 | 0 | -0.0485 | 0.3611 |
| TNFAIP6 | 100 | 0.2379 | -0.1454 | -0.0983 | -0.149 | 0.3595 |
| MXRA5 | 101 | 0 | -0.0162 | -0.0296 | -0.001 | 0.3594 |
| ARL4C | 102 | 0.1305 | -0.0848 | -0.0129 | -0.1572 | 0.359 |
| EPYC | 103 | 0 | 0 | -0.0864 | 0 | 0.3551 |
| COL15A1 | 104 | 0 | -0.0768 | 0 | -0.147 | 0.3536 |
| LMOD1 | 105 | 0 | 0 | 0 | 0 | 0.351 |
| FN1 | 106 | 0 | -0.1868 | -0.062 | 0 | 0.351 |
| DPT | 107 | 0 | -0.016 | 0 | 0 | 0.3467 |
| GNB4 | 108 | 0.159 | -0.158 | 0 | -0.1867 | 0.3441 |
| TWIST1 | 109 | 0 | -0.0276 | 0 | 0 | 0.3422 |
| SDC2 | 110 | 0 | -0.0673 | 0 | 0 | 0.3405 |
| FLRT2 | 111 | 0 | -0.0275 | 0 | 0 | 0.3377 |
| LOXL1 | 112 | 0 | 0 | -0.0073 | -0.0971 | 0.3372 |
| FHL1 | 113 | -0.1256 | 0 | 0 | -0.0116 | 0.3365 |
| MAB21L2 | 114 | 0 | -0.0568 | 0 | 0 | 0.3358 |
| SSPN | 115 | 0 | 0 | 0 | -0.0433 | 0.3358 |
| CTSK | 116 | 0 | -0.074 | 0 | -0.0411 | 0.3336 |
| WWTR1 | 117 | 0 | -0.1856 | 0 | -0.0028 | 0.3325 |
| CYBRD1 | 118 | 0 | -0.0268 | 0 | -0.0662 | 0.329 |
| CLIP4 | 119 | 0 | -0.0923 | 0 | -0.1143 | 0.3283 |
| ZEB2 | 120 | 0 | -0.1273 | 0 | -0.1365 | 0.3267 |
| SYNM | 121 | 0 | -0.0164 | 0 | 0 | 0.3223 |
| SNAI2 | 122 | 0 | -0.0348 | 0 | -0.0455 | 0.3213 |
| DES | 123 | 0 | 0 | 0 | 0 | 0.3147 |
| IGF1 | 124 | -0.014 | 0 | 0 | 0 | 0.3133 |
| TNC | 125 | 0 | -0.1062 | 0 | -0.1138 | 0.3128 |
| GUCY1A3 | 126 | 0 | -0.1277 | 0 | -0.0191 | 0.3077 |
| GULP1 | 127 | 0 | -0.1147 | 0 | 0 | 0.3058 |
| TMEM45A | 128 | 0.0313 | 0 | -0.0696 | -0.2556 | 0.3047 |
| C3 | 129 | 0 | -0.0565 | 0 | -0.1239 | 0.3027 |
| VCAM1 | 130 | 0.0117 | -0.0382 | 0 | -0.1361 | 0.3024 |
| AHNAK2 | 131 | 0 | 0 | -0.0576 | -0.0272 | 0.3022 |
| ACTG2 | 132 | 0 | -0.0303 | 0 | 0 | 0.3016 |
| KAL1 | 133 | 0 | 0 | -0.0417 | 0 | 0.2927 |
| FLNA | 134 | 0 | -0.083 | 0 | 0 | 0.2923 |
| CYR61 | 135 | 0 | 0 | 0 | -0.1072 | 0.2894 |
| NR3C1 | 136 | 0.0048 | -0.1514 | 0 | -0.1891 | 0.2873 |
| DSE | 137 | 0.1549 | -0.0464 | 0 | -0.1602 | 0.2871 |
| PMP22 | 138 | 0 | 0 | 0 | -0.1767 | 0.2832 |
| RBMS1 | 139 | 0 | -0.262 | 0 | 0 | 0.2827 |
| SMARCA1 | 140 | 0 | 0 | -0.0477 | 0 | 0.2797 |
| MAFB | 141 | 0.2127 | -2.00E-04 | 0 | -0.2472 | 0.2746 |
| MAF | 142 | 0 | -0.1091 | 0 | -0.0921 | 0.2734 |
| QKI | 143 | 0.0273 | -0.1498 | 0 | -0.0453 | 0.2713 |
| MMP11 | 144 | 0 | -0.0176 | 0 | 0 | 0.265 |
| CD109 | 145 | 0.1778 | 0 | -0.0866 | -0.1737 | 0.262 |
| SRPX | 146 | 0 | 0 | 0 | -0.045 | 0.2609 |
| EDNRA | 147 | 0 | -0.1215 | 0 | 0 | 0.2602 |
| THBS1 | 148 | 0 | -0.1967 | 0 | 0 | 0.2592 |
| SLC2A3 | 149 | 0.1804 | -0.0548 | -0.0582 | -0.1109 | 0.2585 |
| CHRDL1 | 150 | 0 | -0.0152 | 0 | 0 | 0.2566 |
| APOD | 151 | -0.0583 | 0 | 0 | 0 | 0.2543 |
| RUNX2 | 152 | 0 | 0 | -0.0489 | 0 | 0.2543 |
| COL14A1 | 153 | 0 | 0 | 0 | 0 | 0.2536 |
| GPX3 | 154 | 0 | 0 | 0 | -0.0397 | 0.2519 |
| UBE2E2 | 155 | 0.0158 | 0 | 0 | -0.0714 | 0.2511 |
| GEM | 156 | 0 | -0.0542 | 0 | 0 | 0.2508 |
| LY96 | 157 | 0.24 | -0.0506 | 0 | -0.2613 | 0.2481 |
| FAM126A | 158 | 0 | -0.0339 | 0 | 0 | 0.2475 |
| ANK2 | 159 | 0 | 0 | 0 | 0 | 0.2474 |
| CTGF | 160 | 0 | -0.0021 | 0 | -0.014 | 0.2453 |
| SORBS1 | 161 | -0.1716 | -0.1959 | 0 | 0.0931 | 0.2448 |
| RGS2 | 162 | 0.1026 | 0 | 0 | -0.2979 | 0.2431 |
| C1S | 163 | 0 | 0 | 0 | -0.0506 | 0.2405 |
| CD36 | 164 | 0 | 0 | 0 | -0.0184 | 0.2401 |
| NRP1 | 165 | 0.1361 | -0.032 | 0 | -0.1398 | 0.2378 |
| KLHL5 | 166 | 0 | -0.0881 | 0 | 0 | 0.2345 |
| CFH | 167 | 0 | 0 | 0 | -0.1274 | 0.2341 |
| SPP1 | 168 | 0.2055 | 0 | -0.089 | -0.161 | 0.2331 |
| RDX | 169 | 0 | -0.2345 | 0 | 0 | 0.23 |
| ADH1B | 170 | -0.0944 | -0.047 | 0.3588 | -0.1223 | 0.2296 |
| CCL2 | 171 | 0 | -0.0809 | 0 | -0.1288 | 0.2286 |
| BASP1 | 172 | 0.0223 | -0.0057 | 0 | -0.1244 | 0.2276 |
| ID4 | 173 | -0.0998 | 0 | 0 | 0 | 0.2267 |
| MDFIC | 174 | 0 | 0 | 0 | -0.0892 | 0.2238 |
| RASSF8 | 175 | 0 | -0.0625 | 0 | 0 | 0.2183 |
| C11 orf96 | 176 | 0 | -0.0504 | 0 | -0.0452 | 0.2129 |
| TSPAN2 | 177 | 0 | -0.0929 | 0 | 0 | 0.2064 |
| MEIS2 | 178 | 0.1239 | 0 | 0 | -0.2462 | 0.2042 |
| AMIGO2 | 179 | 0 | 0 | -0.1191 | 0 | 0.199 |
| SHISA2 | 180 | 0 | 0 | 0 | 0 | 0.1975 |
| APOE | 181 | 0.3899 | -0.0674 | -0.1223 | -0.1748 | 0.1969 |
| C5AR1 | 182 | 0.0945 | -0.0012 | -0.0172 | -0.055 | 0.1913 |
| ZCCHC24 | 183 | -0.0876 | -0.2512 | 0 | 0.0882 | 0.1825 |
| MS4A7 | 184 | 0.2031 | -0.0117 | 0 | -0.2421 | 0.1814 |
| DPYD | 185 | 0.3389 | -0.1117 | 0 | -0.3262 | 0.1803 |
| PLXNC1 | 186 | 0.1817 | 0 | 0 | -0.2341 | 0.1757 |
| CFL2 | 187 | 0 | 0 | 0 | -0.0022 | 0.1749 |
| ITGAM | 188 | 0.1167 | 0 | -0.0376 | -0.0827 | 0.1721 |
| SERPINE1 | 189 | 0 | 0 | 0 | 0 | 0.1697 |
| SFRP1 | 190 | 0 | 0 | 0 | 0 | 0.1696 |
| DACT1 | 191 | 0 | 0.0014 | -0.0301 | 0 | 0.1685 |
| CLEC2B | 192 | 0.293 | -0.0682 | 0 | -0.2304 | 0.1652 |
| PAPPA | 193 | 0 | 0 | 0 | 0 | 0.1613 |
| APOC1 | 194 | 0.2984 | -0.1191 | -0.0933 | -0.0629 | 0.1551 |
| RORA | 195 | 0 | -0.1148 | 0 | 0 | 0.1522 |
| CAV2 | 196 | 0.0124 | 0 | 0 | -0.1146 | 0.1474 |
| HDGFRP3 | 197 | 0 | -0.1806 | 0 | 0 | 0.1447 |
| CCL18 | 198 | 0.4083 | -0.1493 | 0 | -0.2446 | 0.1444 |
| ADAMTS1 | 199 | 0 | -0.0193 | 0 | -0.0499 | 0.1373 |
| TBC1D9 | 200 | 0 | -0.1026 | 0 | 0 | 0.1353 |
| KCNMA1 | 201 | 0 | 0 | 0 | -0.0697 | 0.1342 |
| SPON1 | 202 | 0 | 0 | 0.0617 | -0.3125 | 0.1331 |
| MS4A4A | 203 | 0.2638 | -0.0508 | 0 | -0.2333 | 0.1295 |
| PDZRN3 | 204 | 0 | 0 | 0 | 0 | 0.1203 |
| DMD | 205 | -0.2224 | -0.0806 | 0 | 0.1747 | 0.1199 |
| ABI3BP | 206 | 0 | 0 | 0.0262 | 0 | 0.1152 |
| CD163 | 207 | 0.3286 | 0 | 0 | -0.2196 | 0.1121 |
| ABCA8 | 208 | -0.0414 | -0.0288 | 0.1135 | 0 | 0.1119 |
| TYROBP | 209 | 0.263 | 0 | 0 | -0.1942 | 0.1082 |
| FCGR1B | 210 | 0.3114 | -0.059 | -0.1141 | -0.0594 | 0.1054 |
| NCF2 | 211 | 0.303 | 0 | 0 | -0.158 | 0.0996 |
| FCER1G | 212 | 0.3583 | -0.0311 | 0 | -0.2246 | 0.0924 |
| CXCR4 | 213 | 0.2815 | 0 | 0 | -0.3503 | 0.0909 |
| FPR3 | 214 | 0.1715 | 0 | 0 | -0.082 | 0.0885 |
| LAPTM5 | 215 | 0.2666 | 0 | 0 | -0.1998 | 0.0838 |
| PLA1A | 216 | 0 | -0.0425 | -0.0425 | 0 | 0.0837 |
| ANXA1 | 217 | 0.1687 | 0 | -0.0087 | -0.2138 | 0.0831 |
| STC1 | 218 | 0.0323 | 0 | -0.0956 | 0 | 0.083 |
| BEX4 | 219 | 0 | -0.0578 | 0 | 0 | 0.0795 |
| WASF3 | 220 | -0.0237 | -0.0554 | 0 | 0 | 0.0787 |
| SCRN1 | 221 | 0 | -0.0812 | 0 | 0.0666 | 0.0756 |
| CHI3L1 | 222 | 0.0141 | -0.1499 | 0 | 0 | 0.0754 |
| PMEPA1 | 223 | -0.2985 | 0 | 0 | 0.2167 | 0.074 |
| CPE | 224 | -0.2802 | 0 | 0 | 0 | 0.074 |
| SOCS3 | 225 | 0.0681 | 0 | 0 | -0.0698 | 0.0668 |
| BHLHE41 | 226 | 0 | 0 | 0 | -0.1473 | 0.0667 |
| EVI2A | 227 | 0.2373 | 0 | 0 | -0.1574 | 0.0546 |
| ALOX5AP | 228 | 0.1023 | 0 | 0 | -0.092 | 0.0477 |
| CD14 | 229 | 0.2155 | 0 | 0 | -0.2552 | 0.0451 |
| TREM1 | 230 | 0.103 | 0 | -0.0561 | 0 | 0.0447 |
| ETV1 | 231 | 0 | 0 | -0.0593 | -0.0322 | 0.0431 |
| TNFSF13B | 232 | 0.4332 | 0 | -0.0281 | -0.1973 | 0.0427 |
| ITGB2 | 233 | 0.3009 | 0 | 0 | -0.1837 | 0.0382 |
| SLAMF8 | 234 | 0.3982 | 0 | -0.0215 | -0.1979 | 0.0355 |
| CLEC7A | 235 | 0.2954 | -0.0099 | -0.0172 | -0.0839 | 0.0343 |
| KLF9 | 236 | 0 | 0 | 0 | -0.1643 | 0.0338 |
| ENPP2 | 237 | 0 | 0 | 0 | -0.1075 | 0.0326 |
| NRXN3 | 238 | -0.0085 | 0 | -0.0305 | 0.0889 | 0.0311 |
| RGS1 | 239 | 0.1966 | -0.0132 | 0 | -0.1633 | 0.0311 |
| KRT80 | 240 | 0 | 0 | -0.2292 | 0.0388 | 0.0274 |
| TPSAB1 | 241 | 0 | 0 | 0.1991 | -0.061 | 0.0274 |
| SERPINE2 | 242 | -0.1377 | 0 | 0 | 0.1315 | 0.027 |
| KCTD12 | 243 | 0.0303 | 0 | 0 | -0.3168 | 0.0255 |
| S100A8 | 244 | 0.2099 | 0 | 0 | -0.1567 | 0.023 |
| CDKN2B | 245 | 0 | -0.1792 | 0.3967 | -0.1245 | 0.0219 |
| FCGR3B | 246 | 0.2736 | 0 | 0 | -0.1038 | 0.0214 |
| MS4A6A | 247 | 0.168 | 0 | 0 | -0.1139 | 0.02 |
| CPA3 | 248 | 0 | 0 | 0.1955 | -0.0899 | 0.0185 |
| C1QC | 249 | 0.3111 | 0 | 0 | -0.1887 | 0.0149 |
| TPSB2 | 250 | 0 | 0 | 0.1966 | -0.0626 | 0.014 |
| GXYLT2 | 251 | 0 | 0 | -0.0385 | 0.0903 | 0.0126 |
| SRPX2 | 252 | -0.1793 | -0.2719 | 0 | 0.3665 | 0.0107 |
| HSPA6 | 253 | 0.1683 | 0 | -0.165 | 0 | 0.0099 |
| ANO1 | 254 | 0.0451 | 0.1479 | -0.0344 | -0.2397 | 0.0081 |
| EPDR1 | 255 | -0.3884 | -0.1589 | 0 | 0.4415 | 0.0075 |
| HCLS1 | 256 | 0.2762 | 0 | 0 | -0.2442 | 0.0063 |
| APOLD1 | 257 | -0.1946 | -0.0759 | 0 | 0.2333 | 0.0053 |
| BCL2A1 | 258 | 0.3177 | 0 | 0 | -0.1648 | 0.0025 |
| SRGN | 259 | 0.2157 | 0 | 0 | -0.2038 | 5.00E-04 |
| LY6G6D | 260 | -0.4422 | -0.2319 | 0 | 0.6117 | 0 |
| EREG | 261 | -0.1965 | -0.5456 | 0 | 0.5013 | 0 |
| CEL | 262 | -0.2926 | -0.2292 | 0 | 0.4797 | 0 |
| KRT23 | 263 | -0.3572 | -0.1254 | 0 | 0.4685 | 0 |
| ACSL6 | 264 | -0.2303 | -0.1453 | 0 | 0.4613 | 0 |
| QPRT | 265 | -0.4367 | 0 | 0 | 0.4572 | 0 |
| AXIN2 | 266 | -0.48 | 0 | 0 | 0.436 | 0 |
| ABAT | 267 | -0.3786 | -0.1499 | 0 | 0.4343 | 0 |
| FARP1 | 268 | -0.3058 | -0.0872 | 0 | 0.4285 | 0 |
| CELP | 269 | -0.2018 | -0.1363 | 0 | 0.4263 | 0 |
| C13orf18 | 270 | -0.4156 | -0.1525 | 0 | 0.426 | 0 |
| HUNK | 271 | -0.2609 | 0 | 0 | 0.4218 | 0 |
| PLCB4 | 272 | -0.4897 | 0 | 0 | 0.4136 | 0 |
| APCDD1 | 273 | -0.3273 | 0 | 0 | 0.4095 | 0 |
| RNF43 | 274 | -0.3117 | 0 | 0 | 0.4086 | 0 |
| ASCL2 | 275 | -0.1967 | 0 | 0 | 0.4035 | 0 |
| CHN2 | 276 | -0.3353 | 0 | 0 | 0.3934 | 0 |
| AREG | 277 | -0.1461 | -0.2009 | 0 | 0.3823 | 0 |
| PAH | 278 | -0.1139 | 0 | 0 | 0.3687 | 0 |
| NR1I2 | 279 | -0.3552 | 0 | 0 | 0.3667 | 0 |
| FREM2 | 280 | -0.1792 | 0 | 0 | 0.3607 | 0 |
| CTTNBP2 | 281 | -0.3476 | 0 | 0 | 0.3606 | 0 |
| GNG4 | 282 | -0.2338 | -0.1537 | 0 | 0.3511 | 0 |
| PRR15 | 283 | -0.2217 | 0 | 0 | 0.3502 | 0 |
| LOC100288092 | 284 | -0.1822 | -0.0349 | 0 | 0.3502 | 0 |
| CFTR | 285 | -0.2225 | 0 | 0 | 0.3464 | 0 |
| BCL11A | 286 | -0.201 | 0 | 0 | 0.3452 | 0 |
| ERP27 | 287 | -0.1786 | 0 | 0 | 0.3432 | 0 |
| PLA2G12B | 288 | -0.115 | -0.0374 | 0 | 0.3421 | 0 |
| DACH1 | 289 | -0.5464 | 0.0663 | 0 | 0.3403 | 0 |
| SPIN3 | 290 | -0.327 | 0 | -0.0258 | 0.3389 | 0 |
| GGH | 291 | -0.0849 | 0 | 0 | 0.3381 | 0 |
| ACE2 | 292 | -0.2197 | -0.0697 | 0 | 0.3294 | 0 |
| PTPRO | 293 | -0.338 | 0 | 0 | 0.3288 | 0 |
| DPEP1 | 294 | -0.2676 | 0 | 0 | 0.327 | 0 |
| PROX1 | 295 | -0.1874 | 0 | 0 | 0.3247 | 0 |
| ZNRF3 | 296 | -0.1387 | 0 | -0.0483 | 0.3199 | 0 |
| CAB39L | 297 | -0.2759 | -0.0576 | 0 | 0.3197 | 0 |
| LRRC2 | 298 | -0.1842 | 0 | 0 | 0.3162 | 0 |
| REEP1 | 299 | -0.23 | -0.1301 | 0 | 0.312 | 0 |
| CYP2B6 | 300 | -0.1027 | 0 | 0 | 0.2973 | 0 |
| LAMP2 | 301 | -0.1476 | 0 | 0 | 0.2972 | 0 |
| PPP1R14C | 302 | -0.2014 | 0 | 0 | 0.2909 | 0 |
| CBX5 | 303 | -0.245 | 0 | 0 | 0.2881 | 0 |
| NOX1 | 304 | -0.2615 | 0 | 0 | 0.2878 | 0 |
| SLC22A3 | 305 | -0.1052 | 0 | -0.0938 | 0.2869 | 0 |
| TCFL5 | 306 | 0 | -0.0413 | 0 | 0.2846 | 0 |
| SATB2 | 307 | -0.1555 | -0.0645 | 0 | 0.283 | 0 |
| AREGB | 308 | -0.0648 | -0.0127 | 0 | 0.2791 | 0 |
| AZGP1 | 309 | -0.0255 | 0 | 0 | 0.2784 | 0 |
| TMEM150C | 310 | -0.231 | 0 | 0 | 0.2739 | 0 |
| LOC647979 | 311 | -0.1853 | 0 | 0 | 0.269 | 0 |
| LOC100128822 | 312 | -0.1377 | 0 | 0 | 0.2689 | 0 |
| CES1 | 313 | -0.1337 | -0.0587 | 0 | 0.2642 | 0 |
| PTCH1 | 314 | -0.232 | 0 | 0 | 0.263 | 0 |
| PRSS23 | 315 | -0.197 | 0 | -0.0032 | 0.262 | 0 |
| LOC729680 | 316 | 0 | 0 | 0 | 0.2589 | 0 |
| ZBTB10 | 317 | -0.2166 | -0.0677 | 0 | 0.2584 | 0 |
| PRAP1 | 318 | -0.2589 | 0 | 0 | 0.2571 | 0 |
| PM20D2 | 319 | -0.0216 | -0.096 | 0 | 0.2469 | 0 |
| SESN1 | 320 | -0.1806 | 0 | -0.0261 | 0.2444 | 0 |
| QPCT | 321 | -0.1104 | 0 | 0 | 0.2429 | 0 |
| ATP10B | 322 | -0.2544 | 0 | 0 | 0.2413 | 0 |
| ELAVL2 | 323 | 0 | 0 | 0 | 0.2408 | 0 |
| CLDN1 | 324 | 0 | 0 | -0.0731 | 0.2382 | 0 |
| C12orf66 | 325 | -0.0349 | 0 | 0 | 0.2374 | 0 |
| ST6GAL1 | 326 | 0 | -0.0604 | 0 | 0.236 | 0 |
| CTSL2 | 327 | 0 | 0 | 0 | 0.2354 | 0 |
| COL9A3 | 328 | -0.062 | 0 | 0 | 0.2352 | 0 |
| FGGY | 329 | -0.1413 | 0 | 0 | 0.235 | 0 |
| GSPT2 | 330 | -0.2263 | 0 | 0 | 0.2326 | 0 |
| KIAA1704 | 331 | -0.0637 | -0.0524 | 0 | 0.2324 | 0 |
| CYP4F3 | 332 | -0.0075 | -0.0151 | 0 | 0.2295 | 0 |
| SLC19A3 | 333 | -0.0222 | 0 | 0 | 0.2258 | 0 |
| FLJ22763 | 334 | -0.2682 | 0 | 0 | 0.2222 | 0 |
| DNAJC6 | 335 | -0.0255 | 0 | 0 | 0.2166 | 0 |
| FOXQ1 | 336 | -0.0192 | 0 | -0.219 | 0.2165 | 0 |
| MIR374AHG | 337 | -0.2713 | 0 | 0 | 0.2151 | 0 |
| CDCA7 | 338 | 0 | 0 | 0 | 0.2142 | 0 |
| MACC1 | 339 | -0.0934 | 0 | 0 | 0.2136 | 0 |
| OXGR1 | 340 | -0.0511 | 0 | 0 | 0.2133 | 0 |
| PPP2R2C | 341 | -0.0238 | 0 | 0 | 0.2101 | 0 |
| SAMD12 | 342 | -0.3228 | 0 | 0 | 0.207 | 0 |
| CDHR1 | 343 | -0.1486 | 0 | 0 | 0.2067 | 0 |
| NFIB | 344 | -0.3221 | 0 | 0 | 0.2061 | 0 |
| LOC25845 | 345 | -0.0573 | -0.1104 | 0.0266 | 0.2059 | 0 |
| PRLR | 346 | -0.0921 | 0 | 0 | 0.2056 | 0 |
| PTPRD | 347 | -0.1715 | 0 | 0 | 0.2049 | 0 |
| PLAGL1 | 348 | -0.1341 | 0 | 0 | 0.196 | 0 |
| WIF1 | 349 | -0.0592 | 0 | 0 | 0.1958 | 0 |
| CADPS | 350 | -0.2793 | 0.1153 | 0 | 0.1946 | 0 |
| TOB1 | 351 | -0.3904 | 0 | 0 | 0.1943 | 0 |
| MFAP3L | 352 | -0.0401 | 0 | 0 | 0.1941 | 0 |
| MAP7D2 | 353 | -0.0732 | -0.0514 | 0 | 0.1869 | 0 |
| FAM92A1 | 354 | -0.0126 | 0 | -0.0275 | 0.1866 | 0 |
| MUC20 | 355 | -0.2974 | 0 | 0.0492 | 0.1832 | 0 |
| RBM6 | 356 | -0.3166 | 0 | 0 | 0.1808 | 0 |
| PLCB1 | 357 | -0.0974 | 0 | -0.0728 | 0.1804 | 0 |
| HMGA2 | 358 | 0 | 0 | -0.0796 | 0.1802 | 0 |
| CBFA2T2 | 359 | -0.1817 | 0 | 0 | 0.1792 | 0 |
| TNMD | 360 | -0.0216 | 0 | 0 | 0.1775 | 0 |
| FABP6 | 361 | -0.1468 | 0 | 0 | 0.1764 | 0 |
| CEACAM6 | 362 | -0.2263 | 0 | 0 | 0.1748 | 0 |
| ZNF704 | 363 | -0.243 | 0 | 0 | 0.1733 | 0 |
| MYEF2 | 364 | -0.0974 | 0 | 0 | 0.1697 | 0 |
| GDF15 | 365 | 0 | 0 | -0.0544 | 0.1689 | 0 |
| CXCL14 | 366 | -0.4991 | 0 | 0 | 0.1688 | 0 |
| CEACAM5 | 367 | -0.1925 | 0 | 0 | 0.1687 | 0 |
| CDH17 | 368 | -0.1843 | 0 | 0 | 0.1668 | 0 |
| ENPP5 | 369 | -0.0607 | 0 | 0 | 0.1612 | 0 |
| C1orf103 | 370 | -0.0487 | 0 | 0 | 0.1583 | 0 |
| HOXA3 | 371 | -0.0889 | 0 | 0 | 0.1551 | 0 |
| EIF3B | 372 | -0.0227 | 0 | 0 | 0.1548 | 0 |
| LOC100289610 | 373 | -0.188 | 0 | 0 | 0.1546 | 0 |
| ASB9 | 374 | 0 | 0 | -0.1078 | 0.1527 | 0 |
| SLC26A2 | 375 | -0.4098 | -0.1876 | 0.5747 | 0.1523 | 0 |
| PHACTR3 | 376 | -0.0092 | -0.1282 | 0 | 0.1479 | 0 |
| GLS | 377 | 0 | -0.0262 | -0.0338 | 0.1478 | 0 |
| KIAA1199 | 378 | 0 | 0 | -0.2286 | 0.1423 | 0 |
| ZAK | 379 | 0 | 0 | -0.0518 | 0.1417 | 0 |
| NR1D2 | 380 | -0.1145 | 0 | 0 | 0.129 | 0 |
| RBP1 | 381 | -0.1254 | 0 | 0 | 0.129 | 0 |
| ZNF518B | 382 | -0.0681 | 0 | 0 | 0.1279 | 0 |
| GZMB | 383 | 0.1335 | -0.2025 | -0.0266 | 0.1237 | 0 |
| ANKRD10 | 384 | -0.1495 | 0 | 0 | 0.1216 | 0 |
| HENMT1 | 385 | -0.0707 | 0 | 0 | 0.118 | 0 |
| PLEKHB1 | 386 | -0.1526 | 0 | 0 | 0.1167 | 0 |
| FABP1 | 387 | -0.399 | 0 | 0.2884 | 0.1166 | 0 |
| ABCB1 | 388 | -0.189 | 0 | 0 | 0.1138 | 0 |
| MSX2 | 389 | 0 | 0.0851 | -0.2452 | 0.0891 | 0 |
| PDGFA | 390 | -0.1683 | 0 | 0.0013 | 0.0717 | 0 |
| IL17RD | 391 | -0.011 | 0 | -0.1659 | 0.0663 | 0 |
| LRRC16A | 392 | -0.1702 | 0.0048 | 0 | 0.066 | 0 |
| MUC12 | 393 | -0.5343 | 0 | 0.4773 | 0.0633 | 0 |
| HMGCS2 | 394 | -0.3122 | 0.028 | 0 | 0.0598 | 0 |
| FAM134B | 395 | -0.1392 | 0 | 0.0482 | 0.0458 | 0 |
| LEFTY1 | 396 | -0.2547 | 0 | 0.0763 | 0.0113 | 0 |
| TRPM6 | 397 | -0.2627 | -0.0093 | 0.5039 | 0 | 0 |
| PCK1 | 398 | -0.1474 | 0 | 0.4049 | 0 | 0 |
| EDN3 | 399 | -0.016 | 0 | 0.3932 | 0 | 0 |
| SEMA6D | 400 | -0.0291 | -0.0344 | 0.3414 | 0 | 0 |
| SCARA5 | 401 | -0.0852 | 0 | 0.3278 | 0 | 0 |
| METTL7A | 402 | -0.1623 | 0 | 0.3079 | 0 | 0 |
| HPGD | 403 | 0 | -0.0049 | 0.3033 | 0 | 0 |
| CLDN23 | 404 | 0 | -0.0373 | 0.2606 | 0 | 0 |
| SEPP1 | 405 | -0.1604 | 0 | 0.2215 | 0 | 0 |
| CNTN3 | 406 | -0.1222 | 0 | 0.2168 | 0 | 0 |
| SEMA6A | 407 | 0 | 0 | 0.2091 | 0 | 0 |
| PRKACB | 408 | -0.0976 | 0 | 0.2029 | 0 | 0 |
| KRT20 | 409 | -0.3208 | 0 | 0.1815 | 0 | 0 |
| EDNRB | 410 | -0.1973 | 0 | 0.163 | 0 | 0 |
| PID1 | 411 | -0.2336 | 0 | 0.128 | 0 | 0 |
| TSPAN7 | 412 | -0.15 | 0 | 0.1055 | 0 | 0 |
| SRI | 413 | -0.0689 | 0 | 0.0662 | 0 | 0 |
| PCCA | 414 | -0.0818 | 0.4502 | 0 | 0 | 0 |
| SMAD9 | 415 | -0.2481 | 0.365 | 0 | 0 | 0 |
| KLK11 | 416 | 0 | 0.2954 | 0 | 0 | 0 |
| PRUNE2 | 417 | -0.1028 | 0.2936 | 0 | 0 | 0 |
| C11orf93 | 418 | 0 | 0.2583 | 0 | 0 | 0 |
| MATN2 | 419 | -0.0711 | 0.233 | 0 | 0 | 0 |
| APOBEC1 | 420 | -0.0036 | 0.1449 | 0 | 0 | 0 |
| AIM2 | 421 | 0.3861 | 0 | 0 | 0 | 0 |
| AFAP1-AS1 | 422 | 0.1764 | 0 | 0 | 0 | 0 |
| CMPK2 | 423 | 0.2217 | -0.0104 | 0 | 0 | 0 |
| LY6E | 424 | 0.2662 | -0.049 | 0 | 0 | 0 |
| EPSTI1 | 425 | 0.1185 | -0.1598 | 0 | 0 | 0 |
| SLAIN1 | 426 | 0 | 0.3105 | -0.0173 | 0 | 0 |
| PIWIL1 | 427 | 0.2906 | 0 | -0.0227 | 0 | 0 |
| TNFSF9 | 428 | 0.2803 | 0 | -0.0343 | 0 | 0 |
| TMPRSS3 | 429 | 0.1663 | 0 | -0.0531 | 0 | 0 |
| ANKRD37 | 430 | 0.1106 | 0 | -0.0552 | 0 | 0 |
| WISP3 | 431 | 0 | 0.2378 | -0.0988 | 0 | 0 |
| RPL22L1 | 432 | 0.3107 | 0 | -0.1145 | 0 | 0 |
| IGF2BP3 | 433 | 0.2673 | 0 | -0.1308 | 0 | 0 |
| MFI2 | 434 | 0 | 0.102 | -0.1555 | 0 | 0 |
| CA9 | 435 | 0 | 0.2 | -0.1787 | 0 | 0 |
| C8orf84 | 436 | 0 | 0.3554 | -0.184 | 0 | 0 |
| PMAIP1 | 437 | 0.2598 | 0 | -0.2185 | 0 | 0 |
| FRMD5 | 438 | 0.1384 | 0 | -0.2581 | 0 | 0 |
| IFIT1 | 439 | 0.0962 | -0.1168 | 0 | -0.0045 | 0 |
| CALB1 | 440 | 0.2348 | 0 | -0.1107 | -0.0103 | 0 |
| ADRB1 | 441 | 0.0125 | 0.1577 | 0 | -0.0116 | 0 |
| STAT1 | 442 | 0.4213 | 0 | 0 | -0.0126 | 0 |
| MICB | 443 | 0.2977 | 0 | 0 | -0.0208 | 0 |
| ISG15 | 444 | 0.3148 | 0 | 0 | -0.0216 | 0 |
| IFI44L | 445 | 0.2383 | -0.0365 | 0 | -0.0293 | 0 |
| GBP4 | 446 | 0.5181 | 0 | 0 | -0.0304 | 0 |
| TLR8 | 447 | 0.2868 | 0 | -0.0144 | -0.0312 | 0 |
| DDX60 | 448 | 0.1355 | 0 | 0 | -0.0339 | 0 |
| P2RY14 | 449 | 0 | 0 | 0.1921 | -0.0349 | 0 |
| ADAMDEC1 | 450 | 0 | 0 | 0.2241 | -0.0421 | 0 |
| CPM | 451 | 0 | 0 | 0.3583 | -0.0446 | 0 |
| LCK | 452 | 0.3028 | 0 | 0 | -0.046 | 0 |
| GBP5 | 453 | 0.49 | 0 | -0.0152 | -0.0474 | 0 |
| IFIT2 | 454 | 0.2739 | -0.0225 | 0 | -0.0503 | 0 |
| PLA2G7 | 455 | 0.2799 | -0.0206 | 0 | -0.0551 | 0 |
| OAS2 | 456 | 0.2432 | 0 | 0 | -0.0603 | 0 |
| RSAD2 | 457 | 0.2188 | -0.1364 | 0 | -0.0635 | 0 |
| XAF1 | 458 | 0.2921 | 0 | 0 | -0.0641 | 0 |
| PNMA2 | 459 | 0.0477 | 0.0594 | -0.1392 | -0.0683 | 0 |
| MMP12 | 460 | 0.2904 | 0 | 0 | -0.07 | 0 |
| KIAA1211 | 461 | 0 | 0 | 0.115 | -0.073 | 0 |
| APOBEC3G | 462 | 0.4443 | -0.0042 | 0 | -0.0731 | 0 |
| IFI44 | 463 | 0.3353 | 0 | 0 | -0.074 | 0 |
| EPHA4 | 464 | 0 | 0.346 | 0 | -0.075 | 0 |
| FAM26F | 465 | 0.4467 | 0 | 0 | -0.0821 | 0 |
| GIMAP6 | 466 | 0.1788 | 0 | 0 | -0.0837 | 0 |
| HSPA2 | 467 | -0.0469 | 0.3272 | 0 | -0.0885 | 0 |
| CXCL11 | 468 | 0.4731 | 0 | 0 | -0.0907 | 0 |
| MNDA | 469 | 0.1817 | 0 | 0 | -0.0952 | 0 |
| CCL4 | 470 | 0.3826 | 0 | 0 | -0.0976 | 0 |
| TRBC1 | 471 | 0.2654 | 0 | 0 | -0.1004 | 0 |
| TAGAP | 472 | 0.1869 | 0 | 0 | -0.1035 | 0 |
| FGFR2 | 473 | 0 | 0.1763 | 0 | -0.1081 | 0 |
| CD55 | 474 | 0.0466 | 0.1687 | 0 | -0.1089 | 0 |
| CXCL9 | 475 | 0.5397 | 0 | -0.0055 | -0.1101 | 0 |
| CYBB | 476 | 0.2122 | 0 | 0 | -0.1111 | 0 |
| PLK2 | 477 | 0.2547 | 0 | -0.061 | -0.1115 | 0 |
| IL1RN | 478 | 0.2248 | 0 | 0 | -0.114 | 0 |
| HOXC6 | 479 | 0.4209 | 0 | -0.2279 | -0.1143 | 0 |
| BTN3A3 | 480 | 0.1554 | 0 | 0 | -0.1162 | 0 |
| BAG2 | 481 | 0.2725 | 0 | 0 | -0.1189 | 0 |
| IGLL3P | 482 | 0 | 0 | 0.0601 | -0.1194 | 0 |
| PLA2G4A | 483 | 0.1896 | 0.1581 | 0 | -0.1209 | 0 |
| BST2 | 484 | 0.4116 | 0 | 0 | -0.1213 | 0 |
| HLA-DMB | 485 | 0.382 | 0 | 0 | -0.1217 | 0 |
| SLAMF7 | 486 | 0.312 | 0 | 0 | -0.1229 | 0 |
| IGLV1-44 | 487 | 0.0202 | 0 | 0.1734 | -0.1247 | 0 |
| IFIT3 | 488 | 0.3968 | 0 | 0 | -0.126 | 0 |
| GBP1 | 489 | 0.5015 | -0.0061 | 0 | -0.1332 | 0 |
| IGJ | 490 | 0 | 0 | 0.4497 | -0.1364 | 0 |
| FSCN1 | 491 | 0.1698 | 0 | -0.0764 | -0.1381 | 0 |
| FYB | 492 | 0.2848 | 0 | 0 | -0.1386 | 0 |
| CXCL10 | 493 | 0.5197 | 0 | 0 | -0.1394 | 0 |
| CD74 | 494 | 0.3213 | 0 | 0 | -0.1423 | 0 |
| SERPINB5 | 495 | 0.1117 | 0.1027 | 0 | -0.1425 | 0 |
| IFI6 | 496 | 0.2833 | 0 | 0 | -0.147 | 0 |
| FGL2 | 497 | 0.1238 | 0 | 0 | -0.1474 | 0 |
| PRKAR2B | 498 | 0.0934 | 0 | 0 | -0.1513 | 0 |
| POU2AF1 | 499 | 0 | 0 | 0.131 | -0.1532 | 0 |
| BIRC3 | 500 | 0.4733 | 0 | 0 | -0.1535 | 0 |
| EPB41L3 | 501 | 0 | 0 | 0.1808 | -0.1547 | 0 |
| MPEG1 | 502 | 0.091 | 0 | 0 | -0.1574 | 0 |
| IGKC | 503 | 0 | 0 | 0.0947 | -0.1618 | 0 |
| CCL8 | 504 | 0.3808 | -0.0649 | 0 | -0.1634 | 0 |
| IFI16 | 505 | 0.2516 | 0 | 0 | -0.17 | 0 |
| MT1F | 506 | 0.1194 | 0 | 0.113 | -0.1761 | 0 |
| CSF2RB | 507 | 0.2068 | 0 | 0 | -0.1775 | 0 |
| SAMD9 | 508 | 0.1828 | 0 | 0 | -0.1809 | 0 |
| LYZ | 509 | 0.2329 | 0.1665 | 0 | -0.1816 | 0 |
| MMP28 | 510 | 0 | 0.0164 | 0.2038 | -0.1829 | 0 |
| CCL5 | 511 | 0.5145 | 0 | 0 | -0.1855 | 0 |
| HLA-DPA1 | 512 | 0.4238 | 0 | 0 | -0.1885 | 0 |
| HLA-DMA | 513 | 0.4118 | 0 | 0 | -0.191 | 0 |
| KYNU | 514 | 0.4072 | 0 | -0.0714 | -0.1914 | 0 |
| CFD | 515 | 0.0805 | 0 | 0 | -0.1943 | 0 |
| CD69 | 516 | 0.2467 | 0 | 0 | -0.1981 | 0 |
| ITM2A | 517 | 0.0869 | 0 | 0 | -0.1983 | 0 |
| TRIM22 | 518 | 0.2913 | 0 | 0 | -0.2005 | 0 |
| MT1M | 519 | 0 | 0 | 0.5267 | -0.2011 | 0 |
| C1QA | 520 | 0.3547 | 0 | 0 | -0.2015 | 0 |
| HLA-DPB1 | 521 | 0.3403 | 0 | 0 | -0.2053 | 0 |
| LCP2 | 522 | 0.3956 | -0.0091 | 0 | -0.2147 | 0 |
| MT1G | 523 | 0.1359 | 0 | 0.0953 | -0.2166 | 0 |
| C1QB | 524 | 0.3862 | 0 | 0 | -0.221 | 0 |
| CD53 | 525 | 0.3244 | 0 | 0 | -0.2255 | 0 |
| CYTIP | 526 | 0.1751 | 0 | 0 | -0.2264 | 0 |
| SAMSN1 | 527 | 0.344 | 0 | 0 | -0.2288 | 0 |
| HLA-DRA | 528 | 0.3527 | 0 | 0 | -0.255 | 0 |
| CD52 | 529 | 0.2716 | 0 | 0 | -0.2573 | 0 |
| EVI2B | 530 | 0.2485 | 0 | 0 | -0.2577 | 0 |
| MT1H | 531 | 0.1867 | 0 | 0.0606 | -0.2578 | 0 |
| PTPRC | 532 | 0.3709 | -0.0259 | 0 | -0.2584 | 0 |
| SAMD9L | 533 | 0.4904 | 0 | 0 | -0.2659 | 0 |
| DAPK1 | 534 | 0.1474 | 0.1093 | -0.0256 | -0.2736 | 0 |
| DUSP4 | 535 | 0.3433 | 0.3562 | -0.2053 | -0.2761 | 0 |
| RARRES3 | 536 | 0.5944 | 0 | 0 | -0.2781 | 0 |
| MT1X | 537 | 0.2251 | 0 | 0 | -0.2785 | 0 |
| DOCK8 | 538 | 0.2125 | 0 | 0 | -0.2859 | 0 |
| MT2A | 539 | 0.2765 | 0 | 0 | -0.288 | 0 |
| CRIP1 | 540 | 0.227 | 0.1036 | 0 | -0.2928 | 0 |
| CXCL13 | 541 | 0.6193 | -0.0086 | 0 | -0.2928 | 0 |
| MT1E | 542 | 0.2144 | 0 | 0.1515 | -0.3251 | 0 |
| ALOX5 | 543 | 0.116 | 0.1858 | 0 | -0.3513 | 0 |
| RARRES1 | 544 | 0.1835 | 0 | 0 | -0.3703 | 0 |
| GRM8 | 545 | -0.1842 | 0 | 0 | 0.3559 | -0.0017 |
| FAM55D | 546 | -0.2397 | 0 | 0.4172 | 0 | -0.0021 |
| ABP1 | 547 | -0.3797 | 0 | 0.1849 | 0.0557 | -0.0035 |
| LOC401022 | 548 | 0 | 0 | 0.1009 | -0.0626 | -0.0046 |
| ISX | 549 | -0.2577 | 0 | 0.2822 | 0.1497 | -0.0047 |
| CDC6 | 550 | 0.044 | 0 | -0.1237 | 0.028 | -0.0047 |
| FAM105A | 551 | -0.2265 | 0 | 0 | 0.2478 | -0.005 |
| IDO1 | 552 | 0.5825 | 0 | 0 | -0.0333 | -0.0055 |
| SLC28A3 | 553 | 0.1386 | 0.2023 | -0.109 | 0 | -0.006 |
| CDK6 | 554 | -0.0651 | 0.0397 | 0 | 0.1329 | -0.0062 |
| TFF2 | 555 | 0.1662 | 0.0636 | 0 | 0 | -0.0067 |
| PITX2 | 556 | 0 | 0 | 0 | 0.1789 | -0.0068 |
| NEBL | 557 | -0.0922 | 0 | 0 | 0.2638 | -0.0069 |
| ANXA10 | 558 | 0.2257 | 0 | -0.0479 | 0 | -0.0071 |
| GPR160 | 559 | -0.0944 | 0 | 0 | 0.2195 | -0.0073 |
| PAQR5 | 560 | 0 | -0.0031 | 0.0384 | 0.0606 | -0.0081 |
| CCL24 | 561 | -0.1823 | 0.2141 | 0 | 0.0784 | -0.0085 |
| VNN1 | 562 | 0.2993 | 0.0071 | 0 | -0.2398 | -0.0087 |
| WFDC2 | 563 | -0.0944 | 0.2396 | 0 | 0 | -0.0102 |
| PSMB9 | 564 | 0.3035 | 0 | 0 | 0 | -0.0103 |
| GZMA | 565 | 0.5439 | 0 | 0 | -0.2148 | -0.0103 |
| VAV3 | 566 | -0.4096 | 0 | 0 | 0.423 | -0.0118 |
| LY75 | 567 | 0 | 0 | 0 | 0.2712 | -0.0119 |
| CACNA1D | 568 | -0.2181 | 0 | 0 | 0.3298 | -0.0122 |
| TBX3 | 569 | 0 | 0.2417 | -0.1916 | 0 | -0.0155 |
| MFSD4 | 570 | -0.0284 | 0 | 0.4083 | 0 | -0.0157 |
| ATP8A1 | 571 | 0 | 0.0759 | 0 | -0.0393 | -0.0167 |
| PPP1R14D | 572 | -0.2943 | 0 | 0.0147 | 0.2496 | -0.0177 |
| FRMD3 | 573 | 0 | 0 | 0.125 | -0.0431 | -0.0181 |
| CPS1 | 574 | 0 | 0.3391 | -0.005 | 0 | -0.0196 |
| CYP39A1 | 575 | -0.2247 | 0 | 0 | 0.2655 | -0.02 |
| IL1R2 | 576 | 0.1142 | 0.2611 | 0 | -0.2802 | -0.0202 |
| IGHM | 577 | 0 | 0 | 0.2346 | -0.1786 | -0.0209 |
| GABRP | 578 | 0.0041 | 0.1624 | 0 | 0 | -0.0221 |
| ARSE | 579 | -0.0085 | 0 | 0 | 0.2053 | -0.0253 |
| ZIC2 | 580 | 0.3979 | 0 | 0 | -0.1145 | -0.0299 |
| TNFRSF17 | 581 | 0 | 0 | 0.1733 | 0 | -0.0334 |
| LOC653602 | 582 | -0.151 | 0 | 0 | 0.1509 | -0.0362 |
| SPAG1 | 583 | 0 | 0 | 0 | 0.1439 | -0.0395 |
| NEDD4L | 584 | -0.0333 | 0 | 0.0707 | 0 | -0.0399 |
| UGT2A3 | 585 | -0.2127 | -0.0638 | 0.4365 | 0.0923 | -0.0404 |
| SLC1A1 | 586 | 0.0619 | 0 | 0 | -0.0697 | -0.041 |
| LGALS2 | 587 | 0 | 0 | 0.2603 | 0 | -0.0413 |
| CLDN8 | 588 | -0.0779 | -0.0473 | 0.9237 | 0 | -0.0415 |
| TOX | 589 | 0 | 0.5363 | 0 | -0.1211 | -0.0441 |
| TFAP2A | 590 | 0.3438 | 0.2136 | -0.189 | -0.069 | -0.0444 |
| TOX3 | 591 | 0 | 0 | 0 | 0.1406 | -0.0465 |
| C17orf73 | 592 | -0.0771 | 0 | 0.0831 | 0.0209 | -0.0475 |
| MLPH | 593 | 0 | 0.33 | 0 | -0.1434 | -0.0511 |
| FAS | 594 | 0.1759 | 0 | 0.0573 | -0.1003 | -0.0522 |
| F3 | 595 | 0.0335 | 0.1539 | 0 | -0.0159 | -0.0529 |
| FMO5 | 596 | -0.1242 | 0 | 0.0561 | 0 | -0.0544 |
| SPINK1 | 597 | -0.2495 | 0 | 0 | 0.1836 | -0.055 |
| GUCY2C | 598 | -0.3597 | 0 | 0 | 0.2594 | -0.0562 |
| FGFR3 | 599 | -0.0124 | 0 | 0 | 0.1569 | -0.0564 |
| PCSK1 | 600 | -0.0537 | 0.5971 | 0 | 0 | -0.0574 |
| TCN1 | 601 | 0 | 0.6045 | -0.012 | -0.1313 | -0.0578 |
| MALL | 602 | 0 | 0 | 0.1103 | 0 | -0.0579 |
| SLC3A1 | 603 | -0.2476 | 0 | 0 | 0.2101 | -0.0584 |
| CD 177 | 604 | 0 | 0 | 0.4963 | -0.0076 | -0.059 |
| HNRNPH1 | 605 | 0.1863 | 0 | 0 | 0 | -0.0593 |
| TMEM37 | 606 | 0 | 0 | 0.3281 | 0 | -0.0596 |
| E2F7 | 607 | 0.1305 | 0 | -0.1327 | 0 | -0.0612 |
| CLDN3 | 608 | -0.1747 | 0 | 0 | 0.2229 | -0.0614 |
| DHRS 11 | 609 | -0.0404 | 0 | 0.2028 | 0.0508 | -0.0625 |
| SERPINA1 | 610 | 0 | 0.4433 | 0 | -0.0382 | -0.0625 |
| SLC16A9 | 611 | -0.0604 | 0 | 0.061 | 0.0346 | -0.0639 |
| GNLY | 612 | 0.5097 | 0 | 0 | -0.0483 | -0.0645 |
| ZNF165 | 613 | 0.1911 | 0 | -0.0189 | 0 | -0.0666 |
| UGT2B17 | 614 | -0.091 | 0 | 0.4545 | 0 | -0.0669 |
| CLDN18 | 615 | 0.1004 | 0.0605 | 0 | 0 | -0.0672 |
| ZFP36L2 | 616 | -0.0876 | 0 | 0 | 0.1365 | -0.0678 |
| LOC646627 | 617 | -0.286 | 0 | 0.7338 | 0 | -0.0682 |
| ANXA13 | 618 | 0 | 0.1626 | 0 | 0 | -0.0691 |
| LASS6 | 619 | 0 | 0 | 0 | 0.1218 | -0.0697 |
| TFF3 | 620 | 0 | 0.2859 | 0 | 0 | -0.0699 |
| SGK2 | 621 | -0.2125 | 0 | 0.0205 | 0.3224 | -0.0713 |
| RNF125 | 622 | 0.1626 | 0.0824 | 0.0249 | -0.2444 | -0.0719 |
| CHP2 | 623 | -0.2525 | 0 | 0.412 | 0 | -0.0724 |
| ANKRD43 | 624 | -0.2059 | 0 | 0.0255 | 0.3164 | -0.074 |
| PYY | 625 | 0 | 0 | 0.5285 | 0 | -0.077 |
| B3GNT7 | 626 | 0 | 0 | 0.6661 | -0.0172 | -0.0773 |
| FAM84A | 627 | -0.2409 | 0 | 0 | 0.2504 | -0.0775 |
| SCGB2A1 | 628 | 0 | 0.1165 | 0.2545 | -0.022 | -0.0782 |
| BLNK | 629 | 0 | 0.1155 | 0 | -0.0025 | -0.0784 |
| DEFA5 | 630 | -0.2069 | 0.4097 | 0 | 0 | -0.0796 |
| STS | 631 | 0.137 | 0.0511 | 0 | -0.0493 | -0.0797 |
| AQP8 | 632 | -0.0967 | -0.0503 | 0.6919 | 0 | -0.0813 |
| DDC | 633 | -0.0506 | 0 | 0 | 0.3179 | -0.0814 |
| SLC26A3 | 634 | -0.4869 | -0.3214 | 0.8633 | 0.2214 | -0.0827 |
| ENPP3 | 635 | -0.2751 | 0 | 0.068 | 0.2982 | -0.083 |
| MOCOS | 636 | 0.1547 | 0 | 0 | 0 | -0.083 |
| ARL14 | 637 | 0 | 0 | 0.2233 | 0 | -0.0847 |
| PDE9A | 638 | -0.1238 | 0 | 0.2265 | 0 | -0.0849 |
| VSIG2 | 639 | -0.0998 | 0.1561 | 0.5903 | -0.1277 | -0.0855 |
| EPHB3 | 640 | 0 | 0.0699 | 0 | 0.0728 | -0.0879 |
| UGT2B15 | 641 | 0 | 0.0291 | 0.2061 | 0 | -0.0889 |
| SCIN | 642 | 0 | 0 | 0.2905 | -0.0701 | -0.0909 |
| GCG | 643 | -0.0333 | 0 | 0.6672 | 0 | -0.0915 |
| EIF5A | 644 | 0.2584 | 0 | 0 | 0 | -0.0957 |
| SLC7A11 | 645 | 0.2432 | 0 | -0.0564 | 0 | -0.0965 |
| DEFA6 | 646 | -0.2071 | 0.2699 | 0 | 0.1026 | -0.0967 |
| HSPA4L | 647 | 0.4777 | 0 | -0.1142 | 0 | -0.0977 |
| NR5A2 | 648 | 0 | 0 | 0.2702 | 0 | -0.0978 |
| FAM46C | 649 | 0.058 | 0.156 | 0.0039 | -0.1788 | -0.0981 |
| MUC1 | 650 | 0.1133 | 0.2233 | 0 | -0.2325 | -0.0986 |
| SEMG1 | 651 | 0.2915 | 0 | 0 | -0.0107 | -0.0988 |
| CA12 | 652 | 0 | 0.0193 | 0.1852 | 0 | -0.1029 |
| SSTR1 | 653 | 0 | 0.2208 | 0 | 0 | -0.1029 |
| PBLD | 654 | -0.1626 | 0 | 0.3327 | 0.0387 | -0.1034 |
| SDR16C5 | 655 | 0.1124 | 0.365 | 0 | -0.2206 | -0.104 |
| CA1 | 656 | -0.1556 | -0.106 | 1.1648 | 0 | -0.1047 |
| SLITRK6 | 657 | 0 | 0.6746 | 0 | -0.0671 | -0.1053 |
| C15orf48 | 658 | 0 | 0 | 0.1913 | -0.0205 | -0.1058 |
| RETNLB | 659 | -0.1708 | 0.6788 | 0 | -0.0034 | -0.1068 |
| REG1B | 660 | 0 | 0.265 | 0.1291 | -0.0772 | -0.1068 |
| GPR126 | 661 | 0.3516 | 0.0502 | 0 | -0.1412 | -0.1088 |
| NAT2 | 662 | -0.1429 | 0.0137 | 0.0234 | 0.02 | -0.1099 |
| RNF186 | 663 | 0 | 0.0295 | 0 | 0 | -0.1105 |
| PSAT1 | 664 | 0.1191 | 0 | -0.1161 | 0 | -0.1114 |
| OLFM4 | 665 | -0.1798 | 0 | 0.2002 | 0 | -0.1118 |
| A1CF | 666 | -0.4783 | 0 | 0.0926 | 0.3806 | -0.112 |
| PTGER4 | 667 | 0 | 0.1113 | 0.0641 | 0 | -0.113 |
| AP1S3 | 668 | 0.1181 | 0 | 0 | 0 | -0.1136 |
| SPINK5 | 669 | 0 | 0 | 0.4997 | 0 | -0.1147 |
| CWH43 | 670 | -0.0661 | 0.1188 | 0.0912 | 0 | -0.1153 |
| TRPA1 | 671 | -0.0318 | 0.2025 | 0.0203 | 0 | -0.1164 |
| GCNT3 | 672 | 0.1215 | 0.0448 | 0.1736 | -0.2489 | -0.1169 |
| LAMA1 | 673 | 0 | 0.1677 | 0.2283 | -0.0487 | -0.118 |
| KCNK1 | 674 | 0.1194 | 0.0547 | 0 | -0.0584 | -0.1184 |
| MUC5AC | 675 | 0.1499 | 0.1813 | 0 | -0.0307 | -0.1207 |
| MYRIP | 676 | -0.1778 | 0 | 0 | 0.3282 | -0.1215 |
| FOXA1 | 677 | 0.1204 | 0.0106 | 0 | 0 | -0.1229 |
| C9orf152 | 678 | 0 | 0.1578 | 0 | 0 | -0.123 |
| STX19 | 679 | 0 | 0 | 0 | 0.0071 | -0.124 |
| CTSE | 680 | 0.1232 | 0.3417 | 0 | -0.2717 | -0.1256 |
| PARM1 | 681 | 0 | 0 | 0.0774 | 0 | -0.1265 |
| SI | 682 | 0 | 0 | 0.7566 | -0.1968 | -0.1266 |
| TSPAN12 | 683 | 0 | 0 | 0 | 0.1291 | -0.1268 |
| AQP3 | 684 | 0 | 0.55 | 0 | -0.1016 | -0.1272 |
| PKIB | 685 | 0 | 0 | 0.5363 | -0.0196 | -0.1285 |
| DHRS9 | 686 | 0 | 0 | 0.6841 | -0.0531 | -0.1287 |
| MEP1A | 687 | -0.4152 | 0 | 0.2711 | 0.2924 | -0.1291 |
| FAM55A | 688 | -0.0896 | 0.0635 | 0.3244 | 0 | -0.1302 |
| APOL6 | 689 | 0.1761 | 0 | 0 | 0 | -0.1318 |
| C10orf99 | 690 | -0.4666 | 0 | 0.2794 | 0.2684 | -0.1355 |
| CEACAM1 | 691 | -0.0758 | 0 | 0.1347 | 0.1253 | -0.1364 |
| IQGAP2 | 692 | 0 | 0.0843 | 0 | -0.0291 | -0.137 |
| HGD | 693 | 0 | 0.1104 | 0.0295 | 0 | -0.1379 |
| FAM110C | 694 | 0 | 0 | 0 | 0.0756 | -0.1389 |
| BCL2L15 | 695 | 0 | 3.00E-04 | 0 | 0 | -0.1409 |
| LOC285628 | 696 | 0.1006 | 0 | 0 | 0 | -0.141 |
| MUC13 | 697 | 0 | 0 | 0.0047 | 0 | -0.1415 |
| SRSF6 | 698 | 0.3681 | 0 | 0 | 0 | -0.1421 |
| MAOA | 699 | 0 | 0.0054 | 0.0645 | 0 | -0.1426 |
| REG3A | 700 | 0 | 0.3642 | 0 | 0 | -0.1431 |
| ADH1C | 701 | -0.0505 | 0 | 0.7086 | 0 | -0.1433 |
| RHBDL2 | 702 | 0 | 0.124 | 0.164 | 0 | -0.1433 |
| RASEF | 703 | 0 | 0.0349 | 0 | 0.0076 | -0.1435 |
| GNE | 704 | 0 | 0.2974 | 0 | -0.1021 | -0.1436 |
| EPB41L4B | 705 | -0.0915 | 0 | 0 | 0.1986 | -0.1437 |
| ELOVL7 | 706 | 0.0731 | 0.0922 | 0 | 0 | -0.145 |
| ID1 | 707 | -0.2755 | 0 | 0 | 0.2997 | -0.1463 |
| BCAS1 | 708 | 0 | 0.2379 | 0.2158 | -0.0024 | -0.1501 |
| PLA2G2A | 709 | 0.2168 | 0.0815 | 0.4598 | -0.486 | -0.1579 |
| FAM3D | 710 | -0.1337 | 0.0915 | 0.0829 | 0 | -0.164 |
| TMEM56 | 711 | 0 | 0 | 0 | 0.0477 | -0.1641 |
| HHLA2 | 712 | 0 | 0 | 0.2692 | 0 | -0.166 |
| GPA33 | 713 | 0 | 0 | 0.0895 | 0 | -0.1674 |
| FAM169A | 714 | 0.114 | 0 | -0.1773 | 0.0082 | -0.1709 |
| L1TD1 | 715 | 0 | 0.5659 | 0 | 0 | -0.1713 |
| HIPK2 | 716 | 0 | 0 | 0 | 0.0459 | -0.173 |
| CDHR5 | 717 | -0.1114 | 0 | 0.4598 | 0.0126 | -0.1746 |
| NCRNA00261 | 718 | 0 | 0.6892 | 0 | -0.0846 | -0.1753 |
| GIPC2 | 719 | 0 | 0 | 0 | 0.1653 | -0.1758 |
| SLC44A4 | 720 | 0 | 0 | 0.1474 | 0 | -0.176 |
| TMEM144 | 721 | 0.0196 | 0 | 0 | 0 | -0.1761 |
| CLRN3 | 722 | -0.0303 | 0.0521 | 0 | 0.0239 | -0.1775 |
| MS4A12 | 723 | -0.2189 | -0.085 | 1.2327 | 0 | -0.1794 |
| DMBT1 | 724 | 0 | 0.1604 | 0.0775 | 0 | -0.1811 |
| KLF4 | 725 | 0 | 0.1714 | 0.1652 | -0.0391 | -0.1811 |
| TYMS | 726 | 0.2779 | 0 | 0 | 0 | -0.1827 |
| TCEA3 | 727 | 0 | 0.0673 | 0.1001 | 0 | -0.1849 |
| REG1A | 728 | 0 | 0.3339 | 0.1229 | -0.106 | -0.1862 |
| 03FAR1 | 729 | 0 | 0.2761 | 0.0131 | -0.1374 | -0.1871 |
| AKR1B10 | 730 | 0 | 0 | 0.4524 | 0 | -0.1894 |
| ZG16B | 731 | 0 | 0.0674 | 0 | 0 | -0.1899 |
| DUOXA2 | 732 | -0.0559 | 0.1986 | 0.0347 | 0 | -0.1909 |
| TSPAN1 | 733 | 0 | 0.0296 | 0.2593 | -0.0498 | -0.1927 |
| CMBL | 734 | 0.025 | 0 | 0 | 0 | -0.1931 |
| LRRC19 | 735 | -0.2406 | 0 | 0.4224 | 0.0842 | -0.1958 |
| CA4 | 736 | -0.1041 | -0.1611 | 1.1584 | 0 | -0.1962 |
| PFKFB2 | 737 | 0 | 0 | 0 | 0.0178 | -0.1963 |
| CA2 | 738 | 0 | 0 | 0.8348 | -0.2319 | -0.1966 |
| MUC5B | 739 | 0.0166 | 0.3267 | 0 | -0.1629 | -0.1967 |
| PBK | 740 | 0.2468 | 0 | -0.0355 | 0 | -0.1979 |
| SGPP2 | 741 | 0.0406 | 0 | 0 | 0 | -0.1984 |
| PDZK1IP1 | 742 | 0 | 0.0371 | 0 | 0 | -0.199 |
| LRRC31 | 743 | -0.1468 | 0.0055 | 0.052 | 0.0762 | -0.1996 |
| HSD17B2 | 744 | 0 | 0 | 0.4486 | -0.0518 | -0.2004 |
| PLAC8 | 745 | 0.0752 | 0 | 0.4226 | -0.2386 | -0.213 |
| FUT3 | 746 | 0 | 0.115 | 0 | 0 | -0.2135 |
| AHCYL2 | 747 | 0 | 0 | 0.1925 | 0 | -0.2145 |
| GALNT7 | 748 | 0 | 0.0897 | 0 | 0 | -0.2155 |
| TFF1 | 749 | 0 | 0.3534 | 0 | -0.0308 | -0.2172 |
| KIAA1324 | 750 | 0 | 0.5516 | 0 | 0 | -0.2217 |
| C2CD4A | 751 | 0.06 | 0.1973 | -0.0763 | 0 | -0.2233 |
| HSD11B2 | 752 | -0.2885 | 0 | 0.2056 | 0.122 | -0.2238 |
| ZG16 | 753 | -0.277 | 0 | 1.2747 | -0.0738 | -0.2259 |
| TMPRSS2 | 754 | 0 | 0 | 0.121 | 0 | -0.2314 |
| LOC100505633 | 755 | 0 | 0 | 0.1534 | 0 | -0.2342 |
| CEACAM7 | 756 | -0.0427 | 0 | 0.7139 | 0 | -0.2346 |
| MUC4 | 757 | 0 | 0.3425 | 0.4273 | -0.3378 | -0.2358 |
| C6orf105 | 758 | 0 | 0.0904 | 0.4878 | -0.1345 | -0.2366 |
| FOXA3 | 759 | 0 | 0.3 | 0 | 0 | -0.2388 |
| CLCA1 | 760 | -0.2813 | 0.4699 | 0.7005 | -0.1336 | -0.2418 |
| DUOX2 | 761 | -0.1636 | 0.2113 | 0.3329 | 0 | -0.2425 |
| PIGR | 762 | 0.0536 | 0.0501 | 0.0738 | -0.0398 | -0.2547 |
| RAB27B | 763 | 0.487 | 0.2877 | 0 | -0.3304 | -0.2581 |
| CASP1 | 764 | 0.2001 | 0 | 0 | 0 | -0.2597 |
| STYK1 | 765 | 0 | 0.0156 | 0.0878 | 0 | -0.2605 |
| AGR3 | 766 | 0.2161 | 0.3804 | 0.0991 | -0.3697 | -0.2605 |
| LOC100505989 | 767 | 0 | 0.0763 | 0.0588 | 0 | -0.2608 |
| SLC4A4 | 768 | 0 | 0 | 0.883 | -0.3218 | -0.2627 |
| CLCA4 | 769 | -0.1036 | -0.0549 | 1.2783 | -0.0098 | -0.2643 |
| SLC39A8 | 770 | 0 | 0.1035 | 0 | 0 | -0.2645 |
| LCN2 | 771 | 0.0018 | 0.116 | 0 | 0 | -0.2709 |
| LIMA1 | 772 | 0 | 0.0672 | 0.0444 | 0 | -0.2767 |
| ITLN1 | 773 | -0.1478 | 0.4826 | 0.7893 | -0.264 | -0.2835 |
| TNFRSF11A | 774 | 0.0938 | 0.1267 | 0 | 0 | -0.2837 |
| SPINK4 | 775 | -0.1155 | 0.7836 | 0.4619 | -0.2607 | -0.2948 |
| AGR2 | 776 | 0.2149 | 0.3838 | 0 | -0.2065 | -0.2962 |
| TC2N | 777 | 0.1365 | 0.1647 | 0 | 0 | -0.3055 |
| CCL28 | 778 | 0 | 0 | 0.3981 | -0.0377 | -0.3062 |
| XDH | 779 | 0 | 0 | 0.2604 | 0 | -0.31 |
| HEPACAM2 | 780 | -0.1912 | 0.6205 | 0.5701 | -0.1608 | -0.3109 |
| SELENBP1 | 781 | -0.2011 | 0.0607 | 0.0439 | 0.1747 | -0.3174 |
| NR3C2 | 782 | 0 | 0.1313 | 0.2677 | -0.0233 | -0.3255 |
| REG4 | 783 | 0.0373 | 0.7826 | 0.273 | -0.5249 | -0.3414 |
| MUC2 | 784 | 0 | 0.5434 | 0.5514 | -0.3675 | -0.3415 |
| ST6GALNAC1 | 785 | 0 | 0.4922 | 0.2987 | -0.0889 | -0.4119 |
| FCGBP | 786 | 0 | 0.6699 | 0.521 | -0.3536 | -0.4409 |

According to an embodiment of the present invention, preferred gene profile specific to "Transit-amplifying (TA)" type of CRC is shown in Table 3 and more preferred gene profile specific to "Transit-amplifying (TA)" type of CRC is shown in Table 4. The scores are illustrative only and represent expression profiles (tendencies) of listed genes. Positive score means high expression, negative score means low expression and zero means no change in expression.

**Table 3**

| **Genes** | **Inflammatory** | **Goblet-like** | **Enterocyte** | **TA** | **Stem-like** |
|---|---|---|---|---|---|
| LY6G6D | -0.4827 | -0.278 | 0 | 0.645 | 0 |
| EREG | -0.237 | -0.5917 | 0 | 0.5346 | 0 |
| CEL | -0.3331 | -0.2754 | 0 | 0.513 | 0 |
| KRT23 | -0.3977 | -0.1715 | -0.0151 | 0.5018 | 0 |
| ACSL6 | -0.2707 | -0.1914 | 0 | 0.4946 | 0 |
| QPRT | -0.4772 | -0.0355 | 0 | 0.4905 | 0 |
| AXIN2 | -0.5204 | 0 | -0.041 | 0.4693 | 0 |
| ABAT | -0.419 | -0.196 | 0 | 0.4676 | 0 |
| FARP1 | -0.3463 | -0.1333 | 0 | 0.4618 | 0 |
| CELP | -0.2423 | -0.1824 | 0 | 0.4596 | 0 |
| C13orf18 | -0.4561 | -0.1986 | 0 | 0.4594 | 0 |
| HUNK | -0.3014 | 0 | 0 | 0.4551 | 0 |
| PLCB4 | -0.5302 | 0 | 0 | 0.4469 | 0 |
| APCDD1 | -0.3677 | 0 | -0.0421 | 0.4429 | 0 |
| RNF43 | -0.3522 | 0 | -0.0421 | 0.4419 | 0 |
| ASCL2 | -0.2372 | -0.0094 | 0 | 0.4368 | 0 |
| CHN2 | -0.3758 | 0 | 0 | 0.4267 | -0.0047 |
| AREG | -0.1866 | -0.247 | 0 | 0.4157 | 0 |
| PAH | -0.1544 | 0 | 0 | 0.402 | -0.0157 |
| NR1I2 | -0.3957 | 0 | 0 | 0.4 | 0 |
| FREM2 | -0.2196 | 0 | -0.0068 | 0.394 | 0 |
| CTTNBP2 | -0.388 | 0 | 0 | 0.3939 | 0 |
| GRM8 | -0.2247 | 0 | 0 | 0.3892 | -0.0425 |
| GNG4 | -0.2743 | -0.1998 | 0 | 0.3844 | 0 |
| LOC100288092 | -0.2227 | -0.081 | 0 | 0.3836 | 0 |
| PRR15 | -0.2621 | 0 | 0 | 0.3835 | -0.0293 |
| CFTR | -0.263 | -0.0358 | 0 | 0.3797 | 0 |
| BCL11A | -0.2415 | 0 | 0 | 0.3785 | 0 |
| ERP27 | -0.2191 | -0.0036 | 0 | 0.3765 | 0 |
| PLA2G12B | -0.1554 | -0.0835 | 0 | 0.3755 | 0 |
| SPIN3 | -0.3674 | 0 | -0.0715 | 0.3722 | 0 |
| GGH | -0.1254 | 0 | 0 | 0.3714 | -0.0036 |
| CACNA1D | -0.2586 | 0 | 0 | 0.3631 | -0.053 |
| ACE2 | -0.2602 | -0.1158 | 0 | 0.3627 | 0 |
| PTPRO | -0.3785 | -0.0308 | 0 | 0.3621 | 0 |
| MYRIP | -0.2183 | 0 | 0 | 0.3615 | -0.1623 |
| DPEP1 | -0.308 | 0 | -0.0196 | 0.3604 | 0 |
| PROX1 | -0.2279 | 0 | -0.0268 | 0.358 | -0.005 |
| SGK2 | -0.2529 | -0.0333 | 0.0661 | 0.3557 | -0.1121 |
| ZNRF3 | -0.1792 | 0 | -0.094 | 0.3532 | 0 |
| CAB39L | -0.3164 | -0.1037 | 0 | 0.353 | 0 |
| DDC | -0.091 | 0 | 0 | 0.3513 | -0.1222 |
| LRRC2 | -0.2247 | 0 | 0 | 0.3495 | 0 |
| REEP1 | -0.2705 | -0.1763 | 0 | 0.3453 | 0 |
| ID1 | -0.316 | 0 | 0 | 0.333 | -0.1871 |
| CYP2B6 | -0.1431 | -0.0106 | 0 | 0.3306 | 0 |
| LAMP2 | -0.1881 | -0.0428 | 0 | 0.3305 | 0 |
| PPP1R14C | -0.2419 | -0.0149 | 0 | 0.3242 | 0 |
| CBX5 | -0.2854 | 0 | 0 | 0.3214 | -0.0042 |
| NOX1 | -0.302 | 0 | 0 | 0.3212 | 0 |
| SLC22A3 | -0.1457 | 0 | -0.1395 | 0.3202 | 0 |
| TCFL5 | -0.0319 | -0.0874 | -0.0236 | 0.3179 | 0 |
| SATB2 | -0.196 | -0.1106 | 0 | 0.3163 | 0 |
| AREGB | -0.1052 | -0.0588 | 0 | 0.3124 | 0 |
| AZGP1 | -0.066 | 0 | 0 | 0.3118 | 0 |
| TMEM150C | -0.2715 | 0 | 0 | 0.3072 | 0 |
| LY75 | -0.0015 | -0.0399 | 0 | 0.3045 | -0.0527 |
| LOC647979 | -0.2257 | 0 | 0 | 0.3023 | 0 |
| LOC100128822 | -0.1782 | 0 | 0 | 0.3022 | 0 |

**Table 4**

| **Genes** | **Inflammatory** | **Goblet-like** | **Enterocyte** | **TA** | **Stem-like** |
|---|---|---|---|---|---|
| LY6G6D | -0.4827 | -0.278 | 0 | 0.645 | 0 |
| EREG | -0.237 | -0.5917 | 0 | 0.5346 | 0 |
| CEL | -0.3331 | -0.2754 | 0 | 0.513 | 0 |
| KRT23 | -0.3977 | -0.1715 | -0.0151 | 0.5018 | 0 |
| ACSL6 | -0.2707 | -0.1914 | 0 | 0.4946 | 0 |
| QPRT | -0.4772 | -0.0355 | 0 | 0.4905 | 0 |
| AXIN2 | -0.5204 | 0 | -0.041 | 0.4693 | 0 |
| ABAT | -0.419 | -0.196 | 0 | 0.4676 | 0 |
| FARP1 | -0.3463 | -0.1333 | 0 | 0.4618 | 0 |
| CELP | -0.2423 | -0.1824 | 0 | 0.4596 | 0 |
| C13orf18 | -0.4561 | -0.1986 | 0 | 0.4594 | 0 |
| HUNK | -0.3014 | 0 | 0 | 0.4551 | 0 |
| PLCB4 | -0.5302 | 0 | 0 | 0.4469 | 0 |
| APCDD1 | -0.3677 | 0 | -0.0421 | 0.4429 | 0 |
| RNF43 | -0.3522 | 0 | -0.0421 | 0.4419 | 0 |
| ASCL2 | -0.2372 | -0.0094 | 0 | 0.4368 | 0 |
| CHN2 | -0.3758 | 0 | 0 | 0.4267 | -0.0047 |
| AREG | -0.1866 | -0.247 | 0 | 0.4157 | 0 |
| PAH | -0.1544 | 0 | 0 | 0.402 | -0.0157 |
| NR1I2 | -0.3957 | 0 | 0 | 0.4 | 0 |

In a further embodiment of the present invention, preferred gene profile specific to "Stem-like" type of CRC are shown in Table 5 and more preferred gene profile specific to "Stem-like" type of CRC are shown in Table 6. The scores are illustrative only and represent expression profiles (tendencies) of listed genes. Positive score means high expression, negative score means low expression and zero means no change in expression.

**Table 5**

| **Genes** | **Inflammatory** | **Goblet-like** | **Enterocyte** | **TA** | **Stem-like** |
|---|---|---|---|---|---|
| SFRP2 | 0 | -0.3237 | -0.0307 | -0.2639 | 0.9198 |
| MGP | -0.0156 | -0.2349 | 0 | -0.1809 | 0.7443 |
| COL10A1 | 0 | -0.2045 | -0.1689 | -0.1652 | 0.7253 |
| MSRB3 | 0 | -0.2417 | 0 | -0.1456 | 0.7171 |
| CYP1B1 | 0 | -0.0613 | -0.1731 | -0.1959 | 0.6919 |
| FNDC1 | 0 | -0.2043 | -0.0783 | -0.0828 | 0.6894 |
| SFRP4 | 0 | -0.1449 | -0.1787 | 0 | 0.6878 |
| CCDC80 | 0 | -0.2075 | 0 | -0.1757 | 0.6772 |
| SPOCK1 | 0 | -0.1981 | -0.0783 | -0.1568 | 0.6726 |
| THBS2 | 0 | -0.2384 | -0.1937 | -0.0919 | 0.6622 |
| MFAP5 | -0.038 | -0.1853 | 0 | -0.0968 | 0.6545 |
| ASPN | 0 | -0.1971 | -0.0474 | -0.0832 | 0.6523 |
| TNS1 | 0 | -0.251 | 0 | -0.1417 | 0.6479 |
| TAGLN | 0 | -0.2068 | 0 | -0.1631 | 0.6451 |
| COMP | 0 | -0.0213 | -0.2292 | 0 | 0.6221 |
| NTM | 0 | -0.156 | -0.1646 | -0.1041 | 0.6122 |
| HOPX | 0 | -0.1899 | -0.0595 | -0.1683 | 0.6045 |
| AEBP1 | 0 | -0.1322 | -0.0542 | -0.1414 | 0.596 |
| PLN | 0 | -0.1551 | 0 | -0.1516 | 0.594 |
| FBN1 | 0 | -0.1951 | 0 | -0.1472 | 0.5937 |
| ANTXR1 | 0 | -0.1536 | 0 | -0.1127 | 0.5877 |
| MIR100HG | 0 | -0.1035 | 0 | -0.0684 | 0.5838 |
| PCDH7 | 0 | -0.1446 | 0 | -0.1002 | 0.5825 |
| DDR2 | 0 | -0.1712 | 0 | -0.1708 | 0.5791 |
| MYL9 | -0.0079 | -0.2503 | 0 | 0 | 0.5767 |
| FERMT2 | 0 | -0.1628 | 0 | -0.0848 | 0.5699 |
| VCAN | 0 | -0.1243 | 0 | -0.1048 | 0.557 |
| CDH11 | 0 | -0.0178 | 0 | -0.0787 | 0.5535 |
| FAP | 0 | -0.0726 | -0.1104 | -0.1726 | 0.544 |
| COL3A1 | 0 | -0.1255 | -0.0761 | -0.145 | 0.5437 |
| COL1A2 | 0 | -0.1251 | 0 | -0.1194 | 0.541 |
| TIMP2 | 0 | -0.1668 | 0 | -0.1667 | 0.5372 |
| BGN | 0 | -0.1576 | -0.0846 | -0.0992 | 0.5313 |
| GLT8D2 | 0 | -0.1068 | 0 | -0.1186 | 0.5301 |
| DCN | 0 | -0.1975 | 0 | -0.1426 | 0.5282 |
| FABP4 | 0 | -0.0557 | -0.0133 | -0.0637 | 0.5223 |
| FBLN1 | -0.0055 | -0.1684 | 0 | -0.0536 | 0.5197 |
| EFEMP1 | 0 | -0.1512 | 0 | -0.0935 | 0.5179 |
| VGLL3 | 0 | -0.1314 | -0.0875 | -0.1076 | 0.5177 |
| SPARC | 0 | -0.1647 | 0 | -0.0886 | 0.5134 |
| ITGBL1 | 0 | -0.084 | -0.162 | 0 | 0.5123 |
| AKAP12 | 0 | -0.1466 | 0 | -0.0646 | 0.5113 |
| INHBA | 0 | -0.0576 | -0.1452 | -0.0939 | 0.5113 |
| COL5A2 | 0 | -0.1516 | -0.0767 | -0.0742 | 0.508 |
| ISLR | 0 | -0.2185 | 0 | -0.0207 | 0.5012 |
| STON1 | 0 | -0.1002 | 0 | -0.0241 | 0.4967 |
| NOX4 | 0 | -0.0543 | -0.2136 | -0.0344 | 0.4961 |
| ECM2 | 0 | -0.0213 | -0.1591 | 0 | 0.4897 |
| LHFP | 0 | -0.0889 | 0 | -0.0575 | 0.4882 |
| SERPINF1 | 0 | -0.1386 | 0 | -0.1229 | 0.4827 |
| NNMT | 0.0158 | -0.014 | 0 | -0.2425 | 0.4801 |
| PTGIS | -0.0048 | -0.0911 | 0 | 0 | 0.4753 |
| MYLK | 0 | -0.1963 | 0 | -0.0459 | 0.4733 |
| MAP1B | 0 | -0.0398 | 0 | -0.0155 | 0.4723 |
| CALD1 | 0 | -0.1353 | 0 | -0.0784 | 0.4712 |
| GREM1 | 0 | -0.2299 | 0 | -0.2345 | 0.4697 |
| COL5A1 | 0 | -0.0655 | 0 | -0.1038 | 0.4643 |
| CNN1 | 0 | -0.0833 | 0 | -0.0431 | 0.4586 |
| TIMP3 | 0 | -0.3474 | 0 | 0 | 0.4561 |
| COL6A2 | 0 | -0.1303 | 0 | -0.2002 | 0.4545 |
| ZEB1 | 0 | -0.1147 | 0 | -0.021 | 0.4529 |
| PPAPDC1A | 0 | -0.0298 | -0.1981 | -0.0236 | 0.4488 |
| OLFML2B | 0 | -0.0555 | -0.1035 | -0.0691 | 0.4468 |
| HTRA1 | 0 | -0.0174 | -0.0398 | -0.0382 | 0.446 |
| CXCL12 | 0 | -0.1121 | 0 | -0.1192 | 0.4437 |
| DPYSL3 | 0 | 0 | -0.1589 | -0.0235 | 0.4429 |
| PDGFC | 0 | 0 | -0.0047 | -0.0611 | 0.4418 |
| COL6A3 | 0 | -0.1477 | 0 | -0.1135 | 0.4412 |
| COL1A1 | 0 | -0.1544 | -0.0401 | -0.0656 | 0.4386 |
| MYH11 | -0.1148 | -0.0855 | 0 | 0 | 0.4349 |
| AOC3 | 0 | -0.0871 | 0 | -0.0998 | 0.4342 |
| SPARCL1 | 0 | -0.1426 | 0 | -0.1981 | 0.4337 |
| COL12A1 | 0 | 0 | -0.0304 | -0.052 | 0.4335 |
| PHLDB2 | 0 | 0 | 0 | -0.2135 | 0.4252 |
| TPM2 | 0 | -0.1578 | 0 | 0 | 0.4211 |
| TGFB1I1 | 0 | 0 | 0 | -0.0459 | 0.4176 |
| MITF | 0 | -0.0391 | -0.0183 | -0.1459 | 0.4176 |
| GPC6 | 0 | -0.1575 | 0 | -0.0883 | 0.4147 |
| MMP2 | 0 | -0.0659 | 0 | -0.1281 | 0.4117 |
| FIBIN | 0 | -0.0109 | -0.0755 | 0 | 0.4042 |
| TMEM47 | 0 | -0.1747 | 0 | 0 | 0.4029 |
| IGFBP5 | 0 | -0.2509 | 0 | -0.0818 | 0.4019 |
| MXRA5 | 0 | -0.0623 | -0.0753 | -0.0343 | 0.4002 |
| EPYC | 0 | 0 | -0.1321 | 0 | 0.3959 |
| COL15A1 | 0 | -0.1229 | 0 | -0.1803 | 0.3944 |
| LMOD1 | 0 | -0.0425 | 0 | 0 | 0.3918 |
| FN1 | 0 | -0.2329 | -0.1076 | 0 | 0.3918 |
| DPT | 0 | -0.0621 | 0 | 0 | 0.3875 |
| TWIST1 | 0 | -0.0737 | 0 | -0.025 | 0.383 |
| SDC2 | 0 | -0.1134 | 0 | 0 | 0.3813 |
| FLRT2 | 0 | -0.0736 | 0 | -0.0084 | 0.3785 |
| LOXL1 | 0 | 0 | -0.0529 | -0.1304 | 0.378 |
| SSPN | 0 | 0 | 0 | -0.0767 | 0.3766 |
| MAB21L2 | 0 | -0.1029 | 0 | -0.0181 | 0.3766 |
| CTSK | 0 | -0.1202 | 0 | -0.0744 | 0.3744 |
| WWTR1 | 0 | -0.2317 | 0 | -0.0362 | 0.3733 |
| CYBRD1 | 0 | -0.0729 | 0 | -0.0995 | 0.3698 |
| SYNM | -0.0337 | -0.0625 | 0 | 0 | 0.3631 |
| SNAI2 | 0 | -0.0809 | 0 | -0.0788 | 0.3621 |
| DES | 0 | -0.0091 | 0 | 0 | 0.3555 |
| IGF1 | -0.0545 | -0.0135 | 0 | 0 | 0.3541 |
| TNC | 0 | -0.1523 | 0 | -0.1472 | 0.3536 |
| GUCY1A3 | 0 | -0.1738 | 0 | -0.0524 | 0.3485 |
| GULP1 | 0 | -0.1608 | 0 | -0.0069 | 0.3466 |
| AHNAK2 | 0 | 0 | -0.1033 | -0.0605 | 0.3429 |
| ACTG2 | -0.0116 | -0.0764 | 0 | -0.0126 | 0.3424 |
| KAL1 | 0 | -0.0134 | -0.0873 | -0.0238 | 0.3335 |
| FLNA | 0 | -0.1291 | 0 | 0 | 0.3331 |
| CYR61 | 0 | -0.0167 | 0 | -0.1405 | 0.3302 |
| RBMS1 | 0 | -0.3082 | 0 | 0 | 0.3235 |
| SMARCA1 | 0 | 0 | -0.0933 | 0 | 0.3205 |
| MMP11 | 0 | -0.0637 | 0 | 0 | 0.3058 |
| SRPX | 0 | -0.0028 | 0 | -0.0784 | 0.3017 |
| EDNRA | 0 | -0.1676 | -0.0174 | 0 | 0.301 |
| THBS1 | 0 | -0.2428 | 0 | 0 | 0.3 |

**Table 6**

| **Genes** | **Inflammatory** | **Goblet-like** | **Enterocyte** | **TA** | **Stem-like** |
|---|---|---|---|---|---|
| SFRP2 | 0 | -0.3237 | -0.0307 | -0.2639 | 0.9198 |
| MGP | -0.0156 | -0.2349 | 0 | -0.1809 | 0.7443 |
| COL10A1 | 0 | -0.2045 | -0.1689 | -0.1652 | 0.7253 |
| MSRB3 | 0 | -0.2417 | 0 | -0.1456 | 0.7171 |
| CYP1B1 | 0 | -0.0613 | -0.1731 | -0.1959 | 0.6919 |
| FNDC1 | 0 | -0.2043 | -0.0783 | -0.0828 | 0.6894 |
| SFRP4 | 0 | -0.1449 | -0.1787 | 0 | 0.6878 |
| CCDC80 | 0 | -0.2075 | 0 | -0.1757 | 0.6772 |
| SPOCK1 | 0 | -0.1981 | -0.0783 | -0.1568 | 0.6726 |
| THBS2 | 0 | -0.2384 | -0.1937 | -0.0919 | 0.6622 |
| MFAP5 | -0.038 | -0.1853 | 0 | -0.0968 | 0.6545 |
| ASPN | 0 | -0.1971 | -0.0474 | -0.0832 | 0.6523 |
| TNS1 | 0 | -0.251 | 0 | -0.1417 | 0.6479 |
| TAGLN | 0 | -0.2068 | 0 | -0.1631 | 0.6451 |
| COMP | 0 | -0.0213 | -0.2292 | 0 | 0.6221 |
| NTM | 0 | -0.156 | -0.1646 | -0.1041 | 0.6122 |
| HOPX | 0 | -0.1899 | -0.0595 | -0.1683 | 0.6045 |
| AEBP1 | 0 | -0.1322 | -0.0542 | -0.1414 | 0.596 |
| PLN | 0 | -0.1551 | 0 | -0.1516 | 0.594 |
| FBN1 | 0 | -0.1951 | 0 | -0.1472 | 0.5937 |
| ANTXR1 | 0 | -0.1536 | 0 | -0.1127 | 0.5877 |
| MIR100HG | 0 | -0.1035 | 0 | -0.0684 | 0.5838 |
| PCDH7 | 0 | -0.1446 | 0 | -0.1002 | 0.5825 |
| DDR2 | 0 | -0.1712 | 0 | -0.1708 | 0.5791 |
| MYL9 | -0.0079 | -0.2503 | 0 | 0 | 0.5767 |
| FERMT2 | 0 | -0.1628 | 0 | -0.0848 | 0.5699 |
| VCAN | 0 | -0.1243 | 0 | -0.1048 | 0.557 |
| CDH11 | 0 | -0.0178 | 0 | -0.0787 | 0.5535 |
| FAP | 0 | -0.0726 | -0.1104 | -0.1726 | 0.544 |
| COL3A1 | 0 | -0.1255 | -0.0761 | -0.145 | 0.5437 |
| COL1A2 | 0 | -0.1251 | 0 | -0.1194 | 0.541 |
| TIMP2 | 0 | -0.1668 | 0 | -0.1667 | 0.5372 |
| BGN | 0 | -0.1576 | -0.0846 | -0.0992 | 0.5313 |
| GLT8D2 | 0 | -0.1068 | 0 | -0.1186 | 0.5301 |
| DCN | 0 | -0.1975 | 0 | -0.1426 | 0.5282 |
| FABP4 | 0 | -0.0557 | -0.0133 | -0.0637 | 0.5223 |
| FBLN1 | -0.0055 | -0.1684 | 0 | -0.0536 | 0.5197 |
| EFEMP1 | 0 | -0.1512 | 0 | -0.0935 | 0.5179 |
| VGLL3 | 0 | -0.1314 | -0.0875 | -0.1076 | 0.5177 |
| SPARC | 0 | -0.1647 | 0 | -0.0886 | 0.5134 |
| ITGBL1 | 0 | -0.084 | -0.162 | 0 | 0.5123 |
| AKAP12 | 0 | -0.1466 | 0 | -0.0646 | 0.5113 |
| INHBA | 0 | -0.0576 | -0.1452 | -0.0939 | 0.5113 |
| COL5A2 | 0 | -0.1516 | -0.0767 | -0.0742 | 0.508 |
| ISLR | 0 | -0.2185 | 0 | -0.0207 | 0.5012 |
| STON1 | 0 | -0.1002 | 0 | -0.0241 | 0.4967 |
| NOX4 | 0 | -0.0543 | -0.2136 | -0.0344 | 0.4961 |
| ECM2 | 0 | -0.0213 | -0.1591 | 0 | 0.4897 |
| LHFP | 0 | -0.0889 | 0 | -0.0575 | 0.4882 |
| SERPINF1 | 0 | -0.1386 | 0 | -0.1229 | 0.4827 |
| NNMT | 0.0158 | -0.014 | 0 | -0.2425 | 0.4801 |
| PTGIS | -0.0048 | -0.0911 | 0 | 0 | 0.4753 |
| MYLK | 0 | -0.1963 | 0 | -0.0459 | 0.4733 |
| MAP1B | 0 | -0.0398 | 0 | -0.0155 | 0.4723 |
| CALD1 | 0 | -0.1353 | 0 | -0.0784 | 0.4712 |
| GREM1 | 0 | -0.2299 | 0 | -0.2345 | 0.4697 |
| COL5A1 | 0 | -0.0655 | 0 | -0.1038 | 0.4643 |
| CNN1 | 0 | -0.0833 | 0 | -0.0431 | 0.4586 |
| TIMP3 | 0 | -0.3474 | 0 | 0 | 0.4561 |
| COL6A2 | 0 | -0.1303 | 0 | -0.2002 | 0.4545 |
| ZEB1 | 0 | -0.1147 | 0 | -0.021 | 0.4529 |
| PPAPDC1A | 0 | -0.0298 | -0.1981 | -0.0236 | 0.4488 |
| OLFML2B | 0 | -0.0555 | -0.1035 | -0.0691 | 0.4468 |
| HTRA1 | 0 | -0.0174 | -0.0398 | -0.0382 | 0.446 |
| CXCL12 | 0 | -0.1121 | 0 | -0.1192 | 0.4437 |
| DPYSL3 | 0 | 0 | -0.1589 | -0.0235 | 0.4429 |
| PDGFC | 0 | 0 | -0.0047 | -0.0611 | 0.4418 |
| COL6A3 | 0 | -0.1477 | 0 | -0.1135 | 0.4412 |
| COL1A1 | 0 | -0.1544 | -0.0401 | -0.0656 | 0.4386 |
| MYH11 | -0.1148 | -0.0855 | 0 | 0 | 0.4349 |
| AOC3 | 0 | -0.0871 | 0 | -0.0998 | 0.4342 |
| SPARCL1 | 0 | -0.1426 | 0 | -0.1981 | 0.4337 |
| COL12A1 | 0 | 0 | -0.0304 | -0.052 | 0.4335 |
| PHLDB2 | 0 | 0 | 0 | -0.2135 | 0.4252 |
| TPM2 | 0 | -0.1578 | 0 | 0 | 0.4211 |
| TGFB1I1 | 0 | 0 | 0 | -0.0459 | 0.4176 |
| MITF | 0 | -0.0391 | -0.0183 | -0.1459 | 0.4176 |
| GPC6 | 0 | -0.1575 | 0 | -0.0883 | 0.4147 |
| MMP2 | 0 | -0.0659 | 0 | -0.1281 | 0.4117 |
| FIBIN | 0 | -0.0109 | -0.0755 | 0 | 0.4042 |
| TMEM47 | 0 | -0.1747 | 0 | 0 | 0.4029 |
| IGFBP5 | 0 | -0.2509 | 0 | -0.0818 | 0.4019 |
| MXRA5 | 0 | -0.0623 | -0.0753 | -0.0343 | 0.4002 |

In a further embodiment of the present invention, preferred gene profile specific to "Inflammatory" type of CRC are shown in Table 7 and more preferred gene profile specific to "Inflammatory" type of CRC are shown in Table 8. The scores are illustrative only and represent expression profiles (tendencies) of listed genes. Positive score means high expression, negative score means low expression and zero means no change in expression.

**Table 7**

| **Genes** | **Inflammatory** | **Goblet-like** | **Enterocyte** | **TA** | **Stem-like** |
|---|---|---|---|---|---|
| CXCL13 | 0.6598 | -0.0547 | 0 | -0.3261 | 0 |
| RARRES3 | 0.6349 | 0 | 0 | -0.3114 | -0.0032 |
| IDO1 | 0.623 | 0 | -0.0271 | -0.0666 | -0.0463 |
| GZMA | 0.5844 | 0 | 0 | -0.2481 | -0.0511 |
| CXCL9 | 0.5802 | -0.0267 | -0.0512 | -0.1435 | 0 |
| CXCL10 | 0.5602 | 0 | -0.0101 | -0.1727 | 0 |
| GBP4 | 0.5585 | 0 | -0.021 | -0.0638 | -0.0303 |
| CCL5 | 0.555 | 0 | 0 | -0.2188 | 0 |
| GNLY | 0.5501 | 0 | 0 | -0.0816 | -0.1053 |
| GBP1 | 0.542 | -0.0522 | 0 | -0.1665 | 0 |
| SAMD9L | 0.5309 | 0 | 0 | -0.2992 | 0 |
| GBP5 | 0.5305 | -0.0272 | -0.0609 | -0.0807 | 0 |
| HSPA4L | 0.5182 | 0 | -0.1598 | 0 | -0.1385 |
| BIRC3 | 0.5138 | 0 | 0 | -0.1868 | 0 |
| CXCL11 | 0.5135 | 0 | -0.0231 | -0.124 | 0 |
| FAM26F | 0.4872 | -0.0222 | 0 | -0.1155 | 0 |
| APOBEC3G | 0.4848 | -0.0503 | 0 | -0.1064 | 0 |
| HLA-DPA1 | 0.4643 | 0 | 0 | -0.2218 | 0 |
| STAT1 | 0.4618 | 0 | -0.0287 | -0.0459 | 0 |
| HOXC6 | 0.4614 | 0 | -0.2736 | -0.1476 | 0 |
| HLA-DMA | 0.4523 | 0 | 0 | -0.2243 | 0 |
| BST2 | 0.452 | 0 | 0 | -0.1546 | 0 |
| KYNU | 0.4477 | 0 | -0.1171 | -0.2248 | 0 |
| ZIC2 | 0.4384 | 0 | 0 | -0.1479 | -0.0707 |
| IFIT3 | 0.4373 | -0.0103 | 0 | -0.1593 | 0 |
| AIM2 | 0.4266 | -0.0029 | -0.0266 | 0 | 0 |
| CCL4 | 0.4231 | 0 | 0 | -0.1309 | 0 |
| HLA-DMB | 0.4225 | 0 | 0 | -0.1551 | 0 |
| SRSF6 | 0.4085 | 0 | 0 | 0 | -0.1829 |
| C1QA | 0.3952 | 0 | 0 | -0.2348 | 0 |
| HLA-DRA | 0.3932 | 0 | 0 | -0.2883 | 0 |
| SAMSN1 | 0.3845 | -0.0402 | 0 | -0.2621 | 0 |
| HLA-DPB1 | 0.3808 | 0 | 0 | -0.2387 | 0 |
| IFI44 | 0.3758 | 0 | -0.0372 | -0.1073 | 0 |
| CD74 | 0.3618 | 0 | 0 | -0.1756 | 0 |
| ISG15 | 0.3552 | -0.0342 | 0 | -0.0549 | 0 |
| SLAMF7 | 0.3524 | 0 | 0 | -0.1563 | 0 |
| RPL22L1 | 0.3511 | 0 | -0.1602 | 0 | -0.0111 |
| PSMB9 | 0.344 | 0 | 0 | 0 | -0.0511 |
| LCK | 0.3433 | 0 | 0 | -0.0793 | 0 |
| MICB | 0.3382 | 0 | 0 | -0.0541 | 0 |
| XAF1 | 0.3326 | 0 | 0 | -0.0974 | 0 |
| TRIM22 | 0.3318 | 0 | 0 | -0.2338 | 0 |
| PIWIL1 | 0.3311 | 0 | -0.0683 | 0 | -0.0161 |
| MMP12 | 0.3309 | 0 | 0 | -0.1033 | 0 |
| TLR8 | 0.3273 | 0 | -0.0601 | -0.0645 | 0 |
| FYB | 0.3253 | 0 | 0 | -0.1719 | 0 |
| TNFSF9 | 0.3207 | 0 | -0.08 | 0 | -0.0023 |
| PLA2G7 | 0.3203 | -0.0667 | 0 | -0.0884 | 0 |
| MT2A | 0.317 | 0 | 0 | -0.3213 | 0 |
| IFIT2 | 0.3144 | -0.0687 | 0 | -0.0836 | 0 |
| BAG2 | 0.313 | 0 | 0 | -0.1522 | 0 |
| IGF2BP3 | 0.3078 | 0 | -0.1765 | -0.0041 | 0 |
| LY6E | 0.3066 | -0.0951 | -0.0177 | 0 | 0 |
| TRBC1 | 0.3059 | 0 | 0 | -0.1337 | 0 |
| PMAIP1 | 0.3003 | -0.0328 | -0.2642 | 0 | 0 |

**Table 8**

| **Genes** | **Inflammatory** | **Goblet-like** | **Enterocyte** | **TA** | **Stem-like** |
|---|---|---|---|---|---|
| CXCL13 | 0.6598 | -0.0547 | 0 | -0.3261 | 0 |
| RARRES3 | 0.6349 | 0 | 0 | -0.3114 | -0.0032 |
| IDO1 | 0.623 | 0 | -0.0271 | -0.0666 | -0.0463 |
| GZMA | 0.5844 | 0 | 0 | -0.2481 | -0.0511 |
| CXCL9 | 0.5802 | -0.0267 | -0.0512 | -0.1435 | 0 |
| CXCL10 | 0.5602 | 0 | -0.0101 | -0.1727 | 0 |
| GBP4 | 0.5585 | 0 | -0.021 | -0.0638 | -0.0303 |
| CCL5 | 0.555 | 0 | 0 | -0.2188 | 0 |
| GNLY | 0.5501 | 0 | 0 | -0.0816 | -0.1053 |
| GBP1 | 0.542 | -0.0522 | 0 | -0.1665 | 0 |
| SAMD9L | 0.5309 | 0 | 0 | -0.2992 | 0 |
| GBP5 | 0.5305 | -0.0272 | -0.0609 | -0.0807 | 0 |
| HSPA4L | 0.5182 | 0 | -0.1598 | 0 | -0.1385 |
| BIRC3 | 0.5138 | 0 | 0 | -0.1868 | 0 |
| CXCL11 | 0.5135 | 0 | -0.0231 | -0.124 | 0 |
| FAM26F | 0.4872 | -0.0222 | 0 | -0.1155 | 0 |
| APOBEC3G | 0.4848 | -0.0503 | 0 | -0.1064 | 0 |
| HLA-DPA1 | 0.4643 | 0 | 0 | -0.2218 | 0 |
| STAT1 | 0.4618 | 0 | -0.0287 | -0.0459 | 0 |
| HOXC6 | 0.4614 | 0 | -0.2736 | -0.1476 | 0 |
| HLA-DMA | 0.4523 | 0 | 0 | -0.2243 | 0 |
| BST2 | 0.452 | 0 | 0 | -0.1546 | 0 |
| KYNU | 0.4477 | 0 | -0.1171 | -0.2248 | 0 |
| ZIC2 | 0.4384 | 0 | 0 | -0.1479 | -0.0707 |
| IFIT3 | 0.4373 | -0.0103 | 0 | -0.1593 | 0 |
| AIM2 | 0.4266 | -0.0029 | -0.0266 | 0 | 0 |
| CCL4 | 0.4231 | 0 | 0 | -0.1309 | 0 |
| HLA-DMB | 0.4225 | 0 | 0 | -0.1551 | 0 |
| SRSF6 | 0.4085 | 0 | 0 | 0 | -0.1829 |

In a further embodiment of the present invention, preferred gene profile specific to "Goblet-like" type of CRC are shown in Table 9 and more preferred gene profile specific to "Goblet-like" type of CRC are shown in Table 10. The scores are illustrative only and represent expression profiles (tendencies) of listed genes. Positive score means high expression, negative score means low expression and zero means no change in expression.

**Table 9**

| **Genes** | **Inflammatory** | **Goblet-like** | **Enterocyte** | **TA** | **Stem-like** |
|---|---|---|---|---|---|
| SLITRK6 | 0 | 0.7207 | 0 | -0.1004 | -0.1461 |
| PCSK1 | -0.0942 | 0.6432 | 0 | 0 | -0.0982 |
| L1TD1 | 0 | 0.612 | 0 | 0 | -0.2121 |
| KIAA1324 | -0.0184 | 0.5977 | 0 | 0 | -0.2625 |
| AQP3 | 0 | 0.5962 | 0 | -0.1349 | -0.168 |
| TOX | 0 | 0.5824 | 0 | -0.1544 | -0.0849 |
| PCCA | -0.1223 | 0.4963 | 0 | 0 | 0 |
| SERPINA1 | 0 | 0.4894 | 0 | -0.0715 | -0.1033 |
| DEFA5 | -0.2474 | 0.4559 | 0 | 0.0037 | -0.1204 |
| SMAD9 | -0.2885 | 0.4111 | 0 | 0 | 0 |
| REG3A | 0 | 0.4103 | 0 | 0 | -0.1839 |
| DUSP4 | 0.3838 | 0.4023 | -0.2509 | -0.3094 | 0 |
| C8orf84 | 0 | 0.4015 | -0.2297 | 0 | 0 |
| TFF1 | 0 | 0.3995 | 0 | -0.0641 | -0.258 |
| EPHA4 | 0 | 0.3921 | 0 | -0.1083 | 0 |
| MUC4 | 0 | 0.3886 | 0.473 | -0.3711 | -0.2766 |
| CPS1 | 0 | 0.3852 | -0.0506 | -0.0104 | -0.0604 |
| REG1A | 0 | 0.38 | 0.1686 | -0.1393 | -0.227 |
| HSPA2 | -0.0874 | 0.3733 | 0 | -0.1218 | 0 |
| SLAIN1 | 0 | 0.3567 | -0.063 | 0 | 0 |
| FOXA3 | 0 | 0.3462 | 0 | 0 | -0.2796 |
| KLK11 | 0 | 0.3415 | 0 | 0 | 0 |
| PRUNE2 | -0.1432 | 0.3397 | 0 | 0 | 0 |
| TFF3 | -0.0114 | 0.3321 | 0 | 0 | -0.1107 |
| DEFA6 | -0.2476 | 0.316 | 0 | 0.1359 | -0.1375 |
| C11orf93 | 0 | 0.3045 | 0 | 0 | -0.0334 |

**Table 10**

| **Genes** | **Inflammatory** | **Goblet-like** | **Enterocyte** | **TA** | **Stem-like** |
|---|---|---|---|---|---|
| SLITRK6 | 0 | 0.7207 | 0 | -0.1004 | -0.1461 |
| PCSK1 | -0.0942 | 0.6432 | 0 | 0 | -0.0982 |
| L1TD1 | 0 | 0.612 | 0 | 0 | -0.2121 |
| KIAA1324 | -0.0184 | 0.5977 | 0 | 0 | -0.2625 |
| AQP3 | 0 | 0.5962 | 0 | -0.1349 | -0.168 |
| TOX | 0 | 0.5824 | 0 | -0.1544 | -0.0849 |
| PCCA | -0.1223 | 0.4963 | 0 | 0 | 0 |
| SERPINA1 | 0 | 0.4894 | 0 | -0.0715 | -0.1033 |
| DEFA5 | -0.2474 | 0.4559 | 0 | 0.0037 | -0.1204 |
| SMAD9 | -0.2885 | 0.4111 | 0 | 0 | 0 |
| REG3A | 0 | 0.4103 | 0 | 0 | -0.1839 |
| DUSP4 | 0.3838 | 0.4023 | -0.2509 | -0.3094 | 0 |
| C8orf84 | 0 | 0.4015 | -0.2297 | 0 | 0 |

In a further embodiment of the present invention, preferred gene profile specific to "Enterocyte" type of CRC are shown in Table 11 and more preferred gene profile specific to "Enterocyte" type of CRC are shown in Table 12. The scores are illustrative only and represent expression profiles (tendencies) of listed genes. Positive score means high expression, negative score means low expression and zero means no change in expression.

**Table 11**

| **Genes** | **Inflammatory** | **Goblet-like** | **Enterocyte** | **TA** | **Stem-like** |
|---|---|---|---|---|---|
| CLCA4 | -0.1441 | -0.101 | 1.324 | -0.0431 | -0.3051 |
| ZG16 | -0.3175 | 0 | 1.3204 | -0.1071 | -0.2667 |
| MS4A12 | -0.2593 | -0.1311 | 1.2784 | 0 | -0.2202 |
| CA1 | -0.196 | -0.1521 | 1.2105 | -0.0218 | -0.1455 |
| CA4 | -0.1446 | -0.2072 | 1.2041 | 0 | -0.237 |
| CLDN8 | -0.1183 | -0.0935 | 0.9693 | -0.014 | -0.0823 |
| SLC4A4 | 0 | 0 | 0.9287 | -0.3551 | -0.3035 |
| CA2 | 0 | 0 | 0.8804 | -0.2652 | -0.2374 |
| SI | 0 | 0 | 0.8022 | -0.2301 | -0.1674 |
| LOC646627 | -0.3265 | 0 | 0.7794 | 0 | -0.109 |
| CEACAM7 | -0.0831 | 0 | 0.7596 | 0 | -0.2754 |
| ADH1C | -0.091 | 0 | 0.7543 | -0.0013 | -0.1841 |
| AQP8 | -0.1371 | -0.0965 | 0.7376 | 0 | -0.1221 |
| DHRS9 | 0 | 0 | 0.7298 | -0.0865 | -0.1695 |
| GCG | -0.0738 | 0 | 0.7129 | -0.0109 | -0.1323 |
| B3GNT7 | -0.0364 | 0 | 0.7118 | -0.0505 | -0.118 |
| PKIB | 0 | -0.0011 | 0.582 | -0.053 | -0.1693 |
| PYY | 0 | 0 | 0.5742 | 0 | -0.1178 |
| MT1M | 0 | 0 | 0.5724 | -0.2344 | 0 |
| TRPM6 | -0.3032 | -0.0554 | 0.5496 | 0 | 0 |
| SPINK5 | 0 | 0 | 0.5453 | 0 | -0.1555 |
| CD 177 | 0 | 0 | 0.5419 | -0.0409 | -0.0998 |
| UGT2B17 | -0.1314 | 0 | 0.5002 | 0 | -0.1077 |
| AKR1B10 | -0.0211 | 0 | 0.4981 | 0 | -0.2302 |
| IGJ | 0 | 0 | 0.4954 | -0.1697 | -0.0192 |
| HSD17B2 | 0 | 0 | 0.4943 | -0.0852 | -0.2412 |
| UGT2A3 | -0.2532 | -0.1099 | 0.4822 | 0.1256 | -0.0811 |
| FAM55D | -0.2802 | 0 | 0.4629 | 0 | -0.0429 |
| MFSD4 | -0.0688 | 0 | 0.454 | -0.0054 | -0.0565 |
| PCK1 | -0.1878 | -0.0101 | 0.4506 | 0 | 0 |
| EDN3 | -0.0565 | 0 | 0.4389 | 0 | -0.0176 |
| CPM | 0 | 0 | 0.404 | -0.0779 | 0 |
| SEMA6D | -0.0696 | -0.0805 | 0.3871 | 0 | -0.0194 |
| TMEM37 | -0.0307 | 0 | 0.3738 | 0 | -0.1004 |
| SCARA5 | -0.1256 | 0 | 0.3734 | 0 | -0.0207 |
| METTL7A | -0.2028 | 0 | 0.3536 | 0 | 0 |
| HPGD | 0 | -0.051 | 0.349 | 0 | 0 |
| NR5A2 | 0 | -0.008 | 0.3158 | 0 | -0.1386 |
| HHLA2 | -0.0057 | 0 | 0.3149 | 0 | -0.2068 |
| CLDN23 | 0 | -0.0834 | 0.3063 | 0 | -0.0148 |
| XDH | 0 | 0 | 0.3061 | 0 | -0.3508 |
| LGALS2 | 0 | 0 | 0.3059 | -0.0276 | -0.0821 |

**Table 12**

| **Genes** | **Inflammatory** | **Goblet-like** | **Enterocyte** | **TA** | **Stem-like** |
|---|---|---|---|---|---|
| CLCA4 | -0.1441 | -0.101 | 1.324 | -0.0431 | -0.3051 |
| ZG16 | -0.3175 | 0 | 1.3204 | -0.1071 | -0.2667 |
| MS4A12 | -0.2593 | -0.1311 | 1.2784 | 0 | -0.2202 |
| CA1 | -0.196 | -0.1521 | 1.2105 | -0.0218 | -0.1455 |
| CA4 | -0.1446 | -0.2072 | 1.2041 | 0 | -0.237 |
| CLDN8 | -0.1183 | -0.0935 | 0.9693 | -0.014 | -0.0823 |
| SLC4A4 | 0 | 0 | 0.9287 | -0.3551 | -0.3035 |
| CA2 | 0 | 0 | 0.8804 | -0.2652 | -0.2374 |
| SI | 0 | 0 | 0.8022 | -0.2301 | -0.1674 |
| LOC646627 | -0.3265 | 0 | 0.7794 | 0 | -0.109 |
| CEACAM7 | -0.0831 | 0 | 0.7596 | 0 | -0.2754 |
| ADH1C | -0.091 | 0 | 0.7543 | -0.0013 | -0.1841 |
| AQP8 | -0.1371 | -0.0965 | 0.7376 | 0 | -0.1221 |
| DHRS9 | 0 | 0 | 0.7298 | -0.0865 | -0.1695 |
| GCG | -0.0738 | 0 | 0.7129 | -0.0109 | -0.1323 |
| B3GNT7 | -0.0364 | 0 | 0.7118 | -0.0505 | -0.118 |
| PKIB | 0 | -0.0011 | 0.582 | -0.053 | -0.1693 |
| PYY | 0 | 0 | 0.5742 | 0 | -0.1178 |
| MT1M | 0 | 0 | 0.5724 | -0.2344 | 0 |
| TRPM6 | -0.3032 | -0.0554 | 0.5496 | 0 | 0 |
| SPINK5 | 0 | 0 | 0.5453 | 0 | -0.1555 |
| CD 177 | 0 | 0 | 0.5419 | -0.0409 | -0.0998 |
| UGT2B17 | -0.1314 | 0 | 0.5002 | 0 | -0.1077 |
| AKR1B10 | -0.0211 | 0 | 0.4981 | 0 | -0.2302 |
| IGJ | 0 | 0 | 0.4954 | -0.1697 | -0.0192 |
| HSD17B2 | 0 | 0 | 0.4943 | -0.0852 | -0.2412 |
| UGT2A3 | -0.2532 | -0.1099 | 0.4822 | 0.1256 | -0.0811 |
| FAM55D | -0.2802 | 0 | 0.4629 | 0 | -0.0429 |
| MFSD4 | -0.0688 | 0 | 0.454 | -0.0054 | -0.0565 |
| PCK1 | -0.1878 | -0.0101 | 0.4506 | 0 | 0 |
| EDN3 | -0.0565 | 0 | 0.4389 | 0 | -0.0176 |
| CPM | 0 | 0 | 0.404 | -0.0779 | 0 |

In Figure 1A, CRC samples are arranged by NMF classes in a 'heatmap' to illustrate SAM- and PAM identified gene sets unique to each subtype. Comparable profiles were found in six independent open-access datasets (n=399 and Table 1). Notably, four of the five subtypes are present (Figure 1B) among a panel of CRC cell lines (n=51) and these predictions from CRC cell lines were confirmed using xenograft animal models (n=3, Figure 6), a finding that could enable evaluation of differential drug sensitivities amongst the subtypes.

To determine if particular CRC subtypes amongst the five Applicants identified are associated with survival, Applicants evaluated one of the core CRC datasets, GSE14333, which included disease-free survival (DFS; n=197) information. In this dataset, the median follow up among patients without events was 45.1 months. Applicants first evaluated DFS for all the samples irrespective of their treatments (adjuvant radiation and/or chemotherapy) or Duke's stage (combined Duke's stage A or B and considered C separately), the later of which is known to correlate with CRC-specific survival. Applicants found no significant association of subtypes with DFS (p=0.12; log-rank test; Figure 7A). However, Applicants observed that treatment (p=0.03) and Duke's stage (p=0.0009; log-rank test) were significantly associated with DFS. Applicants also observed that treatment was significantly associated with Duke's stage (p=1.98x10⁻⁴, Fisher's exact test). Since Applicants observed that treatment and Duke's stage were associated with DFS, Applicants examined whether subtype was associated with DFS within subsets defined by these variables. In untreated patients, there was a significant difference amongst the five subtypes in regard to DFS (p=0.0003; log-rank test; n=120). Specifically, stem-like subtype tumors had the shortest DFS (Figure 1C). On the other hand, there is no significant association between subtypes and DFS (p=0.9; log-rank test; n=77) in the treated patients. Similarly, Applicants did not find significant association between subtypes and DFS either in samples with only Duke's stage A or B (p=0.13; n=119) or those with only Duke's stage C (p=0.7; log-rank test; n=98). Since the total number of events for all the samples was only 43, and it was lower in subtypes, more patient samples are needed to fully elucidate the relationship between subtype and DFS.

In an embodiment, the present invention provides an *in-vitro* method for the prognosis of disease-free survival of a subject suffering from colorectal cancer or suspected of suffering therefrom and who has undergone a prior surgical resection of colorectal cancer, the method comprising
(i) providing a biological sample from said subject comprising colorectal cancer cells or suspected to comprise colorectal cancer cells;
(ii) measuring the expression level of one or a combination of genes selected from the group of genes listed in Table 2, and
(iii) classifying said biological sample as "Stem-like", "Inflammatory", "Transit-amplifying (TA)", "Goblet-like" and "Enterocyte" on the basis of the gene expression profile according to Table 2,
wherein
- "Stem-like" type of colorectal cancer indicates poor disease-free survival,
- "Inflammatory" type of colorectal cancer indicates intermediate disease-free survival,
- "Transit-amplifying (TA)" type of colorectal cancer indicates good disease-free survival,
- "Goblet-like" type of colorectal cancer indicates good disease-free survival, and
- "Enterocyte" type of colorectal cancer indicates intermediate disease-free survival.

A further aspect comprises the combination of genes comprising at least two genes selected from Table 2, or at least five genes selected from Table 2, or at least 10 genes selected from Table 2, or at least 20 genes that are selected from Table 2, more preferred at least 30 genes that are selected from Table 2, more preferred at least 40 genes that are selected from Table 2, more preferred at least 50 genes that are selected from Table 2, more preferred at least 60 genes that are selected from Table 2, more preferred at least 70 genes that are selected from Table 2, more preferred at least 80 genes that are selected from Table 2, more preferred at least 90 genes that are selected from Table 2, more preferred at least 100 genes that are selected from Table 2, more preferred at least 120 genes that are selected from Table 2, more preferred at least 140 genes that are selected from Table 2, more preferred at least 160 genes that are selected from Table 2, more preferred at least 180 genes that are selected from Table 2, more preferred at least 200 genes that are selected from Table 2, more preferred at least 220 genes that are selected from Table 2, more preferred at least 240 genes that are selected from Table 2, more preferred at least 260 genes that are selected from Table 2, more preferred at least 280 genes that are selected from Table 2, more preferred at least 300 genes that are selected from Table 2, more preferred at least 320 genes that are selected from Table 2, more preferred at least 340 genes that are selected from Table 2, more preferred at least 360 genes that are selected from Table 2, more preferred at least 380 genes that are selected from Table 2, more preferred at least 400 genes that are selected from Table 2, more preferred at least 420 genes that are selected from Table 2, more preferred at least 460 genes that are selected from Table 2, more preferred at least 480 genes that are selected from Table 2, more preferred at least 500 genes that are selected from Table 2, more preferred at least 520 genes that are selected from Table 2, more preferred at least 540 genes that are selected from Table 2, more preferred at least 560 genes that are selected from Table 2, more preferred at least 580 genes that are selected from Table 2, more preferred at least 600 genes that are selected from Table 2, more preferred at least 620 genes that are selected from Table 2, more preferred at least 640 genes that are selected from Table 2, more preferred at least 660 genes that are selected from Table 2, more preferred at least 680 genes that are selected from Table 2, more preferred at least 700 genes that are selected from Table 2, more preferred at least 720 genes that are selected from Table 2, more preferred at least 740 genes that are selected from Table 2, more preferred at least 760 genes that are selected from Table 2.

In a further preferred embodiment, a method of the invention comprises the combination of genes selected from all 786 genes of Table 2.

More preferably the combination of genes comprises at least two, or at least five, or at least 10, or at least 20, or at least 30, or at least 40 genes selected from Table 2.

Preferably the combination of genes comprises genes listed in Tables 3, 5, 7, 9 and 11. More preferably the combination of genes comprises genes listed in Tables 4, 6, 8, 10 and 12.

More preferably the combination of genes comprises LY6G6D, KRT23, CEL, ACSL6, EREG, CFTR, TCN1, PCSK1, NCRNA00261, SPINK4, REG4, MUC2, TFF3, CLCA4, ZG16, CA1, MS4A12, CA4, CXCL13, RARRES3, GZMA, IDO1, CXCL9, SFRP2, COL10A1, CYP1B1, MGP, MSRB3, ZEB1, FLNA.

Also more preferably the combination of genes comprises SFRP2, ZEB1, RARRES3, CFTR, FLNA, MUC2, TFF3.

Applicants next sought to compare their method with the standard method of CRC classification, namely microsatellite instability (MSI). Applicants assessed subtype prevalence and distribution in samples from a dataset with known MSI status (GSE13294)⁹ and observed that 94% of the inflammatory subtype were MSI whereas 86% of the TA and 77% of the stem-like subtypes were microsatellite stable (MSS, Figure 1D). Consistent data were obtained by predicting MSI status for the samples embodied in the identification of our CRC subtypes from the core datasets, using published MSI gene signatures (Figure 7B and C). Although there is a strong association of MSI or MSS status with particular subtypes, the transcriptome signatures allow refinement beyond what can be achieved using MSI alone.

Numerous cell types with specialized functions make up the colon. While colonic stem cells are thought to be the cell of origin for CRC, more differentiated cells may have similar capacity. In light of these considerations, Applicants performed a series of analyses seeking to describe the cellular phenotypes of the observed CRC subtypes. First, Applicants used a published gene signature that discriminates between the normal colon crypt top (where terminally differentiated cells reside) and the normal crypt base (where the undifferentiated or stem cells reside). Using reside). Using the Nearest Template Prediction (NTP) algorithm, Applicants predicted that 98% of the stem-like subtype tumors were significantly associated with the crypt base signature (statistics includes only those samples that were predicted with FDR<0.2). On the other hand, more than 75% of samples from the enterocyte subtype tumors were significantly associated with crypt top by their concordant gene signatures. Intriguingly, 60% of the TA subtype tumor samples have a crypt top signature with low expression of Wnt signaling targets, *LGR5* and *ASCL2.* In contrast, the rest of the TA subtype tumors are significantly associated with the crypt base and exhibit high mRNA expression of the stem/progenitor markers *LGR5* and *ASCL2* (Figure 2A and Figure 8). This suggests that the TA subtype designation may embody two sub-subtypes. The inflammatory and goblet-like subtypes do not have significant associations with either the crypt base or top. Collectively, the most striking and relevant observation from this analysis is the clear association between the stem-like subtype and the crypt base signature.

To associate CRC subtypes to colon crypt top/base, Applicants used a previously published gene signature (Kosinski, C., et al., Proceedings of the National Academy of Sciences of the United States of America 104, 15418-15423 (2007) of the colon crypt base (see Figure 2A) together with nearest template prediction (NTP). The analysis confirmed that almost all of the samples from the NMF-identified stem-like subtype were associated with the crypt *base* signature. This is accomplished by splitting into two groups the up- and down-regulated signature genes to form a dichotomized gene expression template. The similarity of a sample's gene expression profile to the template is computed using a nearest neighbor approach. By random sub-sampling the gene space, NTP estimates a null distribution of similarity coefficients. Then the similarity coefficient obtained using the published gene signature can be compared to the null distribution so as to compute a p-value. The same approach was followed for the association of CRC subtypes to Wnt signaling (Figure 2A) and FOLFIRI response (Figure 3F) using specific signatures as described in the main text.

After performing NTP algorithm based prediction for association of colon-crypt top/base to each sample using a published gene signature that discriminates between the normal colon crypt top and the normal crypt base, Applicants observed statistically significant (only for samples with FDR<0.2) associations as reported in the main text. Here, Applicants are reporting the statistics for all the samples irrespective of the FDR cut-off. Applicants observed that 55% that 55% (n=77) of the stem-like subtype is associated with the crypt base whereas 33% (n=105) of TA, 43% (n=63) of goblet-like and 75% (n=64) of enterocyte subtypes are associated with the crypt top. On the other hand, Applicants observed that more than 80% (n=78) of the inflammatory subtypes have no significant association with either the crypt base or top.

The colon-crypt base is composed predominantly of stem and progenitor cells, which are known to exhibit high Wnt activity. Thus, Applicants examined Wnt signaling activity in the stem-like subtype by mapping a publicly available gene signature for active Wnt signaling onto the core CRC dataset. Similar to the colon-crypt top/base gene signature comparison, the majority of the stem-like subtype samples were predicted to have high Wnt activity, whereas enterocyte and goblet-like subtypes did not (Figure 2B). In order to validate this prediction, Applicants then performed an *in vitro* Wnt activity assay (TOP-flash) on stem-like subtype CRC cell lines and observed that 57% (n=7) of stem-like subtype cell lines exhibited high Wnt activity, as compared to 17% (n=6) among cell lines from the other subtypes (Figure 2C). To further validate this observation, Applicants performed quantitative (q)RT-PCR and immunofluorescence (IF) assays on a panel of CRC cell lines and xenograft tumors for markers of differentiation or Wnt signaling/stemness. This analysis confirmed that the stem-like subtype was the least differentiated and had the highest expression of Wnt signaling/stem cell markers. The goblet-like subtype, on the other hand, had a well-differentiated marker expression pattern with comparatively low expression of the Wnt markers (Figures 2D-G and Figure 6). These results provide further evidence that the stem-like subtype has a stem or progenitor cell phenotype, and the goblet-like and enterocyte subtype has a differentiated phenotype.

In order to validate the five subtypes in additional datasets, Applicants mapped the SAM and PAM genes-specific to each subtypes onto each of the preprocessed dataset (RMA in the case of Affymetrix arrays and directly from authors in case of other microarray platforms). Later, Applicants performed consensus-based NMF analysis to identify the number of classes. Further, heatmap was generated using NMF class and SAM and PAM genes.

Applicants performed DWD based merging of gene expression profile datasets for CRC cell lines from two different sources, for the purpose of increasing the total number of CRC cell lines, after first removing 14 repeated cell lines between the two datasets. Overall, Applicants obtained 51 unique CRC cell lines. The merged cell lines dataset was later merged again with the CRC core dataset, using the DWD based method. Next, Applicants performed NMF based consensus clustering of the merged CRC cell lines and core dataset, seeking to identify subtypes amongst the cell lines (Figure 6A-B). Applicants identified maximum cophentic coefficient at k=3 and 5. Applicants again selected k=5. Applicants determined that this collection of CRC cell lines represented only 4 subtypes: there was no single cell line that belonged to enterocyte subtype. A few of the duplicate cell lines from different sources showed different subtype identity (probably due to variation in cell culture between different laboratories) after NMF consensus clustering. Applicants tested the subtype of SW620 cell line using RT-PCR analysis and markers of differentiation and stem cells, since this cell line was used for various experiments. Applicants found that SW620 had higher expression of stem cell markers and lower expression of differentiated marker, confirming its stem-like subtype identity (Figure 6C).

Applicants examined the relationship between disease-free survival (DFS) and other histopathological information such as Dukes' stage, age, location of tumors (left or right of colon or rectum) and adjuvant treatment in the GSE14333 dataset; see Table 13.

**Table 13. Clinical/histopathological, subtype and statistical information for GSE14333 samples.**

| | **Enterocyte** | **Goblet-like** | **Inflammatory** | **Stem-like** | **TA** |
|---|---|---|---|---|---|
| **Age** | 66.25±10.17 | 64.52±12.33 | 60.02±12.74 | 61.66±12.27 | 67.13±15.28 |
| **Number of tumors** | 34 (17.26%) | 31 (15.74%) | 41 (20.8%) | 38 (19.29%) | 53 (26.9%) |
| | | | | | |
| **Tumor Duke's Stage** | | | | | |
| **A** | 3 (9.1%) | 10 (3.03%) | 3 (9.1%) | 4 (12.12%) | 13 (39.39%) |
| **B** | 12 (13.95%) | 14 (16.28%) | 20 (23.26%) | 18 (20.93%) | 22 (25.58%) |
| **C** | 19 (24.36%) | 7 (8.97%) | 18 (23.08%) | 16 (20.51%) | 18 (23.08%) |
| | | | | | |
| **Location of tumors** | | | | | |
| **Left colon** | 16 (19.28%) | 9 (10.84%) | 11 (13.25%) | 21 (25.3%) | 26 (31.33%) |
| **Right colon** | 10 (11.24%) | 20 (22.47%) | 30 (33.71%) | 9 (10.1%) | 20 (22.47%) |
| **Rectum** | 7 (30.43%) | 2 (8.7%) | 0 | 8 (34.78%) | 6 (26.09%) |
| **unknown colon** | 1 (0.5%) | 0 | 0 | 0 | 1 (0.5%) |
| | | | | | |
| **Adjuvant Radiation and/or chemotherapy** | | | | | |
| **Yes** | 14 (18.18%) | 13 (16.88%) | 16 (20.78%) | 14 (18.18%) | 20 (25.97%) |
| **No** | 20 (16.7%) | 18 (20.22%) | 25 (28.1%) | 24 (26.97%) | 23 (37.08%) |

Applicants censored those patients who were alive without tumor recurrence or dead at last contact. Since subtype is not significantly associated with DFS for all the data, Applicants first used a Cox model to do an adjusted analysis using the variables of Duke's stage or adjuvant treatment. As subtype was not significant in the adjusted analysis, Applicants examined the relationships between subtype and DFS on subsets based on these variables as shown in the main text.

In this dataset, the median follow up among patients without events (tumor recurrence) was 45.1 months. As already mentioned, Applicants first evaluated DFS for all the samples irrespective of treatment (adjuvant chemotherapy and/or radiotherapy - standard chemotherapy of either single agent 5-fluouracil;5-FU/capecitabine or 5-FU and oxaliplatin) or Dukes' stage (for analysis, Applicants considered Dukes' stage A and B patients with lymph node negativity together whereas Dukes' stage C patients with lymph node positivity separately), the latter known to correlate with CRC survival. Applicants did not find a significant association between subtype and DFS (p=0.12; Figure 7A and Table 13). As previously known, Applicants also observed in the current set of samples that treatment (p=0.03) and Dukes' stage (p=0.0009) were significantly associated with DFS. Similarly, Applicants also observed that treatment was significantly associated with Dukes' stage (p=0.0002, Fisher's exact test). Since treatment and Dukes' stage were associated with DFS, Applicants examined whether subtype was associated with DFS within subsets defined by these variables. In untreated patients, there was a significant association between subtypes and DFS (p=0.0003; n=120), with stem-like subtype tumors having the shortest DFS and inflammatory and enterocyte subtypes having the intermediate DFS (Figure 1C). On the other hand, there was no significant association between subtype and DFS (p=0.9; n=77) in treated patients (Figure 7B). Similarly, Applicants did not find significant association between subtype and DFS in Dukes' stages A and B (p=0.13; n=119) or in Dukes' stage C (p=0.7; n=98) patients. Applicants also observed that treatment preferentially improved DFS in stem-like subtype patients (though not statistically significant, Figure 7C).

The monoclonal anti-EGFR antibody cetuximab is a mainstay of treatment for metastasitc CRC with wild-type Kras; however, cetuximab has failed to show benefit in the adjuvant setting, irrespective of *KRAS* genotype. Applicants examined the possibility that tumors from our subtypes respond differently to cetuximab. To this end, Applicants correlated their subtypes with cetuximab response using a CRC liver metastases microarray (Khambata-Ford) dataset with matched therapy response from patients (n=80). In this particular dataset, Applicants predicted three of their five CRC subtypes using NMF consensus clustering and CRCassigner genes (Figure 3A and Figure 9A). The enterocyte and inflammatory subtypes were not present in this dataset, consistent with our results from another CRC dataset with metastatic information (Figure 9B) suggesting that they have lower metastatic potential. Applicants observed another unknown subtype in Khambata-Ford dataset that has a gene expression profile which is highly similar to normal liver and may represent tissue contamination and Applicants avoided this subtype in their further analyses (Figure 3A). Interestingly, Applicants found that 54% (n=26) of patients within the TA subtype had clinical benefit from cetuximab therapy (complete response, partial response and stable disease were considered as beneficial), while only 26% (n=42) of the patients within all the other subtypes had benefit from the drug (Figure 3A; p<0.05, Fisher Exact test). Although method of predicting cetuximab-response is independent of KRAS mutational status, its predictive value using TA subtype alone is roughly equivalent to that of using wildtype KRAS status (Figures 9C-F). Importantly, Applicants also observed TA subtype-specific sensitivity to cetuximab in the panel of CRC cell lines (Figure 3B and Figure 9G). While cell lines sensitive to cetuximab were only present within the TA subtype, there was not a uniform response among all the TA cell lines. As such, the cetuximab sensitive and resistant TA subtype tumors and cell lines were henceforth subdivided into two sub-subtypes: cetuximab-sensitive (CS)-TA and cetuximab-resistant (CR)-TA. This further sub-classification brought the total number of CRC subtypes to six.

In the course of further characterizing the two TA subtypes, Applicants observed that CS-TA tumors have significantly higher expression of epiregulin *(EREG)* and amphiregulin (*AREG*), which are epidermal growth factor receptor (*EGFR*) ligands known to be positive predictors of cetuximab response, compared to CR-TA tumors, using SAM analysis (TA signature; FDR=0.1 and delta=0.8, Figure 3C and Figures 9H-I. Among the three most negative predictors of response to cetuximab (high expression in the CR-TA subtype) was filamin A (FLNA), which regulates the expression and signaling of the cMET receptor (Figure 3C). Interestingly, high *FLNA* expression is significantly associated with poor prognosis only within the TA subtype tumors (Figure 3D), and *FLNA* expression did not show prognostic differences when samples from all the subtypes were included or when compared by *KRAS* status (Figures 9K-M). Furthermore, CR-TA cell lines were much more sensitive to cMet inhibition than CS-TA cell lines (Figure 3E). This suggests that screening for TA subtype followed by *EREG* and *FLNA* expression would predict response to cetuximab and cMet inhibitor, respectively.

Figures 9D-E illustrate comparable differential responses to cetuximab treatment when restricting the analysis to the TA subtype (p=1.4x10⁻⁶; n=26; Figure 9D) versus KRAS WT patients (p=1.9x10⁻⁶; n=39; Figure 9E) using Khambata-Ford dataset. By comparing Figures 9F-G, one can gauge the contribution of the TA subtype to the overall differential response to cetuximab: when excluding the TA subtypes, one finds a markedly reduced significance of differential response (p=1.9x10⁻⁴; n=22; Figure 9F) when compared to the same analysis using all 3 of the identified subtypes (specific to this dataset, p=1.6x10⁻¹⁰; n=48; Figure G) suggesting that patients falling into the TA subtype are largely responsible for the population-wide cetuximab response. For all four of these Kaplan-Meier plots, Applicants excluded samples falling into the "unknown" subtype, which Applicants suspect to have been contaminated by liver metastases, based on expression response signatures (Figure 3A). Survival statistics for responders (R), evaluated based on modified WHO criteria, were differentiated from non-responders (NR) using a log-rank test.

**Table 14. List of t test gene signatures that are differentially expressed between CS-TA and CR-TA Khambata-Ford samples.**

| Genes | Response predictor |
|---|---|
| MMP12 | Non Responsive |
| BCL2A1 | Non Responsive |
| ALOX5AP | Non Responsive |
| TREM1 | Non Responsive |
| CYP1B1 | Non Responsive |
| BHLHE41 | Non Responsive |
| EPHA4 | Non Responsive |
| AHNAK2 | Non Responsive |
| DUSP4 | Non Responsive |
| TMPRSS3 | Non Responsive |
| FLNA | Non Responsive |
| PLEKHB1 | Non Responsive |
| TGFB1I1 | Non Responsive |
| DACT1 | Non Responsive |
| CCL2 | Non Responsive |
| AKAP12 | Non Responsive |
| ANO1 | Non Responsive |
| ZFP36L2 | Non Responsive |
| GLS | Non Responsive |
| CCL24 | Non Responsive |
| ASB9 | Non Responsive |
| GALNT7 | Non Responsive |
| HSPA2 | Non Responsive |
| ANKRD10 | Non Responsive |
| CD55 | Non Responsive |
| GCNT3 | Non Responsive |
| SERPINB5 | Non Responsive |
| LAMP2 | Non Responsive |
| CA9 | Non Responsive |
| HLA-DPA1 | Responsive |
| PLA1A | Responsive |
| CTSL2 | Responsive |
| FGFR3 | Responsive |
| GZMB | Responsive |
| PRSS23 | Responsive |
| SGK2 | Responsive |
| FABP4 | Responsive |
| AQP3 | Responsive |
| LRRC31 | Responsive |
| GGH | Responsive |
| AREG | Responsive |
| EREG | Responsive |
| FMO5 | Responsive |
| SPAG1 | Responsive |
| HPGD | Responsive |
| SI | Responsive |
| CLDN8 | Responsive |
| ZG16 | Responsive |
| FAM55D | Responsive |
| TNS1 | Responsive |
| SEMA6D | Responsive |
| DMBT1 | Responsive |
| TRPM6 | Responsive |

In another embodiment, the present invention provides an *in-vitro* method for predicting the likelihood that a subject suffering from colorectal cancer or suspected of suffering therefrom and who has undergone a prior surgical resection of colorectal cancer will respond to therapies inhibiting or targeting EGFR, such as cetuximab, and/or cMET, the method comprising
(i) providing a biological sample from said subject comprising colorectal cancer cells or suspected to comprise colorectal cancer cells;
(ii) measuring the expression level of one or a combination of genes selected from the group of genes listed in Table 2, and
(iii) classifying said biological sample as "Stem-like", "Inflammatory", "Transit-amplifying (TA)", "Goblet-like" and "Enterocyte" on the basis of the gene expression profile according to Table 2,
wherein
- high expressions of AREG and EREG genes and low expressions of BHLHE41, FLNA and PLEKHB1 genes in "Transit-amplifying (TA)" type indicates that at metastatic setting said subject will be responsive to cetuximab treatment and resistant to cMET inhibitor therapy and this signature defines a subtype of TA type designed as "Cetuximab-sensitive transit-amplifying subtype (CS-TA)".
- low expressions of AREG and EREG genes and high expressions of BHLHE41, FLNA and PLEKHB1 genes in "Transit-amplifying (TA)" type indicates that at metastatic setting said subject will be resistant to cetuximab treatment and will be responsive to cMET inhibitor therapy, and this signature defines a second subtype of TA type named as "Cetuximab-resistant transit-amplifying subtype (CR-TA)".

This analysis of cetuximab/cMET response based subtypes forms six integrated gene expression and drug response based subtypes.

A further aspect comprises the combination of genes comprising at least at least five genes selected from Table 2, or at least 10 genes selected from Table 2, or at least 20 genes that are selected from Table 2, more preferred at least 30 genes that are selected from Table 2, more preferred at least 40 genes that are selected from Table 2, more preferred at least 50 genes that are selected from Table 2, more preferred at least 60 genes that are selected from Table 2, more preferred at least 70 genes that are selected from Table 2, more preferred at least 80 genes that are selected from Table 2, more preferred at least 90 genes that are selected from Table 2, more preferred at least 100 genes that are selected from Table 2, more preferred at least 120 genes that are selected from Table 2, more preferred at least 140 genes that are selected from Table 2, more preferred at least 160 genes that are selected from Table 2, more preferred at least 180 genes that are selected from Table 2, more preferred at least 200 genes that are selected from Table 2, more preferred at least 220 genes that are selected from Table 2, more preferred at least 240 genes that are selected from Table 2, more preferred at least 260 genes that are selected from Table 2, more preferred at least 280 genes that are selected from Table 2, more preferred at least 300 genes that are selected from Table 2, more preferred at least 320 genes that are selected from Table 2, more preferred at least 340 genes that are selected from Table 2, more preferred at least 360 genes that are selected from Table 2, more preferred at least 380 genes that are selected from Table 2, more preferred at least 400 genes that are selected from Table 2, more preferred at least 420 genes that are selected from Table 2, more preferred at least 460 genes that are selected from Table 2, more preferred at least 480 genes that are selected from Table 2, more preferred at least 500 genes that are selected from Table 2, more preferred at least 520 genes that are selected from Table 2, more preferred at least 540 genes that are selected from Table 2, more preferred at least 560 genes that are selected from Table 2, more preferred at least 580 genes that are selected from Table 2, more preferred at least 600 genes that are selected from Table 2, more preferred at least 620 genes that are selected from Table 2, more preferred at least 640 genes that are selected from Table 2, more preferred at least 660 genes that are selected from Table 2, more preferred at least 680 genes that are selected from Table 2, more preferred at least 700 genes that are selected from Table 2, more preferred at least 720 genes that are selected from Table 2, more preferred at least 740 genes that are selected from Table 2, more preferred at least 760 genes that are selected from Table 2.

In a further preferred embodiment, a method of the invention comprises the combination of genes selected from all 786 genes of Table 2.

More preferably the combination of genes comprises at least five, or at least 10, or at least 20, or at least 30, or at least 40 genes selected from Table 2.

Preferably the combination of genes comprises AREG, EREG, BHLHE41, FLNA, PLEKHB1 and genes listed in Tables 3, 5, 7, 9 and 11. More preferably the combination of genes comprises AREG, EREG, BHLHE41, FLNA, PLEKHB1 genes listed in Tables 4, 6, 8, 10 and 12.

Next, Applicants examined the possibility that the subtypes may exhibit differential response to first line colorectal chemotherapy (*i.e*. FOLFIRI) using a published FOLFIRI response signature. FOLFIRI is a current chemotherapy regimen for treatment of colorectal cancer. It comprises the following drugs:
- FOL - folinic acid (leucovorin),a vitamin B derivative used as a "rescue" drug for high doses of the drug methotrexate and that modulates/potentiates/reduces the side effects of fluorouracil;
- F - fluorouracil (5-FU), a pyrimidine analog and antimetabolite which incorporates into the DNA molecule and stops synthesis; and
- IRI - irinotecan (Camptosar), a topoisomerase inhibitor, which prevents DNA from uncoiling and duplicating.
Cetuximab can sometimes added to FOLFIRI.
The regimen consists of:
- Irinotecan (180 mg/m² IV over 90 minutes) concurrently with folinic acid (400 mg/m² [or 2 x 250 mg/m²] IV over 120 minutes).
- Followed by fluorouracil (400-500 mg/m² IV bolus) then fluorouracil (2400-3000 mg/m² intravenous infusion over 46 hours).
This cycle is typically repeated every two weeks. The dosages shown above may vary from cycle to cycle.

Intriguingly, 100% of the stem-like and 77% of the inflammatory subtype samples were predicted to respond to FOLFIRI, as compared to less than 14% of the TA subtype tumors (statistics include only samples with FDR<0.2, Figure 3F and Figures 10A-B). Similarly, cell lines from the stem-like subtype were predicted to respond to FOLFIRI (Figure 10). The finding that the stem-like subtype has a comparatively poorer prognosis and is more responsive to chemotherapy is consistent with data from other cancer subtypes with poor prognosis, such as basal and claudin-low breast cancer and quasi-mesenchymal pancreatic adenocarcinoma.

In a further embodiment, the present invention provides an *in-vitro* method for predicting the likelihood that a subject suffering from colorectal cancer or suspected of suffering therefrom and who has undergone a prior surgical resection of colorectal cancer will respond to cytotoxic chemotherapies such as FOLFIRI, the method comprising
(i) providing a biological sample from said subject comprising colorectal cancer cells or suspected to comprise colorectal cancer cells;
(ii) measuring the expression level of one or a combination of genes selected from the group of genes listed in Table 2, and
(iii) classifying said biological sample as "Stem-like", "Inflammatory", "Transit-amplifying (TA)", "Goblet-like" and "Enterocyte" on the basis of the gene expression profile according to Table 2,
wherein
- "Stem-like" type of colorectal cancer predicts good response in both adjuvant and metastatic settings,
- "Inflammatory" type of colorectal cancer predicts good response in adjuvant setting,
- "TA (transit-amplifying)" type of colorectal cancer predicts poor response in both adjuvant and metastatic settings,
- "Goblet-like" type of colorectal cancer predicts poor response in adjuvant setting, and
- "Enterocyte" type of colorectal cancer predicts good response in adjuvant setting.

Preferably the combination of genes comprises genes listed in Tables 3, 5, 7, 9 and 11. More preferably the combination of genes comprises genes listed in Tables 4, 6, 8, 10 and 12.

A further aspect comprises the combination of genes comprising at least two genes selected from Table 2, or at least five genes selected from Table 2, or at least 10 genes selected from Table 2, or at least 20 genes that are selected from Table 2, more preferred at least 30 genes that are selected from Table 2, more preferred at least 40 genes that are selected from Table 2, more preferred at least 50 genes that are selected from Table 2, more preferred at least 60 genes that are selected from Table 2, more preferred at least 70 genes that are selected from Table 2, more preferred at least 80 genes that are selected from Table 2, more preferred at least 90 genes that are selected from Table 2, more preferred at least 100 genes that are selected from Table 2, more preferred at least 120 genes that are selected from Table 2, more preferred at least 140 genes that are selected from Table 2, more preferred at least 160 genes that are selected from Table 2, more preferred at least 180 genes that are selected from Table 2, more preferred at least 200 genes that are selected from Table 2, more preferred at least 220 genes that are selected from Table 2, more preferred at least 240 genes that are selected from Table 2, more preferred at least 260 genes that are selected from Table 2, more preferred at least 280 genes that are selected from Table 2, more preferred at least 300 genes that are selected from Table 2, more preferred at least 320 genes that are selected from Table 2, more preferred at least 340 genes that are selected from Table 2, more preferred at least 360 genes that are selected from Table 2, more preferred at least 380 genes that are selected from Table 2, more preferred at least 400 genes that are selected from Table 2, more 2, more preferred at least 420 genes that are selected from Table 2, more preferred at least 460 genes that are selected from Table 2, more preferred at least 480 genes that are selected from Table 2, more preferred at least 500 genes that are selected from Table 2, more preferred at least 520 genes that are selected from Table 2, more preferred at least 540 genes that are selected from Table 2, more preferred at least 560 genes that are selected from Table 2, more preferred at least 580 genes that are selected from Table 2, more preferred at least 600 genes that are selected from Table 2, more preferred at least 620 genes that are selected from Table 2, more preferred at least 640 genes that are selected from Table 2, more preferred at least 660 genes that are selected from Table 2, more preferred at least 680 genes that are selected from Table 2, more preferred at least 700 genes that are selected from Table 2, more preferred at least 720 genes that are selected from Table 2, more preferred at least 740 genes that are selected from Table 2, more preferred at least 760 genes that are selected from Table 2.

In a further preferred embodiment, a method of the invention comprises the combination of genes selected from all 786 genes of Table 2.

More preferably the combination of genes comprises at least two, or at least five, or at least 10, or at least 20, or at least 30, or at least 40 genes selected from Table 2.

More preferably the combination of genes comprises LY6G6D, KRT23, CEL, ACSL6, EREG, CFTR, TCN1, PCSK1, NCRNA00261, SPINK4, REG4, MUC2, TFF3, CLCA4, ZG16, CA1, MS4A12, CA4, CXCL13, RARRES3, GZMA, IDO1, CXCL9, SFRP2, COL10A1, CYP1B1, MGP, MSRB3, ZEB1, FLNA.

Also more preferably the combination of genes comprises SFRP2, ZEB1, RARRES3, CFTR, FLNA, MUC2, TFF3.

Methods according to the invention preferably further comprise determining a strategy for treatment of the patient. Treatment may include, for example, radiation therapy, chemotherapy, targeted therapy, or some combination thereof. Treatment decisions for individual colorectal cancer patients are currently based on stage, patient age and condition, the location and grade of the cancer, the number of patient lymph nodes involved, and the absence or presence of distant metastases.

Classifying colorectal cancers into subtypes at the time of diagnosis using the methods disclosed in the present invention provides an additional or alternative treatment decision-making factor, thereby providing additional information for adapting the treatment of a subject suffering from colorectal cancer (see Figure 15). The methods of the invention permit the differentiation of six types of colorectal cancers, termed as "Stem-like" type, "Inflammatory" type, "Transit-amplifying cetuximab-sensitive (CS-TA)" type, "Transit-amplifying cetuximab-resistant (CR-TA)" type, "Goblet-like" type and "Enterocyte" type.

"Stem-like" type of colorectal cancer indicates good response to FOLFIRI treatment and poor response to cetuximab treatment, which means that patients suffering from or suspected to suffer from "Stem-like" type of colorectal cancer should be rather treated with adjuvant chemotherapy, preferably FOLFIRI treatment, to classic colorectal cancer surgical resection. Chemotherapy, preferably adjuvant FOLFIRI, would be also beneficial in case of metastatic treatment.

"Inflammatory" type of colorectal cancer indicates good response to chemotherapy, preferably FOLFIRI treatment, which means that patients suffering from or suspected to suffer from "Inflammatory" type of colorectal cancer should be rather treated with adjuvant chemotherapy, preferably adjuvant FOLFIRI treatment.

"Transit-amplifying cetuximab-sensitive (CS-TA)" type of colorectal cancer indicates poor response to FOLFIRI treatment and good response to cetuximab treatment, which means that patients suffering from or suspected to suffer from "Transit-amplifying cetuximab-sensitive (CS-TA)" type of colorectal cancer should be rather treated with cetuximab treatment at metastatic setting. Thus at adjuvant setting (adjuvant therapy to surgical resection of colorectal cancer), this CS-TA type indicates that patients will not require any treatment in addition to surgical resection of colorectal cancer, but a watchful-surveillance until the patient recur with the disease to be treated with cetuximab.

"Transit-amplifying cetuximab-resistant (CR-TA)" type of colorectal cancer indicates poor response to FOLFIRI treatment and almost no response to cetuximab treatment but shows good response to cMET inhibition, which menas that patients suffering form or suspected to suffer from "Transit-amplifying cetuximab-resistant (CR-TA)" type of colorectal cancer should be rather treated with cMET inhibitor at metastatic setting. Thus at adjuvant setting (adjuvant therapy to surgical resection of colorectal cancer), this CR-TA subtype indicates that patients will not require any treatment, but a watchful-surveillance until the patient recur with the disease to be treated with cMet inhibitors.

"Goblet-like" type of colorectal cancer indicates intermediate response to adjuvant FOLFIRI treatment and poor response to cetuximab treatment.

"Enterocyte" type of colorectal cancer indicates poor response to adjuvant FOLFIRI treatment.

Moreover, "Stem-like" type of colorectal cancer and "Inflammatory" type of colorectal cancer that have a poor or intermediate prognosis, as determined by gene expression profiling of the present invention, may benefit from adjuvant therapy (e.g., radiation therapy or chemotherapy). Chemotherapy for these patients may include FOLFIRI treatment, fluorouracil (5-FU), 5-FU plus leucovorin (folinic acid); 5-FU, leucovorin plus oxaliplatin; 5-FU, leucovorin plus irinotecan; capecitabine, and/or drugs for targeted therapy, such as an anti-VEGF antibody, for example Bevacizumab, and an anti-Epidermal growth factor receptor antibody, for example Cetuximab and/or combinations of said treatments. Radiation therapy may include external and/or internal radiation therapy. Radiation therapy may be combined with chemotherapy as adjuvant therapy.

In another embodiment of the present invention, the patients suffering from or suspected to suffer from "Transit-amplifying" type of colorectal cancer, may take advantage of the following treatment depending on expressions of EREG gene and FLNA gene:
1) EREG gene is highly expressed and FLNA is low expressed, then cetuximab alone treatment should be used.
2) EREG gene is low expressed and FLNA is highly expressed, then cMET inhibitor alone treatment should be used.
3) both EREG and FLNA are highly expressed, then a combination of cetuximab and cMET inhibitor treatment should be used.
4) both EREG and FLNA are low expressed, then cetuximab and/or cMET inhibitor treatment do not seem to be effective.

A biological sample comprising a cancer cell of a colorectal cancer or suspected to comprise a cancer cell of a colorectal cancer is provided after the removal of all or part of a colorectal cancer sample from the subject during surgery or colonoscopy. For example, a sample may be obtained from a tissue sample or a biopsy sample comprising colorectal cancer cells that was previously removed by surgery. Preferably a biological sample is obtained from a tissue biopsy.

A sample of a subject suffering from colorectal cancer or suspected of suffering there from can be obtained in numerous ways, as is known to a person skilled in the art. For example, the sample can be freshly prepared from cells or a tissue sample at the moment of harvesting, or they can be prepared from samples that are stored at -70°C until processed for sample preparation. Alternatively, tissues or biopsies can be stored under conditions that preserve the quality of the protein or RNA. Examples of these preservative conditions are fixation using e.g. formaline and paraffin embedding, RNase inhibitors such as RNAsin (Pharmingen) or RNasecure (Ambion), aquous solutions such as RNAlater (Assuragen; US06204375), Hepes-Glutamic acid buffer mediated Organic solvent Protection Effect (HOPE; DE 10021390), and RCL2 (Alphelys; WO04083369), and non-aquous solutions such as Universal Molecular Fixative (Sakura Finetek USA Inc.; US7138226). Alternatively, a sample from a colorectal cancer patient may be fixated in formalin, for example as formalin-fixed paraffin-embedded (FFPE) tissue.

Preferably measuring the expression level of genes in methods of the present invention is obtained by a method selected from the group consisting of:
(a) detecting RNA levels of said genes, and/or
(b) detecting a protein encoded by said genes, and/or
(c) detecting a biological activity of a protein encoded by said genes.

The detecting RNA levels is obtained by any technique known in the art, such as Microarray hybridization, quantitative real-time polymerase chain reaction, multiplex-PCR, Northern blot, In Situ Hybridization, sequencing-based methods, quantitative reverse transcription polymerase-chain reaction, RNAse protection assay or an immunoassay method.

The detecting of protein levels of aforementioned genes is obtained by any technique known in the art, such as Western blot, immunoprecipitation, immunohistochemistry, ELISA, Radio Immuno Assay, proteomics methods, or quantitative immunostaining methods.

According to another embodiment, expression of a gene of interest is considered elevated when compared to a healthy control if the relative mRNA level of the gene of interest is greater than 2 fold of the level of a control gene mRNA. According to another embodiment, the relative mRNA level of the gene of interest is greater than 3 fold, 5 fold, 10 fold, 15 fold, 20 fold, 25 fold, or 30 fold compared to a healthy control gene expression level.

For example the microarray method comprises the use of a microarray chip having one or more nucleic acid molecules that can hybridize under stringent conditions to a nucleic acid molecule encoding a gene mentioned above or having one or more polypeptides (such as peptides or antibodies) that can bind to one or more of the proteins encoded by the genes mentioned above.

For example the immunoassay method comprises binding an antibody to protein expressed from a gene mentioned above in a patient sample and determining if the protein level from the patient sample is elevated. The immunoassay method can be an enzyme-linked immunosorbent assay (ELISA), electro-chemiluminescence assay (ECLA), or multiplex microsphere-based assay platform, e.g., Luminex® platform. Described herein is a kit for classifying a sample of a subject suffering from colorectal cancer or suspected of suffering there from, the kit comprising a set of primers, probes or antibodies specific for genes selected from the group of genes listed in Table 2.

The kit can further comprise separate containers, dividers, compartments for the reagents or informational material. The informational material of the kits is not limited in its form. In many cases, the informational material, e.g., instructions, is provided in printed matter, e.g., a printed text, drawing, and/or photograph, e.g., a label or printed sheet. However, the informational material can also be provided in other formats, such as Braille, computer readable material, video recording, or audio recording. Of course, the informational material can also be provided in any combination of formats. Also described herein is the provision of immunohistochemistry and quantitative real-time PCR based assays for identifying CRC subtypes. Immunohistochemistry markers were developed for at least following four CRC subtypes (see Figure 11):
A) TA subtype where CFTR has 3+ staining intensity and other markers have 1+ staining intensity.
B) Goblet-like subtype where MUC2 and TFF3 (2 markers) have 3+ staining intensity and other markers have 1+ staining intensity.
C) Enterocyte subtype where MUC2 has 3+ staining intensity and other markers have 1+ staining intensity.
D) Stem-like subtype where Zeb1 has 3+ staining intensity and other markers have 1+ staining intensity.

Table 15 (A) and (B) shows the quantitative RT-PCR results (qRT-PCR) for subtype-specific markers in CRC patient tumors. The values represent copy number/ng of cDNA for each gene. The positive values in the column represent those values above average value for that marker whereas negative values represent below average value. Using the average cut- off, Applicants could identify 11/19 samples that represent all the 6 subtypes including CR-TA and CS-TA. (B)

**Table 15 (A)**

| **Samples** | **MUC2** | **TFF3** | **SFRP2** | **RARRES3** | **CFTR** | **FLNA** | **Subtypes** |
|---|---|---|---|---|---|---|---|
| CR559251 | 0.17861 | 24.5687 | 31.482 | 12.47621 | 1.468 | 25.55 | Stem-like |
| CR559521 | 133.207 | 2181.53 | 4.8301 | 4.710633 | 25.716 | 15.11 | Goblet-like |
| CR560026 | 26.179 | 1830.28 | 0 | 5.813822 | 27.688 | 17.88 | Unpredictable |
| CR560030 | 1.22231 | 1272.48 | 30.474 | 14.49112 | 47.279 | 6.631 | Unpredictable |
| CR560080 | 0.06094 | 412.549 | 40.077 | 19.7314 | 22.443 | 15.89 | Stem-like |
| CR560126 | 3.78387 | 1567.72 | 11.231 | 81.04012 | 14.428 | 8.245 | Unpredictable |
| CR560191 | 2.33406 | 490.949 | 13.978 | 32.20789 | 8.9398 | 5.144 | Unpredictable |
| CR560367 | 62.6451 | 400.288 | 12.123 | 406.0998 | 8.1013 | 27.25 | Inflammatory |
| CR560403 | 0.24779 | 85.9297 | 2.1521 | 24.71503 | 8.1945 | 3.665 | Unpredictable |
| CR560476 | 10.5152 | 324.581 | 40.265 | 6.529803 | 3.9446 | 9.282 | Stem-like |
| CR560523 | 133.426 | 696.831 | 32.503 | 15.24705 | 23.075 | 86.19 | Unpredictable |
| CR560527 | 1.85148 | 2083.62 | 37.311 | 7.212504 | 51.276 | 89.99 | Unpredictable |
| CR560590 | 698.171 | 9815.49 | 31.575 | 23.04962 | 29.946 | 13.51 | Unpredictable |
| CR560603 | 98.3348 | 570.059 | 7.3503 | 16.20295 | 10.585 | 12.51 | Enterocyte |
| CR560671 | 30.8062 | 892.399 | 10.128 | 14.60695 | 107.31 | 27.44 | CR-TA |
| CR560973 | 2.9832 | 304.316 | 0.373 | 37.2808 | 68.207 | 6.068 | CS-TA |
| CR560974 | 0.52935 | 1417.92 | 0 | 14.07925 | 207.07 | 80.22 | CR-TA |
| CR561060 | 209.86 | 1950.79 | 8.6177 | 25.15537 | 0 | 21.77 | Goblet-like |
| CR561163 | 342.859 | 2774.7 | 6.8036 | 65.19357 | 43.742 | 47.16 | Unpredictable |

**Table 15 (B)**

| **Samples** | **MUC2** | **TFF3** | **SFRP2** | **RARRES3** | **CFTR** | **FLNA** | **Subtypes** |
|---|---|---|---|---|---|---|---|
| CR559251 | Negative | Negative | Positive | Negative | Negative | Negative | Stem-like |
| CR559521 | Positive | Positive | Negative | Negative | Negative | Negative | Goblet-like |
| CR560026 | Negative | Positive | Negative | Negative | Negative | Negative | Unpredictable |
| CR560030 | Negative | Negative | Positive | Negative | Positive | Negative | Unpredictable |
| CR560080 | Negative | Negative | Positive | Negative | Negative | Negative | Stem-like |
| CR560126 | Negative | Positive | Negative | Positive | Negative | Negative | Unpredictable |
| CR560191 | Negative | Negative | Negative | Negative | Negative | Negative | Unpredictable |
| CR560367 | Negative | Negative | Negative | Positive | Negative | Negative | Inflammatory |
| CR560403 | Negative | Negative | Negative | Negative | Negative | Negative | Unpredictable |
| CR560476 | Negative | Negative | Positive | Negative | Negative | Negative | Stem-like |
| CR560523 | Positive | Negative | Positive | Negative | Negative | Positive | Unpredictable |
| CR560527 | Negative | Positive | Positive | Negative | Positive | Positive | Unpredictable |
| CR560590 | Positive | Positive | Positive | Negative | Negative | Negative | Unpredictable |
| CR560603 | Positive | Negative | Negative | Negative | Negative | Negative | Enterocyte |
| CR560671 | Negative | Negative | Negative | Negative | Positive | Positive | CR-TA |
| CR560973 | Negative | Negative | Negative | Negative | Positive | Negative | CS-TA |
| CR560974 | Negative | Negative | Negative | Negative | Positive | Positive | CR-TA |
| CR561060 | Positive | Positive | Negative | Negative | Negative | Negative | Goblet-like |
| CR561163 | Positive | Positive | Negative | Positive | Positive | Positive | Unpredictable |

Summary of subtype-specific candidate biomarkers (CRCassignor-7) that were tested using qRT-PCR and immunohistochemistry (IHC) are shown in Table 16:

**Table 16**

| CRC subtype | Signature genes | Biomarkers for qRT-PCR assay | Biomarkers for IHC |
|---|---|---|---|
| Stem-like | SFRP2, ZEB1 | SFRP2+ | ZEB1+ |
| Inflammatory | RARRES3 | RARRES3+ | [RARRES3 TBD] |
| CR-TA | CFTR, FLNA | CFTR+, FLNA+ | CFTR+ [FLNA TBD] |
| CS-TA | CFTR, (FLNA) | CFTR, (FLNA-) | CFTR+ [FLNA TBD] |
| Goblet-like | MUC2, TFF3 | MUC2+, TFF3+ | MUC2+, TFF3+ |
| Eneterocyte | MUC2, (TFF3) | MUC2+, (TFF3-) | MUC2+, (TFF3-) |

Applicants herein document the existence of six subtypes of CRC based on the combined analysis of gene expression and response to cetuximab. Notably, these subtypes are predictive of disease-free prognosis and response to selected therapies (Figure 4A). This indicates that the selection of therapeutic agents for patients with CRC could be more effective if CRC subtypes and their differential responses to targeted and conventional therapies were taken into account. Namely three subtypes have markedly better disease-free survival after surgical resection, suggesting these patients might be spared from the adverse effects of chemotherapy when they have localized disease. Applicants also associated these CRC subtypes with an anatomical location within colon crypts (phenotype) and with the crypt location-dependent differentiation state (Figure 4B), a finding that may aid in our understanding or identification of the cell of origin in CRC tumors. In addition, Applicants validated the subtype and cellular phenotype phenotype specific gene signatures using RT-PCR, which may serve as prognostic and/or predictive markers in clinic for CRC. Lastly, Applicants demonstrate that subtype-specific CRC cell lines and xenograft tumors can serve as surrogates for clinical features of CRC. Recognition of these subtypes may allow for the assessment of candidate drugs and combinations in preclinical assays that could in turn guide "personalized" therapeutic trial designs that target such CRC subtype sensitivities only in those patients likely to see clinical benefit, much as is becoming standard of care in non-small cell lung cancer.

Those skilled in the art will appreciate that the invention described herein is susceptible to variations and modifications other than those specifically described. It is to be understood that the invention includes all such variations and modifications without departing from the spirit or essential characteristics thereof. The invention also includes all of the steps, features, compositions and compounds referred to or indicated in this specification, individually or collectively, and any and all combinations or any two or more of said steps or features. The present disclosure is therefore to be considered as in all aspects illustrated and not restrictive, the scope of the invention being indicated by the appended Claims.

The foregoing description will be more fully understood with reference to the following Examples. Such Examples are, however, exemplary of methods of practising the present invention and are not intended to limit the scope of the invention.

### EXAMPLES

### Methodology

**Processing of Microarrays.** The processing of microarrays from CEL files was performed as already described. Published microarray data were obtained from GEO Omnibus and the raw CEL files from Affymetrix GeneChip® arrays for all samples were processed, robust multiarray averaged (RMA), and normalized using R-based Bioconductor. The patient characteristics for the published microarray data were obtained from GEO Omnibus using Bioconductor package, GEOquery.

**Combining Different Microarray Datasets.** Microarray datasets from different published studies were screened separately for variable genes using standard deviation (SD) cut off greater than 0.8. The screened datasets were column (sample) normalized to N(0,1) and row (gene) normalized and then merged using Java-based DWD. Finally, the rows were median centered before further downstream analysis, as already described.

**NMF, SAM and PAM analysis.** The stable subtypes were identified using consensus clustering-based NMF followed by SAM (using classes defined by NMF analysis) and PAM (using significant genes defined by SAM) analysis to identify gene signature specific to each of the subtypes.

**Survival Statistics.** Kaplan-Meier Survival curves were plotted and log-rank test were performed using GenePattern based Survival Curve and Survival Difference programs. Multivariate Cox Regression analysis was performed using R based library, survival.

**Cell Lines.** Colon cancer cell lines were grown in DMEM (Gibco, USA) plus 10% FBS (Invitrogen, USA) without antibiotics/antimycotics. All the cell lines were confirmed to be negative for mycoplamsa by PCR (VenorGeM kit, Sigma, USA) prior to use and were tested monthly.

**Drug response in Cell Lines.** Cells were added (5 x 10³) into 96-well plates on day 0 and treated with cetuximab (Merck Serono, Geneva, Switzerland), cMet inhibitor (PFA 665752, Santa Cruz Biotechnology, Inc., Santa Cruz, CA) or vehicle control (media alone or DMSO) on day 1. Proliferation was monitored using CellTiter-Glo® assay kit according to the manufacturer's instruction (Promega, Dubendorf, Switzerland) on day 3 (72 h).

**RNA isolation and RT-PCR.** RNA was isolated using miReasy kit (Qiagen, Hombrechtikon, Switzerland) as per the manufacturer's instructions. The sample preparation for Real-time RT-PCR was performed using QIAgility (automated PCR setup, Qiagen) and PCR assay was performed using QuantiTect SYBR Green PCR kit (Qiagen), gene specific primers (see Table 17) and Rotor-Gene Q (Qiagen) real-time PCR machine.

**Table 17. List of primers for qRT-PCR; Annealing temperature for all the samples are 60 C.**

| **Gene Name** | **Primer sequence Forward** | **Primer sequence Reverse** |
|---|---|---|
| KRT20 | ACG CCA GAA CAA CGA ATA CC (SEQ ID NO : 787) | ACG ACC TTG CCA TCC ACT AC (SEQ ID NO : 788) |
| MUC2 | CAA GAT CTT CAT GGG GAG GA (SEQ ID NO : 789) | AAC ACG GTG GTC CTC TTG TC (SEQ ID NO : 790) |
| CCND1 | AAC TAC CTG GAC CGC TTC CT (SEQ ID NO : 791) | CCA CTT GAG CTT GTT CAC CA (SEQ ID NO : 792) |
| MYC | TTC GGG TAG TGG AAA ACC AG (SEQ ID NO : 793) | CAG CAG CTC GAA TTT CTT CC (SEQ ID NO : 794) |
| CD44 | AGC AAC CAA GAG GCA AGA AA (SEQ ID NO : 795) | GTG TGG TTG AAA TGG TGC TG (SEQ ID NO : 796) |
| FLNA | CAT TCA GAT TGG GGA GGA GA (SEQ ID NO : 797) | ACA TCC ACC TCT GAG CCA TC (SEQ ID NO : 798) |

**TOP Flash Assay.** The TOP/FOP-flash assay was performed as instructed by the manufacturer (Upstate, USA). Briefly, colon cancer cell lines were plated into 24-well dishes in biological triplicate at 10K cells/well in full growth media (RPMI + 10% FBS). The next day, the media was changed to that containing 3 uL of PEI (stock, 1 mg/mL), TOP or FOP-flash DNA (0.25 ug/well) and a plasmid encoding constitutive expression of Renilla luciferase (to normalize for transfection efficiency). Two days later, the cells were assayed. Samples were prepared in biological triplicate (s.d. n=3) and the experiment was repeated twice.

**Immunofluorescence.** Colon cancer cell lines were plated, and allowed to set overnight, onto gelatin-coated (0.1% solution in PBS) cover slides in 24-well dishes. The following day, the cells were fixed with 4% paraformaldehyde in PBS (20 minutes, room temperature) and washed twice. Immunofluorescent analysis was performed as described³⁶. Antibody dilutions are as follows: MUC2 (1:100, SC7314; Santa Cruz, USA) and KRT20 (1:50, M7019; DAKO, USA).

**Orthotopic implantation of CRC cell lines into mice and RNA isolation.** NMRI nu/nu mice (6-8 week old females) were anesthetized with Ketamine and Xylazin, additionally receving buprenorphin (0,05-2,5 mg/kg) before surgery. The animals were placed on a heated operation table. A midline incision was performed and the descending colon was identified. A polyethylene catheter was inserted rectally and the descending colon was bedded extra-abdominally. To obtain a transplant tumor, human CRC cell lines (2 million cells per site) were injected into the wall of the descending colon. Care was taken not to puncture the thin wall and inject the cells into the lumen of the colon. Presence of growing tumors at the site of injection was detected by colonoscopy or laparatomy 21 days after the initial surgery. The animals were sacrificed and tumors were explanted and immediately frozen in liquid nitrogen, and tumor samples were stored at -80° C. The animals were cared for per institutional guidelines from Charite - Universitatsmdizin Berlin, Berlin, Germany and the experiments were performed after approval from the Berlin animal research authority LAGeSo (registration number G0068/10).

Snap-frozen tissue samples were embedded in Tissue-Tek® OCT™ (Sakura, Alphen aan den Rijn, The Netherlands) and cut into 20 micrometer sections. Sections corresponding to 5-10 mg of tissue were collected in a microtube. RNA from these samples was prepared using the miRNeasy kit (Qiagen, Hilden, Germany) according to the manufacturer's protocol. RNA concentration and purity were determined using spectrophotometric measurement at 260 and 280 nm, integrity of the RNA was evaluated using a total RNA nano microfluidic cartridge on the Bioanalyzer 2100 (Agilent, Böblingen, Germany).

### Immunohistochemistry

Immunohistochemistry results are shown in Table 18 for subtype-specific markers in CRC patient markers in CRC patient in CRC patient tumors from tissue microarray (Pantomics). If a marker has +++ or ++ while other markers have ++ or +, respecitively, the subtype was assigned accordingly. No inflammatory specific assay due to lack of specific antibodies. Out of 120 samples from TMA only the following were useful for analysis.

**Table 18**

| **Samples** | **CFTR-Intensity** | **MUC2-Intensity** | **TFF3-Intensity** | **ZEB1-Intensity** | **Subtype assignment** |
|---|---|---|---|---|---|
| COC1021,E12,M,67,Colon, Adenocarcinoma,II,T3N1M0,Malignant | ++ | +++ | ++ | ++ | Enterocyte |
| COC1021,G4,F,76,Colon, Adenocarcinoma,II∼III,T2N1M0,Malignant | ++ | +++ | ++ | + | Enterocyte |
| COC1021,D3,M,70,Colon, Adenocarcinoma,I∼II,T3N0M0,Malignant | + | +++ | ++ | ++ | Enterocyte |
| COC1021,A2,F,55,Colon, Normal colonic tissue,,,Normal | + | +++ | ++ | + | Enterocyte |
| COC1021,B9,M,45,Colon, Adenocarcinoma,I∼II,T3NIM0,Malignant | + | +++ | + | ++ | Enterocyte |
| COC1021,G2,M,72,Colon, Adenocarcinoma,II,T3N1M0,Malignant | + | +++ | + | ++ | Enterocyte |
| COC1021,A13,F,55,Colon,Mucinous adenocarcinoma,,T3N0M0,Malignant | ++ | +++ | +++ | ++ | Goblet-like |
| COC1021,B8,M,34,Colon, Adenocarcinoma,I∼II,T3N0M0,Malignant | ++ | +++ | +++ | ++ | Goblet-like |
| COC1021,E7,F,70,Colon, Adenocarcinoma,II,T2N0M0,Malignant | ++ | +++ | +++ | ++ | Goblet-like |
| COC1021,B3,F,60,Colon, Mucinous adenocarcinoma,,T3N1M0,Malignant | ++ | +++ | +++ | + | Goblet-like |
| COC1021,A6,M,67,Colon, Papillary Adenocarcinoma,,T3N1M0,Malignant | + | +++ | +++ | ++ | Goblet-like |
| COC1021,B4,F,61,Colon, Adenocarcinoma,I,T3N0M0,Malignant | + | +++ | +++ | ++ | Goblet-like |
| COC1021,E2,M,70,Colon, Adenocarcinoma,II,T2N0M0,Malignant | + | +++ | +++ | ++ | Goblet-like |
| COC1021,A12,F,74,Colon, Mucinous adenocarcinoma,,T3N0M0,Malignant | + | +++ | +++ | + | Goblet-like |
| COC1021,D6,M,54,Colon, Adenocarcinoma,I∼II,T2N0M0,Malignant | + | +++ | +++ | + | Goblet-like |
| COC1021,C9,F,57,Colon, Adenocarcinoma,I∼II,T2N0M0,Malignant | ++ | ++ | ++ | +++ | Stem-like |
| COC1021,F13,M,73,Colon, Adenocarcinoma,II,T3N0M0,Malignant | ++ | ++ | ++ | +++ | Stem-like |
| COC1021,F1,F,73,Colon, Adenocarcinoma,II,T3N0M0,Malignant | ++ | + | ++ | +++ | Stem-like |
| COC1021,D11,M,58,Colon, Adenocarcinoma,II,T2N0M0,Malignant | ++ | + | + | +++ | Stem-like |
| COC1021,B11,F,37,Colon, Adenocarcinoma,I∼II,T3N0M0,Malignant | + | ++ | ++ | +++ | Stem-like |
| COC1021,F4,M,48,Colon, Adenocarcinoma,II,T3N0M0,Malignant | + | ++ | ++ | +++ | Stem-like |
| COC1021,D1,M,63,Colon, Adenocarcinoma,I∼II,T3N1M0,Malignant | + | + | ++ | +++ | Stem-like |
| COC1021,C10,M,51,Colon, Adenocarcinoma,I∼II,T3N0M0,Malignant | +++ | ++ | ++ | + | TA |
| COC1021,F11,M,73,Colon, Adenocarcinoma,II,T3N0M0,Malignant | +++ | ++ | + | ++ | TA |
| COC1021,G12,M,69,Colon, Adenocarcinoma,II∼III,T3N1M0,Malignant | +++ | ++ | + | + | TA |
| COC1021,E13,M,60,Colon, Adenocarcinoma,II,T3N0M0,Malignant | +++ | + | ++ | ++ | TA |
| COC1021,E4,M,70,Colon, Adenocarcinoma,II,T3N0M0,Malignant | + | + | ++ | ++ | Unpredictable |
| COC1021,F6,F,70,Colon, Adenocarcinoma,II,T3N0M0,Malignant | + | + | ++ | ++ | Unpredictable |
| COC1021,B7,M,65,Colon, Adenocarcinoma,II,T3N1M0,Malignant | +++ | ++ | +++ | ++ | Unpredictable |
| COC1021,F5,F,29,Colon, Adenocarcinoma,II,T3N1M0,Malignant | +++ | + | +++ | ++ | Unpredictable |
| COC1021,C12,F,42,Colon, Adenocarcinoma,I∼II,T2N0M0,Malignant | + | ++ | +++ | +++ | Unpredictable |
| COC1021,H8,M,65,Colon, Adenocarcinoma,III,T3N2M0,Malignant | + | ++ | +++ | +++ | Unpredictable |
| COC1021,B10,M,69,Colon, Adenocarcinoma,I∼II,T2N0M0,Malignant | + | ++ | +++ | ++ | Unpredictable |
| COC1021,C11,F,52,Colon, Adenocarcinoma,I∼II,T3N0M0,Malignant | +++ | +++ | +++ | ++ | Unpredictable |
| COC1021,E3,M,78,Colon, Adenocarcinoma,II,T3N0M0,Malignant | +++ | +++ | ++ | +++ | Unpredictable |
| COC1021,A3,F,2,Colon, Congenital megacolon,,,Benign | +++ | +++ | ++ | + | Unpredictable |
| COC1021,A4,M,56,Colon,Adenoma,,,Benign | +++ | +++ | + | +++ | Unpredictable |
| COC1021,G8,F,75,Colon, Adenocarcinoma,II∼III,T3N1M0,Malignant | +++ | +++ | + | +++ | Unpredictable |
| COC1021,H7,M,58,Colon, Adenocarcinoma,III,T4N1M0,Malignant | +++ | +++ | + | + | Unpredictable |
| COC1021,D7,M,75,Colon, Adenocarcinoma,I∼II,T1N0M0,Malignant | ++ | +++ | +++ | +++ | Unpredictable |
| COC1021,G10,M,65,Colon, Adenocarcinoma,II∼III,T3N0M0,Malignant | ++ | +++ | +++ | +++ | Unpredictable |
| COC1021,D10,M,48,Colon, Adenocarcinoma,II,T3N0M0,Malignant | ++ | +++ | + | +++ | Unpredictable |
| COC1021,E10,F,81,Colon, Adenocarcinoma,II,T3N1M0,Malignant | + | +++ | +++ | +++ | Unpredictable |
| COC1021,F2,M,71,Colon, Adenocarcinoma,II,T3N1M0,Malignant | + | +++ | +++ | +++ | Unpredictable |
| COC1021,G6,F,60,Colon, Adenocarcinoma,II∼III,T3N0M0,Malignant | + | +++ | +++ | +++ | Unpredictable |
| COC1021,C8,M,61,Colon, Adenocarcinoma,I∼II,T3N1M0,Malignant | + | +++ | ++ | +++ | Unpredictable |
| COC1021,C3,M,53,Colon, Adenocarcinoma,I∼II,T3N0M0,Malignant | + | +++ | + | +++ | Unpredictable |
| COC1021,H3,F,68,Colon, Adenocarcinoma,III,T4N2M0,Malignant | + | +++ | + | +++ | Unpredictable |
| COC1021,C4,M,50,Colon, Adenocarcinoma,I∼II,T2N0M0,Malignant | + | ++ | ++ | ++ | Unpredictable |
| COC1021,D8,F,64,Colon, Adenocarcinoma,I∼II,T2N0M0,Malignant | +++ | + | +++ | +++ | Unpredictable |
| COC1021,E1,M,79,Colon, Adenocarcinoma,II,T2N0M0,Malignant | +++ | + | +++ | +++ | Unpredictable |
| COC1021,A5,M,48,Colon, Adenoma,,,Benign | ++ | ++ | +++ | + | Unpredictable |
| COC1021,B2,F,54,Colon, Mucinous adenocarcinoma,,T2N0M0,Malignant | ++ | ++ | +++ | + | Unpredictable |

## Claims

1. An *in-vitro* method for the prognosis of disease-free survival of a subject suffering from colorectal cancer or suspected of suffering therefrom and who has undergone a prior surgical resection of colorectal cancer, the method comprising
(i) measuring in a biological sample obtained from said subject comprising colorectal cancer cells or suspected to comprise colorectal cancer cells the expression level of
- the genes listed in Table 2, or
- the combination of genes comprising LY6G6D, KRT23, CEL, ACSL6, EREG, CFTR, TCN1, PCSK1, NCRNA00261, SPINK4, REG4, MUC2, TFF3, CLCA4, ZG16, CA1, MS4A12, CA4, CXCL13, RARRES3, GZMA, IDO1, CXCL9, SFRP2, COL10A1, CYP1B1, MGP, MSRB3, ZEB1, FLNA, or
- the combination of genes comprising SFRP2, ZEB1, RARRES3, CFTR, FLNA, MUC2, TFF3, and
(ii) classifying said biological sample as "Stem-like", "Inflammatory", "Transit-amplifying (TA)", "Goblet-like" and "Enterocyte" on the basis of the gene expression profile according to Table 2,
wherein
- "Stem-like" type of colorectal cancer indicates poor disease-free survival,
- "Inflammatory" type of colorectal cancer indicates intermediate disease-free survival,
- "Transit-amplifying (TA)" type of colorectal cancer indicates good disease-free survival,
- "Goblet-like" type of colorectal cancer indicates good disease-free survival, and
- "Enterocyte" type of colorectal cancer indicates intermediate disease-free survival.

2. An *in-vitro* method for predicting the likelihood that a subject suffering from colorectal cancer or suspected of suffering therefrom and who has undergone a prior surgical resection of colorectal cancer will respond to therapies inhibiting or targeting EGFR, such as cetuximab, and/or cMET, the method comprising
(i) measuring in a biological sample obtained from said subject comprising colorectal cancer cells or suspected to comprise colorectal cancer cells the expression level of the genes listed in Table 2, and
(ii) classifying said biological sample as "Stem-like", "Inflammatory", "Transit-amplifying (TA)", "Goblet-like" and "Enterocyte" on the basis of the gene expression profile according to Table 2,
wherein
- high expressions of AREG and EREG genes and low expressions of BHLHE41, FLNA and PLEKHB1 genes in "Transit-amplifying (TA)" type indicates that at metastatic setting said subject will be responsive to cetuximab treatment and resistant to cMET inhibitor therapy and this signature defines a subtype of TA type designed as "Cetuximab-sensitive transit-amplifying subtype (CS-TA)".
- low expressions of AREG and EREG genes and high expressions of BHLHE41, FLNA and PLEKHB1 genes in "Transit-amplifying (TA)" type indicates that at metastatic setting said subject will be resistant to cetuximab treatment and will be responsive to cMET inhibitor therapy, and this signature defines a second subtype of TA type named as "Cetuximab-resistant transit-amplifying subtype (CR-TA)".

3. An *in-vitro* method for predicting the likelihood that a subject suffering from colorectal cancer or suspected of suffering therefrom and who has undergone a prior surgical resection of colorectal cancer will respond to cytotoxic chemotherapies such as FOLFIRI, the method comprising
(i) measuring in a biological sample obtained from said subject comprising colorectal cancer cells or suspected to comprise colorectal cancer cells the expression level of
- the genes listed in Table 2, or
- the combination of genes comprising LY6G6D, KRT23, CEL, ACSL6, EREG, CFTR, TCN1, PCSK1, NCRNA00261, SPINK4, REG4, MUC2, TFF3, CLCA4, ZG16, CA1, MS4A12, CA4, CXCL13, RARRES3, GZMA, IDO1, CXCL9, SFRP2, COL10A1, CYP1B1, MGP, MSRB3, ZEB1, FLNA, or
- the combination of genes comprising SFRP2, ZEB1, RARRES3, CFTR, FLNA, MUC2, TFF3, and
(iii) classifying said biological sample as "Stem-like", "Inflammatory", "Transit-amplifying (TA)", "Goblet-like" and "Enterocyte" on the basis of the gene expression profile according to Table 2,
wherein
- "Stem-like" type of colorectal cancer predicts good response in both adjuvant and metastatic settings,
- "Inflammatory" type of colorectal cancer predicts good response in adjuvant setting,
- "TA (transit-amplifying)" type of colorectal cancer predicts poor response in both adjuvant and metastatic settings,
- "Goblet-like" type of colorectal cancer predicts poor response in adjuvant setting, and
- "Enterocyte" type of colorectal cancer predicts good response in adjuvant setting.

## Patentansprüche

1. *In-vitro*-Verfahren für die Prognose von krankheitsfreiem Überleben eines Subjekts, der an Kolorektalkrebs leidet oder bei dem der Verdacht daran besteht, und der sich einer vorherigen chirurgischen Resektion eines Kolorektalkrebses unterzogen hat, umfassend::
(i) das Messen in einer vom besagten Subjekt erhaltenen biologischen Probe mit Kolorektalkrebszellen oder die man verdächtigt, Kolorektalkrebszellen zu enthalten, vom Grad der Expression von
- den in Tabelle 2 angegebenen Genen, oder
- einer Kombination von Genen umfassend LY6G6D, KRT23, CEL, ACSL6, EREG, CFTR, TCN1, PCSK1, NCRNA00261, SPINK4, REG4, MUC2, TFF3, CLCA4, ZG16, CA1, MS4A12, CA4, CXCL13, RARRES3, GZMA, IDO1, CXCL9, SFRP2, COL10A1, CYP1B1, MGP, MSRB3, ZEB1, FLNA oder
- der Kombination von Genen umfassend SFRP2, ZEBI, RARRES3, CFTR, FLNA, MUC2, TFF3, und
(ii) das Klassifizieren der biologischen Probe als "mit stammähnlichem Charakter", "entzündlich", "transit-amplifizierend" (TA), "becherförmig" und "enterozyt" auf der Basis des Genexpressionsprofils gemäss Tabelle 2,
worin
- ein Kolorektalkrebs vom Typ "mit stammähnlichem Charakter" ein geringes krankheitsfreies Überleben bezeichnet,
- ein Kolorektalkrebs vom Typ "entzündlich" mittelmässige Chancen für ein krankheitsfreies Überleben bezeichnet,
- ein Kolorektalkrebs vom Typ "transit-amplifizierend" (TA) gute Chancen für ein krankheitsfreies Überleben bezeichnet,
- ein Kolorektalkrebs vom Typ "becherförmig" gute Chancen für ein krankheitsfreies Überleben bezeichnet, und
- ein Kolorektalkrebs vom Typ "enterozyt" mittelmässige Chancen für ein krankheitsfreies Überleben bezeichnet.

2. *In-vitro*-Verfahren für die Prognose der Wahrscheinlichkeit, dass ein Subjekt, der an Kolorektalkrebs leidet oder bei dem der Verdacht daran besteht, und der sich einer vorherigen chirurgischen Resektion eines Kolorektalkrebses unterzogen hat, auf EGFR-hemmende oder anzielenden Therapien wie cetuximab, und/oder cMET, positiv reagiert, wobei das Verfahren umfasst:
(i) das Messen in einer vom besagten Subjekt erhaltenen biologischen Probe mit Kolorektalkrebszellen oder die man verdächtigt, Kolorektalkrebszellen zu enthalten, vom Grad der Expression der in der Tabelle 2 angegebenen Genen, und
(ii) das Klassifizieren der biologischen Probe als "mit stammähnlichem Charakter", "entzündlich", "transit-amplifizierend" (TA), "becherförmig" und "enterozyt" auf der Basis des Genexpressionsprofils gemäss Tabelle 2,
worin
- ein hoher Grad an AREG- und EREG-Genexpression und ein niedriger Grad an BHLHE41-, FLNA- und PLEKHB1-Genexpression im "transit-amplifizierenden"-Typ (TA) angibt, dass im Fall von Metastasen, der besagte Subjekt positiv auf die cetuximab-Behandlung reagieren wird und auf die cMET-hemmende Therapie resistent ist, und diese Signatur definiert einen Untertyp des "transit-amplifizierenden"-Typs (TA), welcher "Cetuximab-empfindlich transit-amplifizierender Untertyp" (CS-TA) benannt wird;
- ein niedriger Grad an AREG- und EREG-Genexpression und ein hoher Grad an BHLHE41-, FLNA- und PLEKHB1-Genexpression im "transit-amplifizierenden"-Typ (TA) angibt, dass im Fall von Metastasen, der besagte Subjekt auf die cetuximab-Behandlung resistent ist und auf die cMET-hemmende Therapie positiv reagieren wird, und diese Signatur definiert einen zweiten Untertyp des "transit-amplifizierenden"-Typs (TA), welcher "Cetuximab-resistent transit-amplifizierender Untertyp" (CR-TA) benannt wird.

3. *In-vitro*-Verfahren zur Prognose der Wahrscheinlichkeit, dass ein Subjekt, der an Kolorektalkrebs leidet oder bei dem der Verdacht daran besteht, und der sich einer vorherigen chirurgischen Resektion eines Kolorektalkrebses unterzogen hat, positiv auf Chemotherapie wie FOLFIRI reagiert, wobei das Verfahren umfasst:
(i) das Messen in einer vom besagten Subjekt erhaltenen biologischen Probe mit Kolorektalkrebszellen oder die man verdächtigt, Kolorektalkrebszellen zu enthalten, vom Grad der Expression der in der Tabelle 2 angegebenen Genen, oder
- die Kombination von Genen umfassend LY6G6D, KRT23, CEL, ACSL6, EREG, CFTR, TCN1, PCSK1, NCRNA00261, SPINK4, REG4, MUC2, TFF3, CLCA4, ZG16, CA1, MS4A12, CA4, CXCL13, RARRES3, GZMA, IDO1, CXCL9, SFRP2, COL10A1, CYP1B1, MGP, MSRB3, ZEB1, FLNA oder
- die Kombination von Genen umfassend SFRP2, ZEBI, RARRES3, CFTR, FLNA, MUC2, TFF3, und
(iii) das Klassifizieren der biologischen Probe als "mit stammähnlichem Charakter", "entzündlich", "transit-amplifizierend" (TA), "becherförmig" und "enterozyt" auf der Basis des Genexpressionsprofils gemäss Tabelle 2,
worin
- ein Kolorektalkrebs vom Typ "mit stammähnlichem Charakter" eine positive Reaktion im Falle sowohl von adjuvanten Therapien wie auch von Metastasen prognostiziert,
- ein Kolorektalkrebs vom Typ "entzündlich" eine positive Reaktion im Falle von adjuvanten Therapien prognostiziert,
- ein Kolorektalkrebs vom Typ "transit-amplifizierend" (TA) eine geringe Reaktion im Falle sowohl von adjuvanten Therapien wie auch von Metastasen prognostiziert,
- ein Kolorektalkrebs vom Typ "becherförmig" eine geringe Reaktion im Falle von adjuvanten Therapien prognostiziert, und
- ein Kolorektalkrebs vom Typ "enterozyt" eine positive Reaktion im Falle von adjuvanten Therapien prognostiziert.

## Revendications

1. Procédé *in-vitro* pour le pronostic de survie sans récidive d'un sujet souffrant de cancel colorectal ou qu'on suppose d'en souffrir et qui a subi préalablement une résection chirurgicale d'un cancer colorectal, le procédé comprenant :
(i) de mesurer dans un échantillon biologique obtenu dudit sujet comprenant des cellules de cancer colorectal ou qu'on soupçonne de comprendre des cellules de cancer colorectal le niveau d'expression de
- les gènes énumérés au tableau 2, ou
- une combinaison de gènes comprenant LY6G6D, KRT23, CEL, ACSL6, EREG, CFTR, TCN1, PCSK1, NCRNA00261, SPINK4, REG4, MUC2, TFF3, CLCA4, ZG16, CA1, MS4A12, CA4, CXCL13, RARRES3, GZMA, IDO1, CXCL9, SFRP2, COL10A1, CYP1B1, MGP, MSRB3, ZEB1, FLNA ou
- la combinaison de gènes comprenant SFRP2, ZEBI, RARRES3, CFTR, FLNA, MUC2, TFF3, et
(ii) de classifier ledit échantillon biologique comme « présentant des caractéristiques comparables à celles des cellules souches », « inflammatoires », « amplifiant le transit » (TA, transit-amplifying), « caliciforme » et « entérocyte » sur la base du profile d'expression génétique selon le tableau 2,
dans lequel
- un type de cancer colorectal « présentant des caractéristiques comparables à celles des cellules souches » indique de faibles perspectives de survie sans récidive,
- un type de cancer colorectal « inflammatoire » indique des perspectives moyennes de survie sans récidive,
- un type de cancer colorectal « amplifiant le transit » (TA) indique de bonne perspectives de survie sans récidive,
- un type de cancer colorectal « caliciforme » indique de bonnes perspectives de survie sans récidive, et
- un type de cancer colorectal « entérocyte » indique des perspectives moyennes de survie sans récidive.

2. Procédé *in-vitro* pour prédire la probabilité qu'un sujet souffrant de cancel colorectal ou qu'on suppose d'en souffrir et qui a subi préalablement une résection chirurgicale d'un cancer colorectal répondra favorablement à des thérapies inhibant ou ciblant ECFR, telles que cetuximab, et/ou cMET, le procédé comprenant :
(i) de mesurer dans un échantillon biologique obtenu dudit sujet comprenant des cellules de cancer colorectal ou qu'on soupçonne de comprendre des cellules de cancer colorectal le niveau d'expression des gènes énumérés au tableau 2, et
(ii) de classifier ledit échantillon biologique comme « présentant des caractéristiques comparables à celles des cellules souches », « inflammatoires », « amplifiant le transit » (TA), « caliciforme » et « entérocyte » sur la base du profile d'expression génétique selon le tableau 2,
dans lequel
- un niveau élevé d'expressions de gènes AREG et EREG et un niveau bas d'expressions de gènes BHLHE41, FLNA et PLEKHB1 dans le type « amplifiant le transit » (TA) indique que dans le cas de métastase, ledit sujet réagira favorablement au traitement cetuximab et résistera à la thérapie par inhibition cMET, et cette signature définit un sous-type du type « amplifiant le transit » (TA) appelé « sous-type amplifiant le transit sensible au cetuximab » (CS-TA) ;
- un niveau bas d'expressions de gènes AREG et EREG et un niveau élevé d'expressions de gènes BHLHE41, FLNA et PLEKHB1 dans le type « amplifiant le transit » (TA) indique que dans le cas de métastase, ledit sujet résistera au traitement cetuximab et réagira favorablement à la thérapie par inhibition cMET, et cette signature définit un deuxième sous-type du type « amplifiant le transit » (TA) appelé « sous-type amplifiant le transit résistant au cetuximab » (CR-TA).

3. Procédé *in-vitro* pour prédire la probabilité qu'un sujet souffrant de cancel colorectal ou qu'on suppose d'en souffrir et qui a subi préalablement une résection chirurgicale d'un cancer colorectal répondra favorablement aux chimiothérapies telles que FOLFIRI, le procédé comprenant :
(i) de mesurer dans un échantillon biologique obtenu dudit sujet comprenant des cellules de cancer colorectal ou qu'on soupçonne de comprendre des cellules de cancer colorectal le niveau d'expression des gènes énumérés au tableau 2, ou
- la combinaison de gènes comprenant LY6G6D, KRT23, CEL, ACSL6, EREG, CFTR, TCN1, PCSK1, NCRNA00261, SPINK4, REG4, MUC2, TFF3, CLCA4, ZG16, CA1, MS4A12, CA4, CXCL13, RARRES3, GZMA, IDO1, CXCL9, SFRP2, COL10A1, CYP1B1, MGP, MSRB3, ZEB1, FLNA ou
- la combinaison de gènes comprenant SFRP2, ZEBI, RARRES3, CFTR, FLNA, MUC2, TFF3, et
(iii) de classifier ledit échantillon biologique comme « présentant des caractéristiques comparables à celles des cellules souches », « inflammatoires », « amplifiant le transit » (TA), « caliciforme » et « entérocyte » sur la base du profile d'expression génétique selon le tableau 2,
dans lequel
- un type de cancer colorectal « présentant des caractéristiques comparables à celles des cellules souches » prédit une réaction favorable dans le cas de traitement adjuvant et dans le cas de métastase,
- un type de cancer colorectal « inflammatoire » prédit une réaction favorable dans le cas de traitement adjuvant,
- un type de cancer colorectal « amplifiant le transit » (TA) prédit une faible réaction tant dans le cas de traitement adjuvant que dans le cas de métastase,
- un type de cancer colorectal « caliciforme » prédit une faible réaction dans le cas de traitement adjuvant, et
- un type de cancer colorectal « entérocyte » prédit une réaction favorable dans le cas de traitement adjuvant.
